# EUROPEAN PATENT APPLICATION

(11) **EP 4 692 346 A1**
(43) Date of publication of application: **11.02.2026**
(21) Application number: 24780394.3
(22) Date of filing: 26.03.2024
(51) Int. Cl.: C12N 15/11, C12N 5/10, C12N 15/09, C12Q 1/686

(54) **CRRNA HAVING MODIFIED NUCLEOTIDE**

(30) Priority: 27.03.2023 JP 2023050681
(71) Applicant: Kyoto University, Kyoto-shi, Kyoto 606-8501 (JP)
(72) Inventor: HOTTA, Akitsu, Kyoto-shi, Kyoto 606-8501 (JP); KOJIMA, Yusuke, Kyoto-shi, Kyoto 606-8501 (JP); KITA, Yuto, Kyoto-shi, Kyoto 606-8501 (JP)
(74) Representative: J A Kemp LLP
(86) International application number: PCT/JP2024/012128
(87) International publication number: WO 2024/204287

(57) **Abstract**

The present invention is a pre-crRNA of a type I CRISPR-Cas system having a modified nucleotide, wherein, in a region consisting of the 5' arm region of the first repeat sequence, the 5'-side stem-forming region of the first repeat sequence, and the loop-forming region of the first repeat sequence, at least one nucleotide is the modified nucleotide.

## Description

### TECHNICAL FIELD

The present invention relates to a pre-crRNA with a modified nucleotide. More specifically, the present invention relates to a pre-crRNA with a specific modification, an allele-specific pre-crRNA, a method for producing a double-stranded DNA-modified cell using such a pre-crRNA, and the like.

### BACKGROUND ART

In recent years, technologies to which the CRISPR-Cas systems with the clustered regularly interspaced short palindromic repeat (CRISPR), which is an acquired immune system of eubacteria and archaeobacteria, and a CRISPR-associated (Cas) protein as main constituent elements is applied have been developed as genome editing technologies such as gene disruption (knockout) by freely designing the target sequences of guide RNAs, and have been widely used. The CRISPR-Cas systems are broadly divided into the class-1 CRISPR-Cas system in which a complex composed of a plurality of proteins functions as an effector acting during the process of cleaving a DNA in a guide RNA dependent manner, and the class-2 CRISPR-Cas system in which a single protein functions as such an effector. Out of these systems, the CRISPR-Cas9 system belonging to type II of the class 2 is most frequently used at present due to its simplicity and broad application range. In addition, as another CRISPR-Cas system belonging to the class 2 used for genome editing, the type-V CRISPR-Cas12a system is also known.

Meanwhile, genome editing using the type I CRISPR-Cas system of the class 1 that uses a Cas protein and a Cascade complex to cleave a DNA is also being developed. For example, the type I-E CRISPR-Cas3 system developed in The University of Osaka (Patent Literature 1), the type I-D CRISPR-Cas10 (TiD) system developed in Tokushima University (Patent Literature 2), and the like are known. Also, previously, the inventors of the present invention focused on a genome editing method using an RNA encoding a Cas protein and a Cascade complex rather than a plasmid DNA that may be unexpectedly incorporated into the cell genome and an RNP (ribonucleoprotein) that requires protein purification, and thus succeeded in introducing a deletion of a nucleotide of more than 100 bases to a target region of the genome DNA using the CRISPR-Cas3 system (Patent Literature 3). The class-1 CRISPR-Cas system needs a plurality of proteins, but has a different DNA cleavage mechanism from that of the class-2 CRISPR-Cas system, and is thus very useful in various cases. For example, it can induce a unique DNA deletion pattern. It is known that the CRISPR-Cas3 system induces, in only one direction, a long-chain DNA deletion of several hundred bases to hundred kilobases or more starting from a position several ten bases to several hundred bases away from the recognition sequence of the guide RNA. The recognition sequence of the guide RNA for CRISPR-Cas3 is longer than that for the CRISPR-Cas9 system, suggesting that the guide RNA for CRISPR-Cas3 has higher specificity.

Experimental cancer cell strains that are often used in the fields of biology and medicine include many strains having high gene transfer efficiency, such as HEK293T cells, and such strains are often used as a model strain for genome editing. However, iPS cells, primary cultured cell strains, and the like intended to be practically applied to regenerative medicine and the like do not necessarily have high gene transfer efficiency. Furthermore, only a small number of cell strains selected for clinical use have been established. Therefore, the most suitable strain for gene transfer cannot always be selected. For this reason, it is desired to develop a method capable of achieving stable and efficient genome editing even in a cell strain having low gene transfer efficiency. From the viewpoint of stable and efficient genome editing, it is important to make the current class-1 CRISPR-Cas system more useful.

In order to make the genome editing a practical technique, it is necessary to precisely analyze a type of DNA mutation that can be induced using the technique, and the efficiency of inducing mutation (cleavage). In particular, it is known that, unlike Cas9, which induces minute cleavage of a DNA double strand, Cas3 and Cas10 induce various long-chain deletions different in length, but a method capable of easily analyzing the length of deletion mutation and the ratio thereof has not been developed yet. At present, in order to analyze the deletion mutation pattern, it is necessary to amplify the target site through PCR or the like. However, the amplification efficiency of PCR varies depending on the length of an amplification product, and deletion mutation (several tens of kilobases or more) beyond an amplifiable range cannot be amplified and cannot be detected consequently. Therefore, it cannot be said that the length of deletion mutation and the efficiency are accurately reflected. If whole-genome sequencing is conducted using a next-generation sequencer, or a sequence in proximity to the target is pulled down and sequenced, cleavage patterns can be analyzed to some extent (Non-Patent Literature 1), but the analysis cost increases, and technology and time are required to analyze a large amount of data.

### Citation List

### Patent Literature

Patent Literature 1: WO 2018/225858
Patent Literature 2: WO 2019/039417
Patent Literature 3: WO 2020/122104

### Non-Patent Literature

Non-Patent Literature 1: Morisaka H. et al., Nat Commun. 6; 10(1): 5302 (2019)

### SUMMARY OF INVENTION

### Technical Problem

In order to make the class-1 CRISPR-Cas system more useful and to make it a more practical technique, it is important to, for example, improve the editing efficiency for cells with low genome editing efficiency, accurately determine a pattern of genome editing induced by the CRISPR-Cas system, and expand a genome editing application method tailored to various demands. Accordingly, it is an object of the present invention to provide a method with which the editing efficiency of genome editing is improved and a pattern of genome editing by a CRISPR-Cas system is analyzed, and a tool that can be used in the method.

A method for treating a genetic disease using genome editing has also been developed. Such a disease includes a dominantly-inherited disease such as myotonic dystrophy I (DM1) caused by abnormality of one allele on the autosomes, but both alleles basically undergo genome editing when the current genome editing technique is used. Also, even if an attempt is made to induce genome editing on only the target allele by targeting a region containing a target allele-specific SNP sequence, a problem arises in that there is no suitable hetero SNP in close proximity to the genome editing target site. Accordingly, it is also an object to provide a method for specifically conducting genome editing on one allele by targeting a hetero SNP away from a genome editing target site.

### Solution to Problem

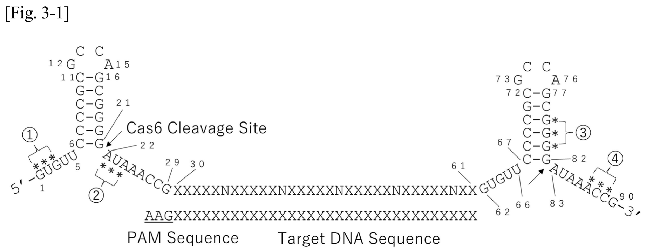

In order to solve the aforementioned problems, the inventors of the present invention came up with an idea that degradation of an RNA by RNase and the like present inside and outside a cell can be suppressed by applying a chemical modification to a specific region of a pre-crRNA, and the genome editing efficiency can be consequently enhanced. In general, it is known that an unmodified RNA is rapidly degraded inside a cell as a target of a nuclease, and modifying an RNA makes it possible to prevent the degradation and stabilize the RNA. However, modifying an RNA may change the physical properties, the secondary structure, and the interaction with a protein of the RNA and thus lead to loss of original functions, and the fact is that even specialists cannot predict which type of modification should be selected and where the modification should be introduced. In fact, various types of RNA modifications have been tested in the guide RNA (sgRNA or gRNA-crRNA) for Cas9, but it is difficult to predict an optimal type of modification and an optimal position. Furthermore, the pre-crRNA for the E. coli CRISPR-Cas3 system is an RNA of 90 bases in total that includes two 29-base repeat sequences and a 32-base spacer sequence (FIG. 3-1), but a chemical modification pattern for this pre-crRNA in use in a mammalian cell has not been investigated yet. The pre-crRNA for the CRISPR-Cas3 system and the guide RNA for the CRISPR-Cas9 system differ from each other in the sequence, the structure, and the functional features, and therefore, the optimal modification positions cannot be referred to. Moreover, it has been reported based on biochemical experiments that pre-crRNA processing by Cas6 is required in the CRISPR-Cas3 system, and the complexity of the mechanism also makes it difficult to predict an optimal modification position.

Accordingly, the inventors of the present invention introduced a chemical modification to the pre-crRNA for the CRISPR-Cas3 system to search for a modification position for enhancing the activity. As a result, it was found that applying a modification to a specific region of the pre-crRNA significantly enhances the genome editing efficiency without loss of the crRNA functions. Surprisingly, although the pre-crRNA has a nearly symmetric structure, it was suggested that left-right asymmetry is observed in the pre-crRNA modification effects. It was also found that introducing a modification similar to the modification applied to an mRNA in vivo enhances the activity of the pre-crRNA.

Furthermore, an attempt was made to develop a single-strand annealing (SSA) reporter vector for the type I CRISPR-Cas system by altering an SSA reporter vector for the CRISPR-Cas9 system. Specifically, the inventors came up with an idea that, based on the DNA alteration pattern for the type I CRISPR-Cas system, a Cas9-recognition sequence in the SSA reporter vector for the CRISPR-Cas9 system is replaced with a longer sequence. Also, the inventors noted that Cas3 is an enzyme nickase, which is a single-stranded DNA cleavage enzyme, and came up with an idea that a mechanism to induce cleavage of a double strand through DNA double nicking can also be applied to this SSA reporter vector. As a result of actually constructing these SSA reporter vectors and conducting experiments, cells in which the type I CRISPR-Cas system was active could be monitored using these SSA reporter vectors, leading to a success in selecting cells with double-stranded DNAs altered by the CRISPR-Cas system.

Also, the inventors of the present invention made an attempt to develop a technique for measuring the length distribution and the ratios of deletion mutations by the CRISPR-Cas system by applying the droplet digital PCR (ddPCR) technique. First, the inventors came up with a method in which a plurality of ddPCR probes are designed at various positions around a target sequence and the copy number at each position is directly measured in the genome DNA of a genome-edited cell. As a result of sincere examination, the inventors succeeded in capturing, through ddPCR, a situation where the genome editing efficiency changed. As a result of further conducting a study based on these findings, the inventors achieved the present invention.

That is to say, the present invention is as follows.
[1-1] A pre-crRNA for a type I CRISPR-Cas system, comprising a modified nucleotide,
   wherein at least one nucleotide is a modified nucleotide in a region consisting of a 5' arm region of a first repeat sequence, a 5'-side stem-forming region of the first repeat sequence, and a loop-forming region of the first repeat sequence.
[1-2] The pre-crRNA according to [1-1], wherein at least one nucleotide is a modified nucleotide in the 5' arm region of the first repeat sequence.
[1-3] The pre-crRNA according to [1-1] or [1-2], wherein a first nucleotide from a 5' terminus is a modified nucleotide.
[1-4] The pre-crRNA according to any one of [1-1] to [1-3], wherein first to third nucleotides from a 5' terminus are modified nucleotides.
[1-5] The pre-crRNA according to any one of [1-1] to [1-4], wherein all nucleotides in the 5' arm region of the first repeat sequence are modified nucleotides.
[2-1] The pre-crRNA according to any one of [1-1] to [1-5], wherein at least one nucleotide in a 3' arm region of a second repeat sequence is a modified nucleotide.
[2-2] The pre-crRNA according to [2-1], wherein a second nucleotide from a 3' terminus is a modified nucleotide.
[2-3] The pre-crRNA according to [2-1] or [2-2], wherein second to fourth nucleotides from a 3' terminus are modified nucleotides.
[3] The pre-crRNA according to any one of [1-1] to [2-3], wherein first to third nucleotides from a 5' terminus and second to fourth nucleotides from a 3' terminus are modified nucleotides.
[4-1] The pre-crRNA according to any one of [1-1] to [3], wherein at least one nucleotide, optionally all nucleotides, in a loop-forming region of the first repeat sequence is a modified nucleotide.
[4-2] The pre-crRNA according to any one of [1-1] to [4-1], wherein at least one nucleotide, optionally all nucleotides, in a loop-forming region of a second repeat sequence is a modified nucleotide.
[4-3] The pre-crRNA according to any one of [1-1] to [4-2], wherein at least one modified nucleotide, optionally all modified nucleotides, is a 2'-O-methyl ribonucleotide.
[4-4] The pre-crRNA according to any one of [1-1] to [4-3], wherein at least one internucleoside bond is a phosphorothioate bond.
[4-5] The pre-crRNA according to any one of [1-1] to [4-4], wherein an internucleoside bond on a 3'-side of at least one or all nucleosides with a modified sugar moiety is a modified internucleoside bond.
[4-6] The pre-crRNA according to [4-5], wherein the internucleoside bond is a phosphorothioate bond.
[4-7] The pre-crRNA according to any one of [1-1] to [4-6], wherein a 3'-side stem-forming region of the first repeat sequence and/or stem-forming regions of a second repeat sequence are all constituted by ribonucleotides.
[4-8] The pre-crRNA according to any one of [1-1] to [4-7], wherein a 3' arm region of the first repeat sequence, a 5' arm region of a second repeat sequence, and/or 5'-side stem-forming region of the second repeat sequence are all constituted by ribonucleotides.
[4-9] The pre-crRNA according to any one of [1-1] to [4-8], wherein a 3'-side stem-forming region of the first repeat sequence, a 3' arm region of the first repeat sequence, a 5' arm region of a second repeat sequence, and stem-forming regions of the second repeat sequence are all constituted by ribonucleotides.
[4-10] The pre-crRNA according to any one of [1-1] to [4-9], wherein an entirety of a spacer sequence is constituted by ribonucleotides.
[4-11] The pre-crRNA of any one of [1-1] to [4-10], wherein a site to be cleaved by Cas6 or Cas5 is not modified.
[5-1] The pre-crRNA according to any one of [1-1] to [4-11], wherein first to third nucleotides from a 5' terminus and second to fourth nucleotides from a 3' terminus are modified nucleotides, all nucleotides included in a 3' arm region of the first repeat sequence, a 5' arm region of a second repeat sequence, and a spacer region are ribonucleotides, and a site to be cleaved by Cas6 or Cas5 is not modified.
[5-2] The pre-crRNA according to [5-1], wherein an internucleoside bond on a 3'-side of at least one or all nucleosides with a modified sugar moiety is a modified internucleoside bond.
[5-3] The pre-crRNA according to [5-1] or [5-2], wherein a bond on a 3'-side of a ribonucleoside is a phosphodiester bond.
[6] The method according to any one of [1-1] to [5-3], comprising two or more spacer regions.
[7] The pre-crRNA according to any one of [1-1] to [6], wherein the type I CRISPR-Cas system is a type I-E CRISPR-Cas system, optionally a CRISPR-Cas system derived from E. coli.
[8-1] A pre-crRNA for a type I CRISPR-Cas system, having a 5' cap and poly-A.
[8-2] The pre-crRNA according to [8-1], wherein the type I CRISPR-Cas system is a type I-E CRISPR-Cas system, optionally a CRISPR-Cas system derived from E. coli.
[8-3] The pre-crRNA according to any one of [1-1] to [7], having a 5' cap and poly-A.
[9-1] A method for designing an allele-specific pre-crRNA for a type I CRISPR-Cas system, comprising a step of designing a pre-crRNA targeting a sequence in which
   a deletion target site is present on an upstream side of a PAM sequence adjacent to a sequence targeted by the pre-crRNA, and
   at least one base that is different between alleles at positions other than 6n^{th} (n is a positive integer) positions from a terminal base on a downstream side of the PAM sequence or in the PAM sequence.
[9-2] The method according to [9-1], wherein the type I CRISPR-Cas system is a type I-E CRISPR-Cas system, optionally a CRISPR-Cas system derived from E. coli.
[10] The method according to [9-1] or [9-2], comprising a step of adjusting a length of the pre-crRNA, optionally a length of a spacer sequence of the pre-crRNA, and/or a step of introducing a mismatch to the pre-crRNA, optionally the spacer sequence of the pre-crRNA.
[11-1] The method according to any one of [9-1] to [10], wherein the deletion target site comprises a repeat.
[11-2] The method according to [11-1], wherein the repeat is a triplet repeat.
[12-1] A type I CRISPR-Cas system comprising (1) or (2) below:
   (1) the pre-crRNA according to any one of [1-1] to [8-3], or a pre-crRNA designed using the method according to any one of [9-1] to [11-2], and
   (2) a protein group included in Cascade, or a nucleic acid encoding the protein group.
[12-2] The CRISPR-Cas system according to [12-1], further comprising (3) Cas3 and/or Cas10d, or a nucleic acid encoding Cas3 and/or Cas10d.
[13] A double-stranded nucleic acid for evaluating activity of a type I CRISPR-Cas system, wherein one nucleic acid strand comprises:
   (1) a sequence encoding a portion of a reporter protein, optionally a fluorescent protein;
   (2) an insertion sequence that includes a sequence targeted by a crRNA or a sequence complementary to the sequence targeted by the crRNA; and
   (3) a sequence that encodes a portion of the reporter protein and is different from the sequence of (1), in this order,

   the sequences of (1) and (3) have an overlapping sequence, the sequence of (1) excluding the overlapping sequence, the overlapping sequence, and the sequence of (3) excluding the overlapping sequence constituting a sequence encoding the entire reporter protein, and
   the insertion sequence of (2) has a length of 100 to 3000 bases.
[14-1] A double-stranded nucleic acid for evaluating activity of a type I CRISPR-Cas system, wherein one nucleic acid strand comprises:
   (1) a sequence encoding a portion of a reporter protein, optionally a fluorescent protein;
   (2) an insertion sequence that includes a sequence targeted by a crRNA and a sequence complementary to the sequence targeted by the crRNA; and
   (3) a sequence that encodes a portion of the reporter protein and is different from the sequence of (1), in this order, and
   the sequences of (1) and (3) have an overlapping sequence, the sequence of (1) excluding the overlapping sequence, the overlapping sequence, and the sequence of (3) excluding the overlapping sequence constituting a sequence encoding the entire reporter protein.
[14-2] An expression vector comprising the double-stranded nucleic acid according to [13] or [14-1].
[15-1] A method for producing a cell with an altered double-stranded DNA, comprising a step of introducing the CRISPR-Cas system according to [12-1] or [12-2] to a cell.
[15-2] A method for altering a double-stranded DNA of a cell, comprising a step of introducing the CRISPR-Cas system according to [12-1] or [12-2] to a cell.
[16] The method according to [15-1] or [15-2], wherein the step of introducing the CRISPR-Cas system to a cell is conducted a plurality of times.
[17] The method according to any one of [15-1] to [16], comprising a step of introducing the double-stranded nucleic acid according to [13] or [14-1], or the expression vector according to [14-2] to a cell.
[18] The method according to [17], wherein two or more types of the double-stranded nucleic acids are introduced.
[19] The method according to [17] or [18], comprising a step of sorting a cell expressing a reporter protein.
[20-1] The method according to any one of [15-1] to [19], wherein the cell is a pluripotent stem cell, optionally an induced pluripotent stem cell.
[20-2] The method according to any one of [15-1] to [20-1], wherein the cell is a mammalian cell, optionally a human cell.
[21-1] A method for analyzing a double-stranded DNA editing pattern by a type I CRISPR-Cas system, comprising a step of conducting digital PCR using a plurality of PCR probes for a deletion target site.
[21-2] The method according to [21-1], wherein the type I CRISPR-Cas system is a type I-E CRISPR-Cas system, optionally a CRISPR-Cas system derived from E. coli.
[22-1] A method for extending a deletion site, comprising a step of introducing a type I CRISPR-Cas system to a cell a plurality of times.
[22-2] The method according to [22-1], wherein the type I CRISPR-Cas system is a type I-E CRISPR-Cas system, optionally a CRISPR-Cas system derived from E. coli.
[23-1] The method according to [22-1] or [22-2], wherein the cell is a pluripotent stem cell, optionally an induced pluripotent stem cell.
[23-2] The method according to any one of [22-1] to [23-1], wherein the cell is a mammalian cell, optionally a human cell.
[24-1] An agent for altering a target sequence of a double-stranded DNA, comprising the CRISPR-Cas system according to [12-1] or [12-2].
[24-2] An agent for regulating a target gene expression, comprising the CRISPR-Cas system according to [12-1] or [12-2].
[25] The agent according to [24-1] or [24-2] for treatment of a disease.

### Advantageous Effects of Invention

With the present invention, it is possible to improve the efficiency of genome editing by the type I CRISPR-Cas system and to analyze a pattern of the genome editing by the system. It is also possible to provide a tool capable of concentrating genome-edited cells and to specifically conduct genome editing on one allele.

### Brief Description of Drawings

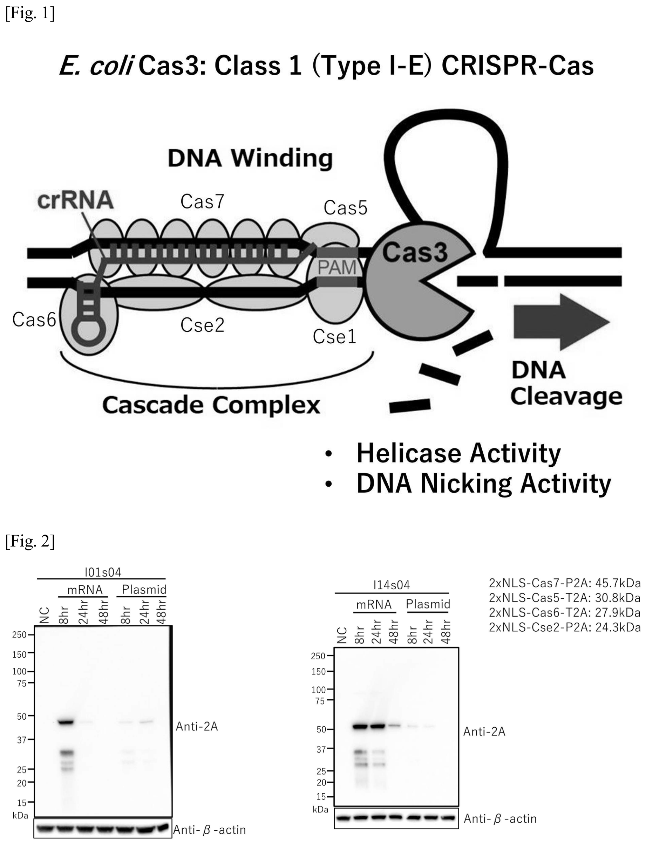

[FIG. 1] FIG. 1 shows a schematic diagram illustrating DNA cleavage by the class-1 CRISPR-Cas system. Cas3 has helicase activity and DNA nicking activity, and introduces a nick to a DNA strand on a side on which a crRNA and a Cascade complex do not bind.

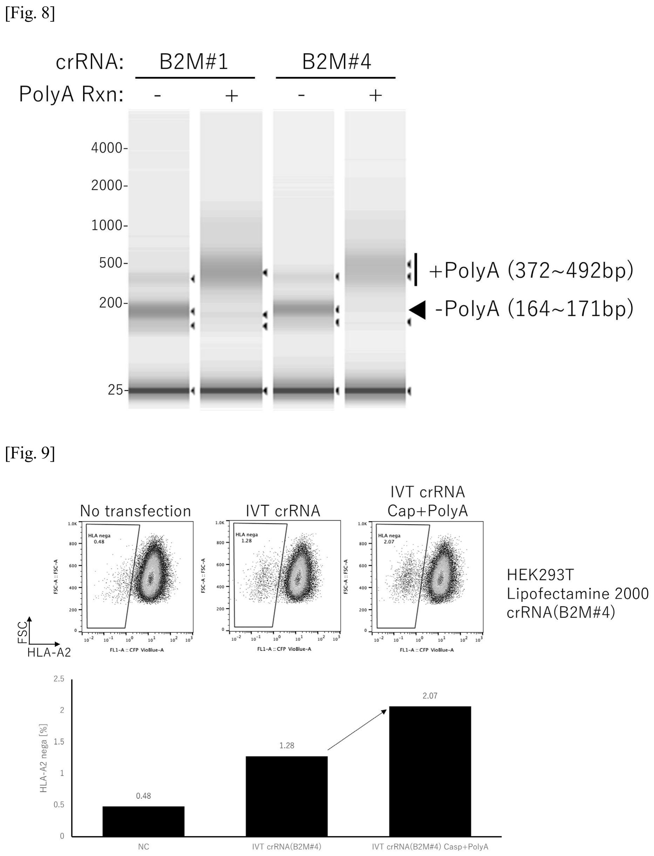

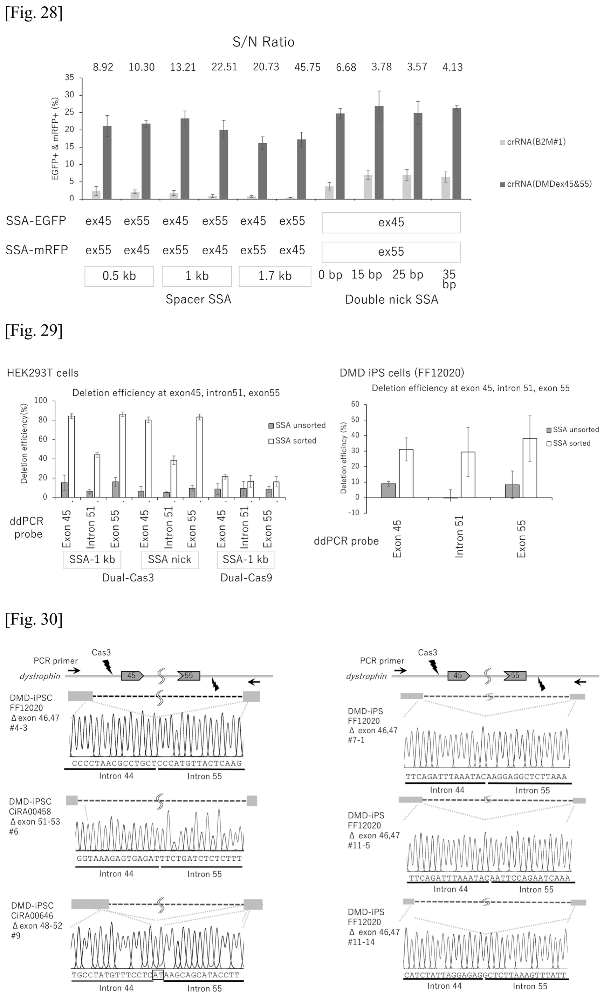

[FIG. 2] FIG. 2 shows the dynamics of expression of proteins (which may be referred to as a "Cascade protein group" hereinafter) included in the Cascade complex by an mRNA. The Cascade protein group was introduced in the form of an mRNA (FIG. 29 in Patent Literature 3) or a plasmid (FIG. 9d in Patent Literature 3) into iPS cell strains (I01s04 or I14s04) using Lipofectamine Stem. NC indicates a negative control. An anti-2A peptide antibody was used to analyze the expression of 2xNLS-Cse2-P2A, 2xNLS-Cas5-T2A, 2xNLS-Cas6-T2A, and 2xNLS-Cas7-P2A in samples 8, 24, and 48 hours after transfection.
[FIG. 3-1] FIG. 3-1 shows a schematic diagram illustrating the results of examination of RNA modification positions in an E. coli Cas3 pre-crRNA. The nucleotides denoted by * have a 2'-O-methyl 3'phosphorothioate (MS) modification. In the nucleotides having the MS modification, an O-methyl group is substituted for a hydroxy group at position 2 of the sugar moiety, and a bond between the sugar moiety of the nucleotide and the sugar moiety of the adjoining nucleotide is a phosphorothioate bond (a sulfur atom (S) is substituted for an oxygen atom at the phosphodiester bond site). The same applies hereinafter. The base sequence of the pre-crRNA in the diagram is represented by SEQ ID NO: 168.

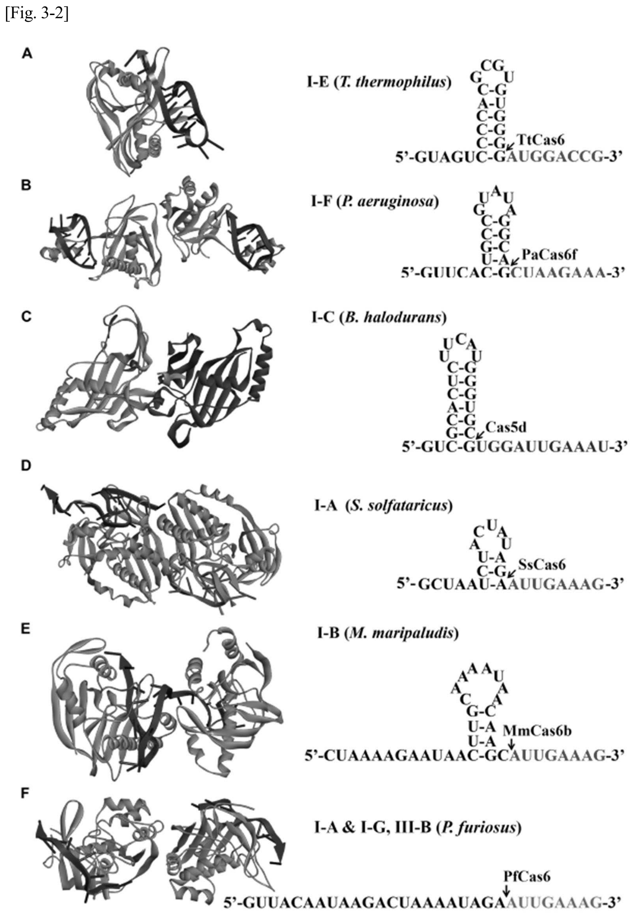

[FIG. 3-2] FIG. 3-2 shows repeat structures of pre-crRNAs other than the E. coli Cas3 pre-crRNA (Reference: Zheng Y. et al., Front Bioeng Biotechnol. 2020 Mar 4; 8: 62). The base sequences of the repeat structures in the diagram are represented by SEQ ID NOS: 169 to 174 in the order from top to bottom.

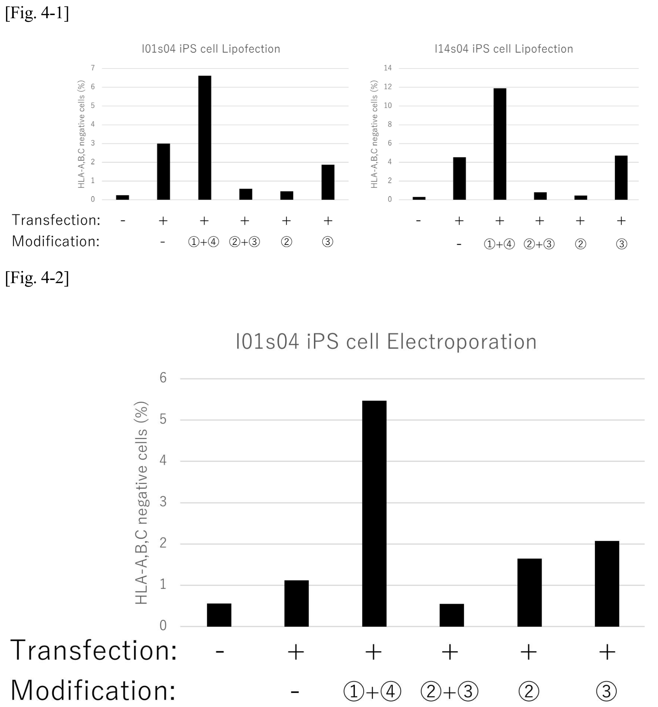

[FIG. 4-1] FIG. 4-1 shows changes in activity depending on the RNA modification position in the E. coli Cas3 pre-crRNA (lipofection using Lipofectamine Stem). The encircled numbers in the graphs correspond to the encircled numbers at the modification positions in FIG. 2-1. In I14s04 and 101s04 iPS cells, the human B2M gene was targeted and the efficiency of genome editing (B2M gene knockout) was measured through cell-surface HLA-A/B/C staining. As a result, the activity was enhanced when the modification was introduced to positions (1) and (4).
[FIG. 4-2] FIG. 4-2 shows changes in activity depending on the RNA modification position in the E. coli Cas3 pre-crRNA (electroporation using 4D-Nucleofector). The encircled numbers in the graphs correspond to the encircled numbers at the modification positions in FIG. 2-1. In I01s04 iPS cells, the human B2M gene was targeted and the genome editing efficiency was measured through HLA staining. As a result, the activity was enhanced when the modification was introduced to positions (1) and (4).

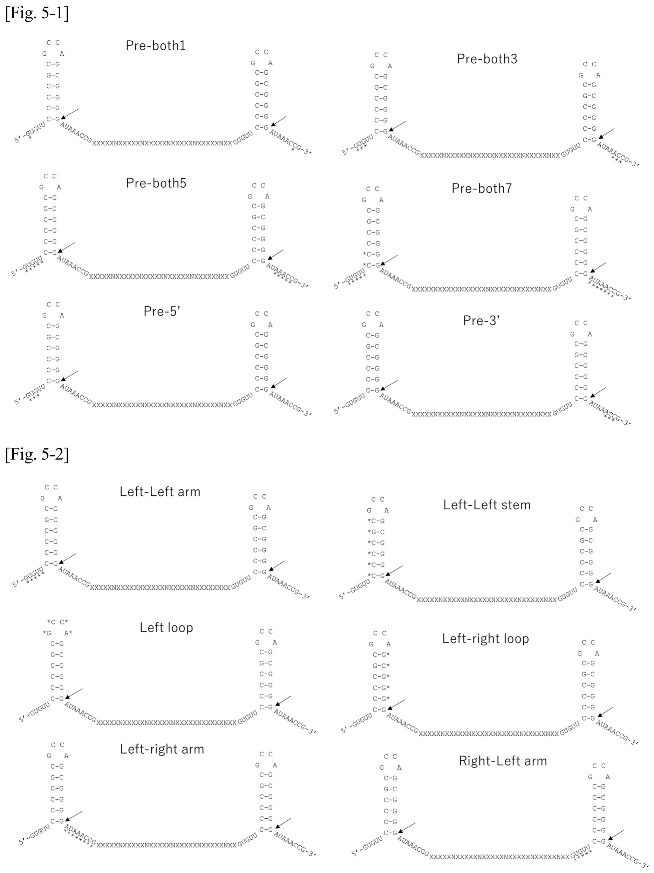

[FIG. 5-1] FIG. 5-1 shows a schematic diagram illustrating the results of additional examination of RNA modification positions in the E. coli Cas3 pre-crRNA. The nucleotides denoted by * have a 2'-O-methyl 3'phosphorothioate (MS) modification. Arrows indicate the Cas6 cleavage sites.
[FIG. 5-2] FIG. 5-2 shows a schematic diagram illustrating the results of additional examination of RNA modification positions in the E. coli Cas3 pre-crRNA. The nucleotides denoted by * have a 2'-O-methyl 3'phosphorothioate (MS) modification. Arrows indicate the Cas6 cleavage sites.

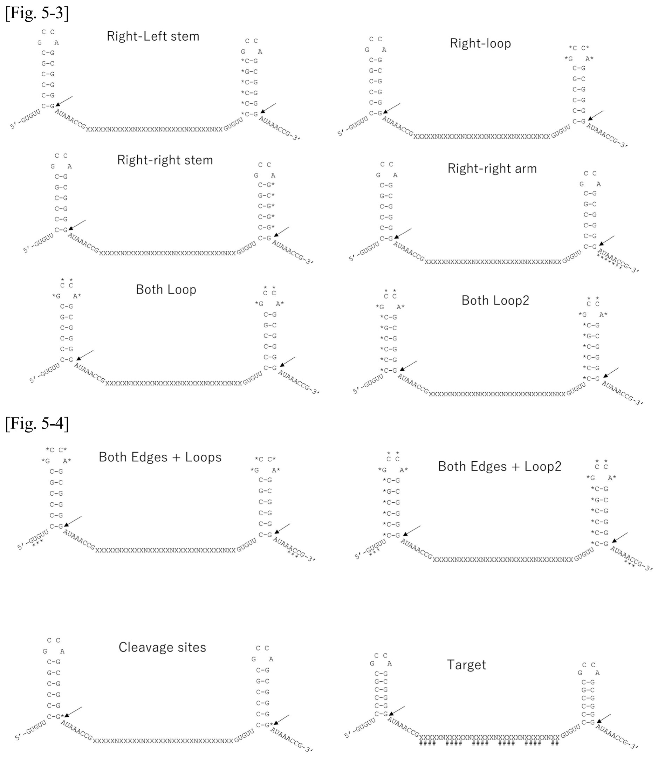

[FIG. 5-3] FIG. 5-3 shows a schematic diagram illustrating the results of additional examination of RNA modification positions in the E. coli Cas3 pre-crRNA. The nucleotides denoted by * have a 2'-O-methyl 3'phosphorothioate (MS) modification. Arrows indicate the Cas6 cleavage sites.
[FIG. 5-4] FIG. 5-4 shows a schematic diagram illustrating the results of additional examination of RNA modification positions in the E. coli Cas3 pre-crRNA. The nucleotides denoted by # have a 2'-O-methyl (M) modification, and the nucleotides denoted by * have a 2'-O-methyl 3'phosphorothioate (MS) modification. Arrows indicate the Cas6 cleavage sites.

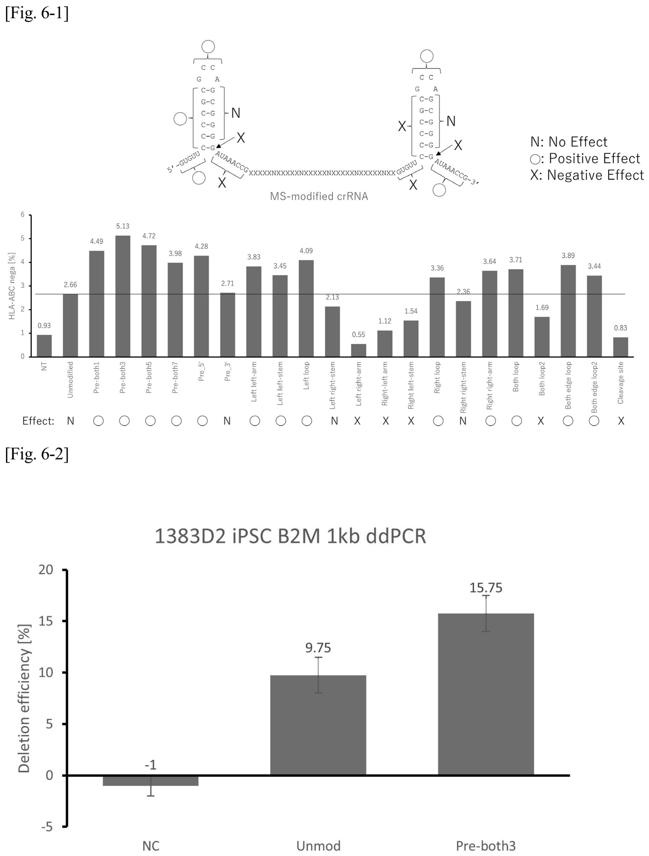

[FIG. 6-1] FIG. 6-1 shows changes in activity depending on the RNA modification positions shown in FIGS. 5-1 to 5-4 in the E. coli Cas3 pre-crRNA. mRNAs encoding Cas3 and the Cascade protein group and pre-crRNAs targeting the human B2M gene were transfected to I14s04 iPS cells using Lipofectamine Stem, and the genome editing efficiency was measured through HLA-A/B/C staining. A circle indicates that an effect of increasing the activity was exhibited, a cross indicates that an effect of reducing the activity was exhibited, and N indicates that there was little or no effect on the activity.
[FIG. 6-2] FIG. 6-2 shows the results of genome editing conducted on a different iPS cell strain (1383D2) using a crRNA (B2M#1). mRNAs for expression of Cas3 and the Cascade proteins and the crRNA were transfected using Lipofectamine Stem, and the gene deletion efficiency was measured through ddPCR. n = 2; error bar: standard deviation. The target sequence was a sequence represented by SEQ ID NO: 4.

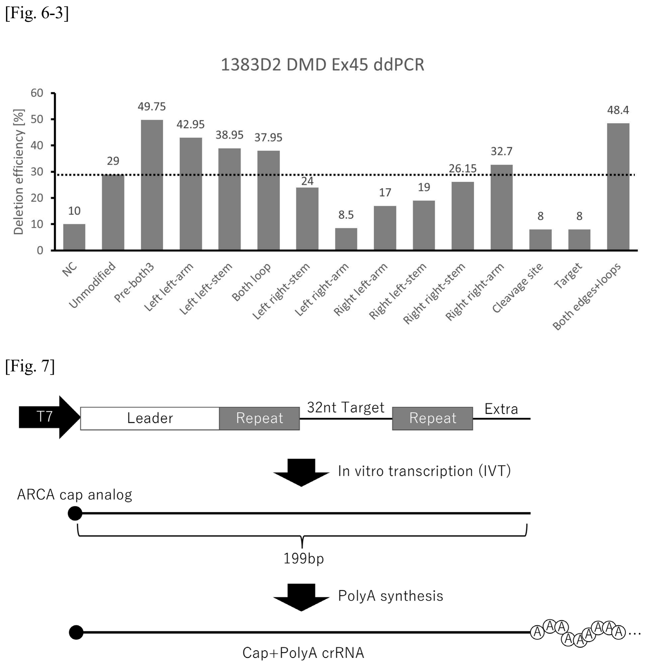

[FIG. 6-3] FIG. 6-3 shows changes in activity depending on the RNA modification positions shown in FIGS. 5-1 to 5-4 in the E. coli Cas3 pre-crRNA. mRNAs encoding Cas3 and the Cascade protein group and pre-crRNAs targeting the exon 45 (Ex45) of the human dystrophin (DMD) gene were transfected to 1383D2 iPS cells using Lipofectamine Stem, and the genome editing efficiency was measured through ddPCR. The target sequence was a sequence represented by SEQ ID NO: 55.
[FIG. 7] FIG. 7 shows a schematic diagram illustrating addition of a 5'-Cap analog and a poly-A tale to the E. coli Cas3 pre-crRNA. A pre-crRNA with an ARCA (anti-reverse cap analog) cap analog was synthesized from a plasmid with the T7 promoter encoding the pre-crRNA through an in-vitro transcription (IVT) reaction, and poly-A was added to the 3' terminus of the obtained RNA.
[FIG. 8] FIG. 8 shows the results of a TapeStation analysis using a pre-crRNA obtained by adding a 5'-Cap and a poly-A tale to the E. coli Cas3 pre-crRNA. In the IVT reaction of pre-crRNAs (B2M#1 and B2M#4) using mMESSAGE mMACHINE T7 Ultra Transcription Kit, PolyA Rxn+ was a sample obtained through PolyA addition using E. coli Poly(A) Polymerase (E-PAP) and ATP, and PolyA Rxn- was a sample before the PolyA addition.
[FIG. 9] FIG. 9 shows the effect of the addition of a 5'-Cap analog and a poly-A tale to the E. coli Cas3 pre-crRNA in HEK293T cells. An mRNA for expression of Cas3 and the Cascade protein group and the pre-crRNA (B2M#4) were introduced to HEK293T cells using Lipofectamine 2000. It was found that the pre-crRNA with the 5'-Cap analog and the poly-A tale exhibited a higher genome editing effect compared with pre-crRNAs without the 5'-Cap analog and the poly-A tale.

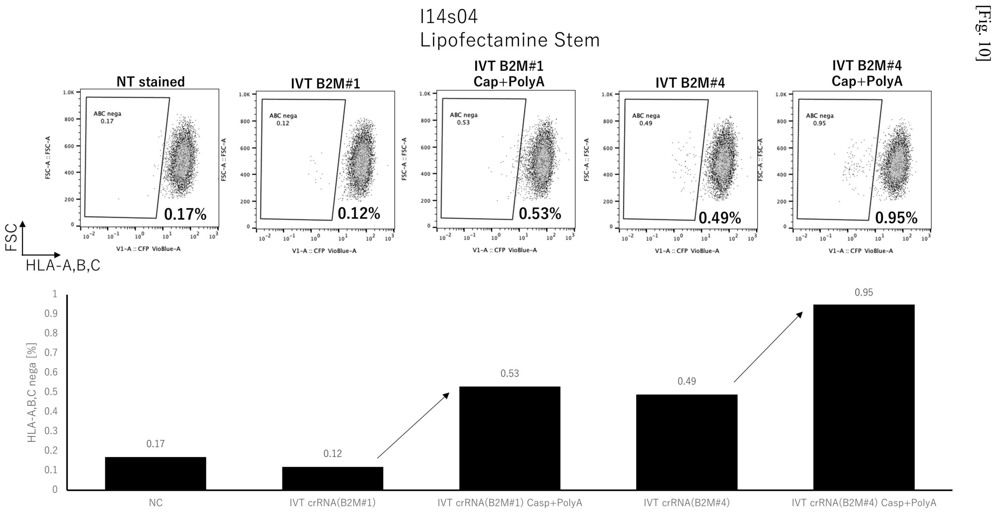

[FIG. 10] FIG. 10 shows the effect of the addition of a 5'-Cap analog and a poly-A tale to the E. coli Cas3 pre-crRNA in the I14s04 strain. An mRNA for expression of Cas3 and the Cascade protein group and the pre-crRNA (B2M#1 or B2M#4) were introduced to the I14s04 strain using Lipofectamine Stem. It was found that the pre-crRNA with the 5'-Cap analog and the poly-A tale exhibited a higher genome editing effect compared with pre-crRNAs without the 5'-Cap analog and the poly-A tale.

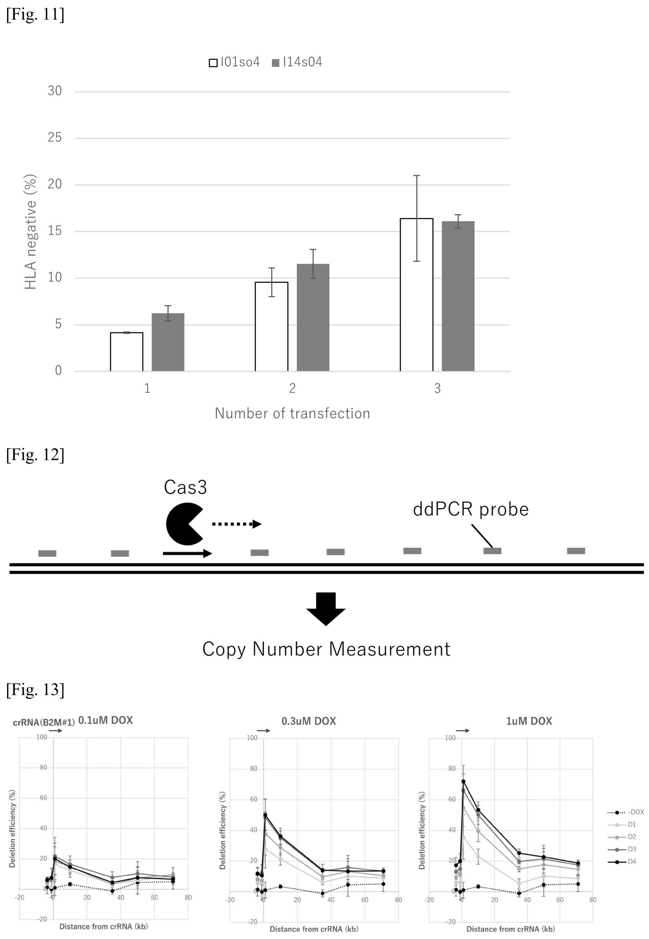

[FIG. 11] FIG. 11 shows the accumulation of the effect caused by conducting Cas3 genome editing a plurality of times. An mRNA for expression of Cas3 and the Cascade protein group and Pre-both3 pre-crRNA (FIG. 5-1) targeting the B2M gene were transfected a plurality of times to I01s04 and I14s04 iPS cells using Lipofectamine Stem. It was found from the results of HLA staining that the genome editing efficiency increased in correlation to the number of transfection. (I01s04: n = 2, I14s04 n = 3, Mean ± SD)
[FIG. 12] FIG. 12 shows a schematic diagram illustrating a technique for analyzing a Cas3 deletion induction pattern through ddPCR.
[FIG. 13] FIG. 13 shows the results of the analysis of the Cas3 genome editing pattern in HEK293T cells using ddPCR. HEK293T cells (Stable strain) in which a pPV-C1-crRNA(B2M#1)-EF1a-G2ABA piggyBac vector for expression of a pre-crRNA targeting the B2M gene and a pPV-TRE3G-Bi(263+751)-EF1a-Tet3G-iPA piggyBac vector capable of adjusting the expression of Cas3 and the Cascade protein group with DOX were stably integrated into the genome DNA were used to express Cas3 and the Cascade protein group through DOX induction for a plurality of days (zero days, one day, two days, three days, or four days) with the concentration of DOX being varied (0.1 µM, 0.3 µM, or 1 µM), and the results obtained are shown. The genome DNA copy numbers at positions about -3.5 kb, -1 kb, 1 kb, 10 kb, 35 kb, 50 kb, and 70 kb away from the crRNA (B2M#1) target sequence were measured through ddPCR (n = 3, mean ± SD). It was shown that the pattern of genome editing by the CRISPR-Cas3 system changed depending on the DOX concentration. "uM" in the diagram means "µM". The same applies hereinafter.

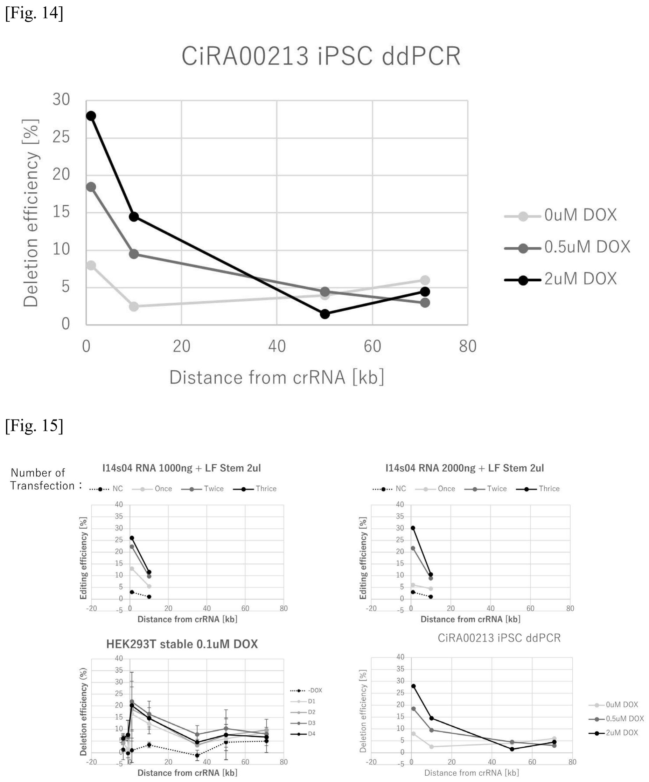

[FIG. 14] FIG. 14 shows the results of the analysis of the Cas3 genome editing pattern in iPS cells using ddPCR. CiRA00213 iPS cells (Stable strain) in which a pPV-C1-crRNA(B2M#1)-EF1a-G2AZA vector for expression of a pre-crRNA targeting the B2M gene and a pPV-TRE3G-Bi(263+751)-EF1a-Tet3G-iPA vector capable of adjusting the expression of Cas3 and the Cascade protein group with DOX were integrated into the genome DNA were used, and the genome DNA copy numbers at positions about 1 kb, 10 kb, 50 kb, and 70 kb away from the crRNA (B2M#1) were measured through ddPCR. As a result, it was shown that the pattern of genome editing by the CRISPR-Cas3 system changed depending on the concentration (0, 0.5, 2 µM) of the DOX allowed to act for nine days.
[FIG. 15] FIG. 15 shows deletion induction patterns when Cas3 genome editing was conducted using RNAs. The upper graphs show the results obtained when the CRISPR-Cas3 system was introduced in the form of an RNA to I14s04 iPS cells (once, twice, or three times) (the copy numbers at positions about 1 kb and 10 kb away from the crRNA target sequence were measured through ddPCR), and the lower graphs show the results obtained when Cas3 and the Cascade protein group were expressed in HEK293T cells and I14s04 iPS cells through DOX induction for a plurality of days (zero days, one day, two days, three days, or four days) or at a different DOX concentration (0, 0.5, 2 µM) (parts thereof are taken from FIGS. 13 and 14 for comparison). LF Stem is an abbreviation for Lipofectamine Stem. "ul" in the diagram means "µl". The same applies hereinafter.

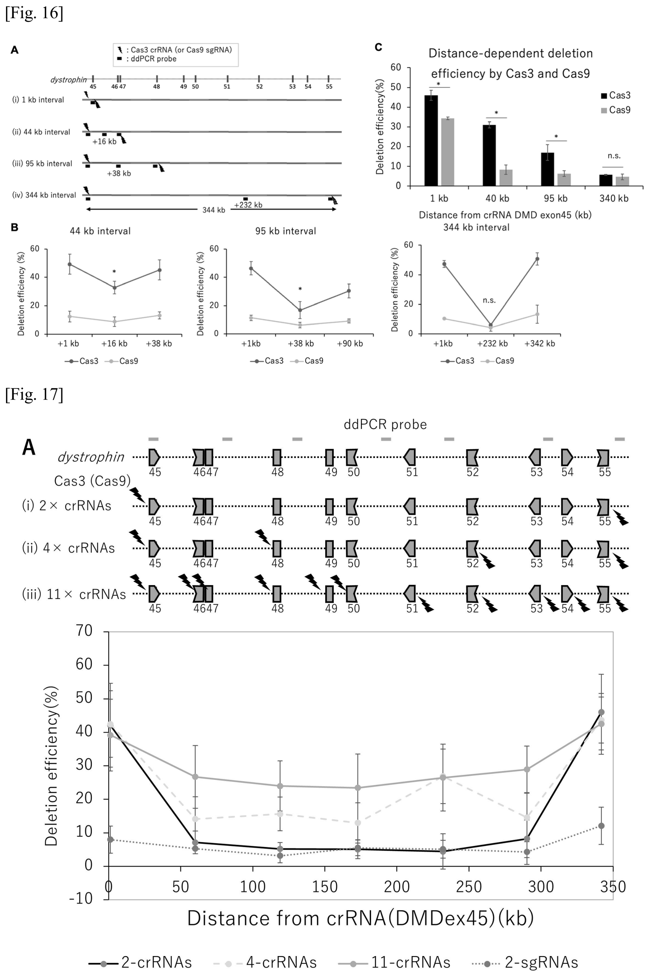

[FIG. 16] FIG. 16 shows the results of comparison of the induction efficiencies of long-chain DNA deletion by Cas3 and Cas9 as applications of the ddPCR analysis. FIG. 16A shows a schematic diagram of the experiment. crRNAs (or sgRNAs) were designed such that a region (target region) to be sandwiched between two types of Cas3 crRNAs (or Cas9 sgRNAs) on different strands in the dystrophin gene locus has a length of (i) 1 kb, (ii) 44 kb, (iii) 95 kb, or (iv) 344 kb, and a ddPCR probe was designed at near the center of each target region. FIG. 16B shows graphs illustrating the results of analysis of deletion frequencies at both ends of the target region (near the crRNAs) and near the center of the target region in (ii) to (iv) (means ± SD, n = 4, *: p < 0.05). FIG. 16C shows graphs illustrating the deletion frequency at near the center in (i) to (iv) (means ± SD, n = 4, *: p < 0.05).
[FIG. 17] FIG. 17 shows the results of evaluation of the gene deletion ratio at each site in Cas3 genome editing using two, four, or eleven types of crRNAs (or Cas9 sgRNAs) as applications of the ddPCR analysis. Gene transfer of a Cas3 vector (All-in-one-Puro) and a crRNA vector (or a Cas9 vector (Cas9-Puro) and an sgRNA vector) to HEK293T cells was conducted through lipofection using Lipofectamine 2000. ddPCR probes were designed for seven positions located at regular intervals in the exon 45-55 region of the dystrophin gene, and the deletion frequencies were analyzed (means ± SD, n = 3, *: p < 0.05).

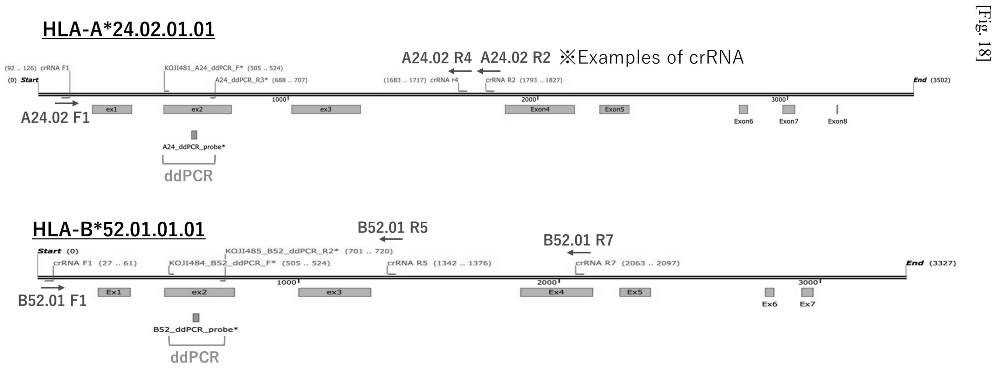

[FIG. 18] FIG. 18 shows a schematic diagram illustrating a method of conducting genome editing on HLA-A*24:02 and B*52:01 in iPSC (I01s04 and I14s04) derived from homodonors with the Japan's most frequent HLA. The sequence of AAG+32nt was extracted and converted into AAG+XXXXXNXXXXXNXXXXXNXXXXXNXXXXXNXX, and then off-target prediction (i.e., examination of whether or not exactly the same sequence is present at another site, particularly whether or not the same sequence is present in another HLA gene) was conducted using GGGenome. Thereafter, the distance and the direction were narrowed down as if clusters in the exons 1 to 3 were trimmed. crRNA sequences (A24:02 F1/R2/R4, B52:01 F1/R5/R7) are examples thereof. ddPCR probes and primers for measurement of the genome editing efficiency were designed on the exon 2 of each gene.

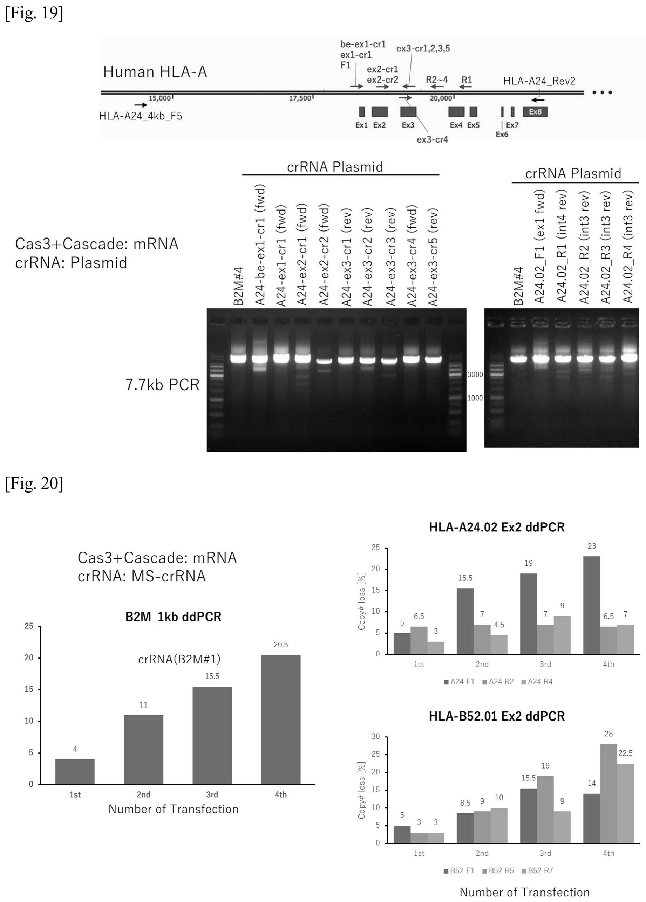

[FIG. 19] FIG. 19 shows the results of activity measurement conducted through PCR (in the case of a crRNA targeting HLA-A*A24:02). Lipofectamine Stem was used to express mRNAs for expression of Cas3 and the Cascade protein group and the crRNA expression vector (pBSIIKS-U6-crRNA or pPV-C1-crRNA) in I14s04 iPS cells, and the obtained genome sample was analyzed through PCR (LA Taq HS). The bands with a size of smaller than 7.7 kb correspond to samples with deletion by Cas3 genome editing.
[FIG. 20] FIG. 20 shows the results of measurement of the exon 2 deletion efficiency of HLA-A*24:02 and B*52:01 through ddPCR (single crRNA). An mRNA for expression of Cas3 and the Cascade protein group and Pre-both3 pre-crRNA (FIG. 5-1) targeting the B2M gene, HLA-A*24:02 and B*52:01 were transfected a plurality of times to I14s04 iPS cells using Lipofectamine Stem, and collected genome samples were analyzed through ddPCR. The experiment using crRNA (B2M#1) was a control experiment (left diagram).

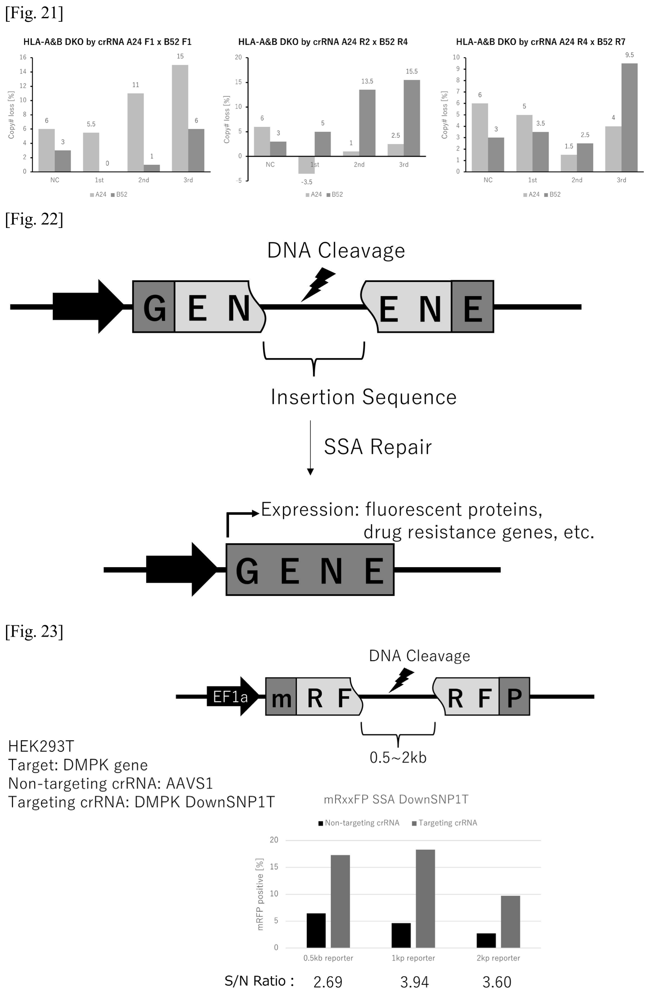

[FIG. 21] FIG. 21 shows the results of measurement of the exon 2 deletion efficiency through ddPCR (double crRNA). Two types of pre-both3 pre-crRNAs, one targeting HLA-A*24:02 and one targeting HLA-B*52:01, were added in the method shown in FIG. 20.
[FIG. 22] FIG. 22 shows a schematic diagram of a single-strand annealing (SSA) reporter vector. In cells with activated Cas, DNA repair via SSA occurs after cleavage of an insertion sequence by the Cas, and a reporter gene with a protein activity is consequently expressed.
[FIG. 23] FIG. 23 shows the results of comparison of the SSA reporter activity depending on the length of the insertion sequence. In HEK293T cells in which the expression of Cas3 and the Cascade protein group can be induced through the addition of DOX, a non-target crRNA (AAVS1#1) or a target crRNA (DMPK DownSNP1T) and an mRFP-SSA vector (for expression of mRFP through SSA) that includes the gene sequence of target DMPK (DMPK DownSNP1T) were introduced while DOX was added, and the ratio of mRFP-positive cells was measured through flow cytometry after three days. The SSA insertion sequences that include the target sequence and have different lengths, namely about 0.5, 1, and 2 kb, were compared.

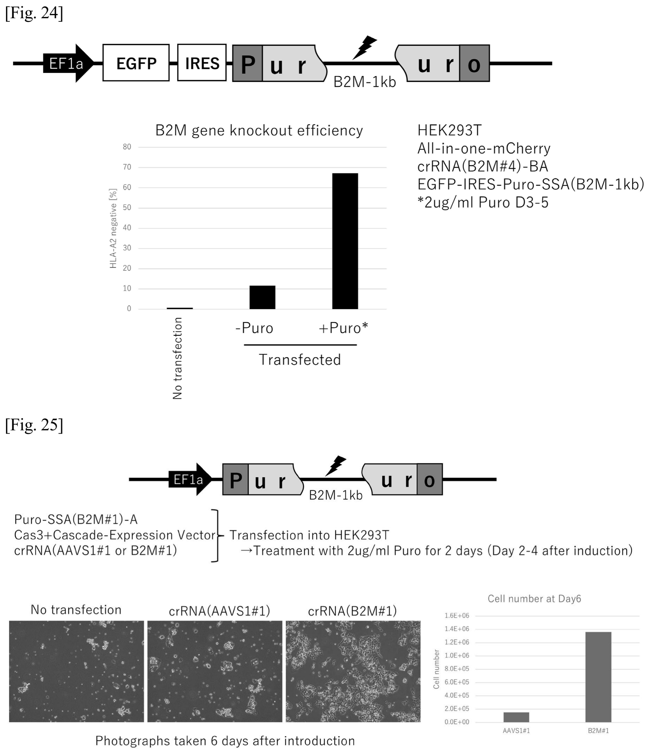

[FIG. 24] FIG. 24 shows about 6-fold concentration of gene-deleted cells due to a Puro-SSA reporter vector. HEK293T cells were used, pPV-Dual_Promoter-EF1a-2xNLS-Cascade+Cas3(RD)-iCA (All-in-one-mCherry) was used as the vector for expression of Cas3 and the Cascade protein group, pPV-C1-crRNA(B2M#4)-EF1a-BA was used as the crRNA, and pHL-EF1a-EGFP-IRES-Puro-SSA(B2M-1kb)-A was used as the SSA reporter vector. 2 µg/ml of puromycin was added three to five days after the transfection (+Puro sample), and the efficiency of B2M deletion by genome editing was measured through HLA-A2 staining. A -Puro sample is a sample that did not undergo puromycin treatment. "ug" in the diagram means "µg". The same applies hereinafter.
[FIG. 25] FIG. 25 shows the results of confirmation of the specificity of the Puro-SSA reporter vector. All-in-one-mCherry serving as the vector for expression of Cas3 and the Cascade proteins, pPV-C1-crRNA(AAVS1#1)-EF1a-BA (control crRNA) or pPV-C1-crRNA(B2M#1)-EF1a-BA serving as the crRNA expression vector, and pHL-EF1a-Puro-SSA(B2M-1kb)-A serving as the Puro-SSA reporter vector were transfected to HEK293T cells, and 2 µg/ml puromycin was added two to four days after the transfection. The numbers of cells six days after the transfection in the photographs were measured. As a result, it was shown that puromycin resistance could be acquired due to the sequence-specific activity of Cas3.

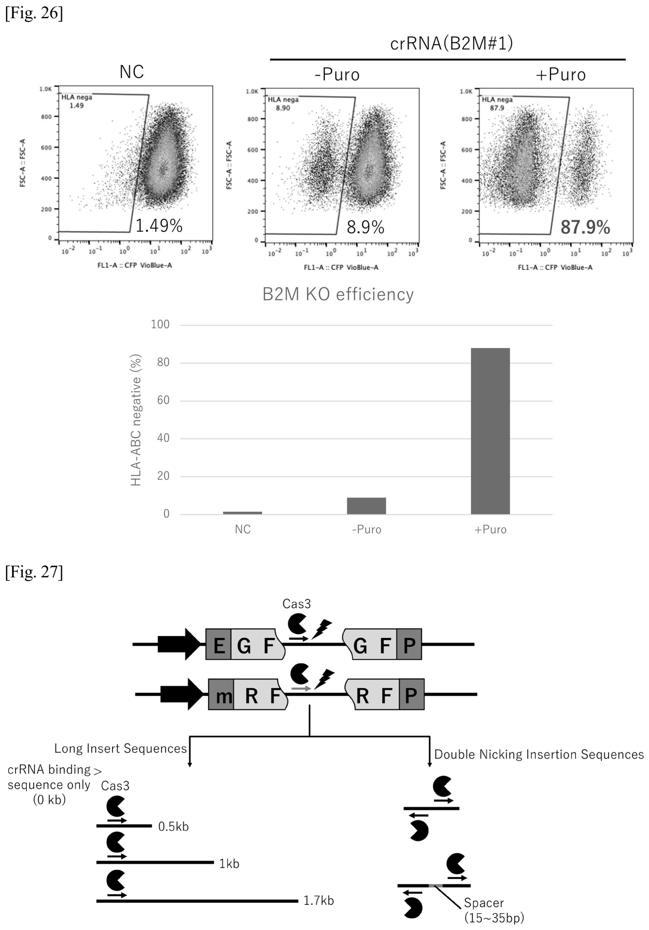

[FIG. 26] FIG. 26 shows that B2M-deleted cells could be concentrated about 10-fold due to the Puro-SSA reporter vector. The ratio of B2M-deleted cells in the cells in FIG. 25 was measured through HLA staining.
[FIG. 27] FIG. 27 shows a schematic diagram illustrating bicolor SSA reporters (SSA-EGFP and SSA-mRFP) and a double nicking SSA reporter vector. Insertion sequences were produced using a long sequence (also referred to as a "long-chain sequence") (left) and a double nicking insertion sequence (right).
[FIG. 28] FIG. 28 shows the results of examination of insertion sequences for the bicolor SSA reporter vectors (SSA-EGFP and SSA-mRFP). Gene transfer of a Cas3 expression vector (All-in-one-Puro) and a crRNA expression vector (DMDex45 and 55 was used as the target crRNA, and B2M#1 was used as the non-target crRNA) to HEK293T cells was conducted through lipofection using Lipofectamine 2000. SSA-EGFP for expression of EGFP through SSA and SSA-mRFP for expression of mRFP through SSA were used as the SSA reporter vectors. Whichever fluorescent reporter and target sequence were used, EGFP fluorescence and mRFP fluorescence, which are high on-target signals, were observed when co-transfection with an appropriate crRNA was conducted. (means ± SD, n = 3, *: p < 0.05).
[FIG. 29] FIG. 29 shows concentration of genome-edited cells by the bicolor SSA reporter vectors (SSA-EGFP and SSA-mRFP). Both HEK293T cells (left) and iPS cells derived from a DMD patient (FF12020: right) could be used to concentrate genome-edited cells. Gene transfer of a Cas3 vector (All-in-one-Puro) and a crRNA vector (DMDex45&55), or a Cas9 vector (Cas9-Puro) and a crRNA vector, and the SSA reporter vectors (SSA-EGFP and SSA-mRFP) to HEK293T cells was conducted through lipofection using Lipofectamine 2000. Gene transfer of the Cas3 vector (All-in-one-Puro), the crRNA vector (DMDex45&55), and the SSA reporter vectors (SSA-EGFP and SSA-mRFP) to iPS cells derived from a DMD patient was conducted through lipofection using Lipofectamine Stem (means ± SD, n = 3, *: p < 0.05). Cells positive for both EGFP and mRFP were subjected to cell sorting, and ddPCR was conducted at positions of DMD Ex45, Int51, and Ex55 of the obtained cellular genome.
[FIG. 30] FIG. 30 shows the results of DNA sequencing of subclones of iPS cells derived from a DMD patient obtained through cell concentration by the bicolor SSA reporter vectors (SSA-EGFP and SSA-mRFP). 24 hours after the transfection using Lipofectamine Stem, iPS cells derived from a DMD patient (FF12020: with deletion of exons 46 and 47, CiRA00458: with deletion of exons 51 to 53, CiRA00646: with deletion of exons 48 to 52) were subjected to drug selection using puromycin for two days, and then EGFP/mRFP double positive cells were sorted using a cell sorter to obtain subclones. For genotyping, the exon 45-55 region of the dystrophin gene was subjected to PCR, and then it was confirmed through Sanger sequencing whether or not deletion was present. As a result, subclones of iPS cells with the exon 45-55 region removed were obtained. The base sequences in the left diagram are represented by SEQ ID NOS: 175 to 177 in the order from top to bottom, and the base sequences in the right diagram are represented by SEQ ID NOS: 178 to 180 in the order from top to bottom.

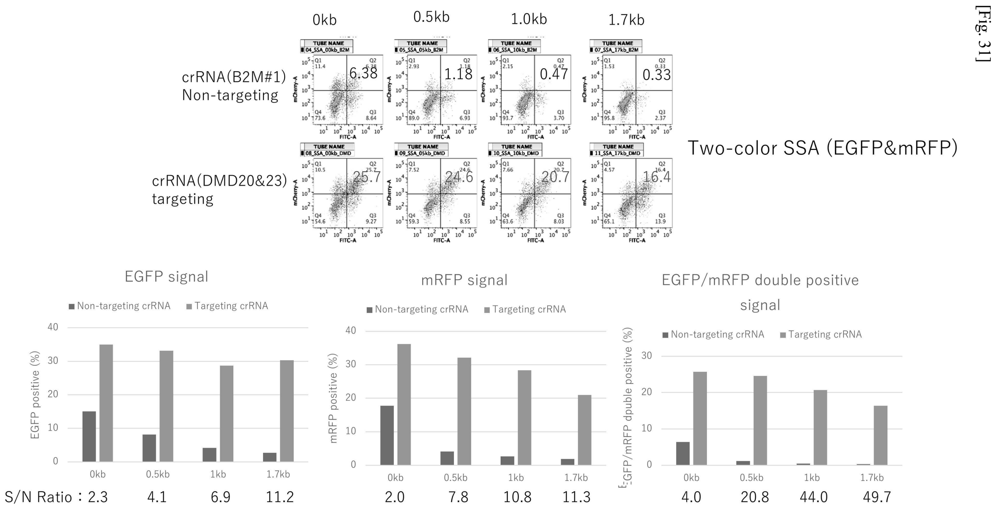

[FIG. 31] FIG. 31 shows the results of comparison of the S/N ratio with an SSA reporter vector having a conventional short insertion sequence. Gene transfer of the Cas3 vector (All-in-one-Puro), the crRNA vectors (DMDex45&55 was used as the target crRNA, and B2M#1 was used as the non-target crRNA), and SSA-EGFP and SSA-mRFP serving as the SSA reporter vectors to HEK293T cells was conducted through lipofection using Lipofectamine 2000, and the ratio of cells positive for EGFP and mRFP was measured through flow cytometry. The SSA insertion sequence had a length of 0 kb (only the target sequence), 0.5 kb, 1.0 kb, or 1.7 kb.

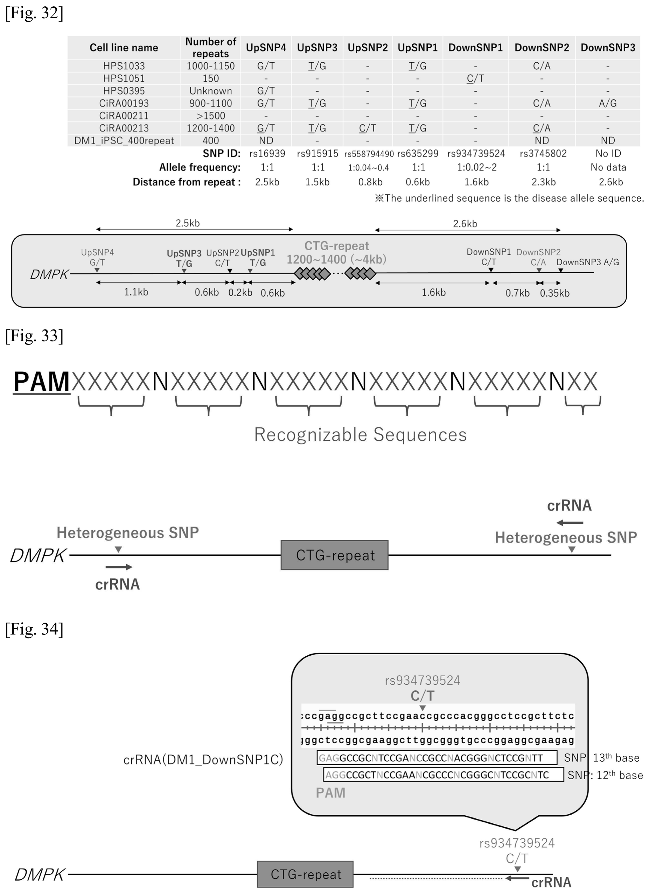

[FIG. 32] FIG. 32 shows a list of hetero SNPs around the CTG repeat in iPS cells derived from various DM1 patients. The underlined sequence is a target disease allele sequence. ND: not analyzed.
[FIG. 33] FIG. 33 shows a schematic diagram illustrating a method for designing an allele-specific crRNA. In the target sequence, crRNA recognizable sequences are illustrated using X.
[FIG. 34] FIG. 34 shows the results of the design of an allele-specific crRNA (DMPK CTG repeat upstream: DM1_DownSNP1C). The base sequences in the diagram are represented by SEQ ID NOS: 181 to 184 in the order from top to bottom. The crRNA (DM1_DownSNP1C_12nt) in which a SNP is present at base position 12 was a control crRNA with no allele specificity.

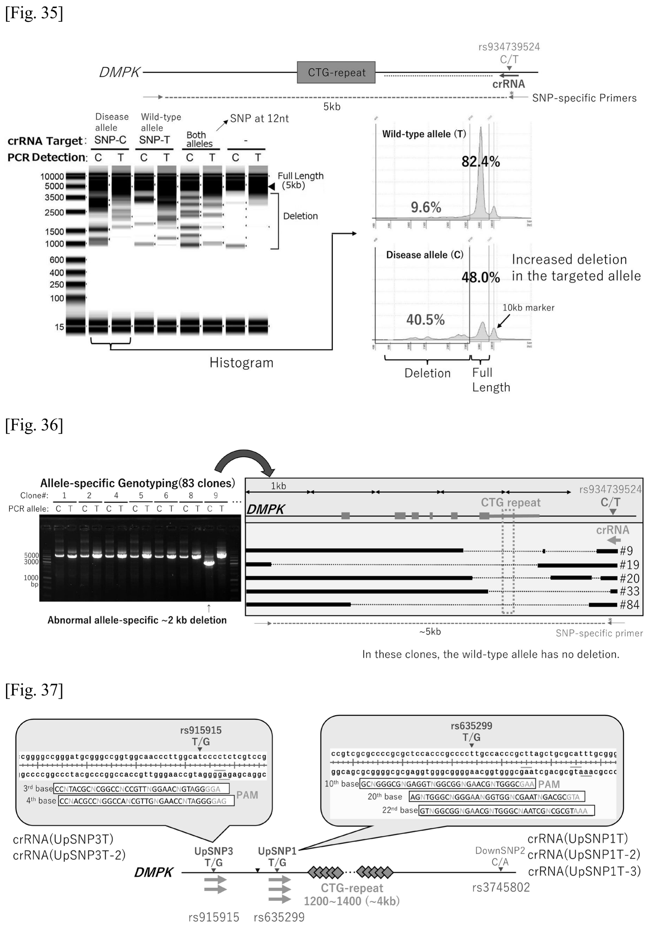

[FIG. 35] FIG. 35 shows the results of evaluation of the activity of allele-specific crRNAs (DM1_DownSNP1C/T and DM1_DownSNP1C_12nt). In iPS cells HPS1051 derived from a DM1 patient with a hetero SNP (C/T) of rs934739524, a pPV-Dual_Promoter-EF1a-2xNLS-Cascade+Cas3(RD)-iPA vector for expression of Cas3 and the Cascade protein group and pPV-C1-crRNA-EF11-G2ABA vector for expression of the pre-crRNAs were integrated into the genome (stable strain). The genome of each of these cell samples was amplified through SNP-specific (allele-specific) PCR and was analyzed using TapeStation. A histogram in the case where a crRNA (DownSNP1C) was used and a disease allele was targeted is shown as an example (right diagram).
[FIG. 36] FIG. 36 shows that using the allele-specific crRNA (DMPK CTG repeat downstream) made it possible to obtain subclones in which the CTG repeat is removed in only the disease allele. The cell population produced in FIG. 35 were subjected to single-cell cloning, and then genotyping was conducted. As a result, subclones #9, #19, #20, #33, and #84 in which the CTG repeat region is deleted in only the disease allele (DownSNP1C) were obtained.
[FIG. 37] FIG. 37 shows the results of the design of an allele-specific crRNA (DMPK CTG repeat upstream). The base sequences in the diagram are represented by SEQ ID NOS: 185 to 188 (rs915915: UpSNP3) and SEQ ID NOS: 189 to 193 (rs635299: UpSNP1) in the order from top to bottom.

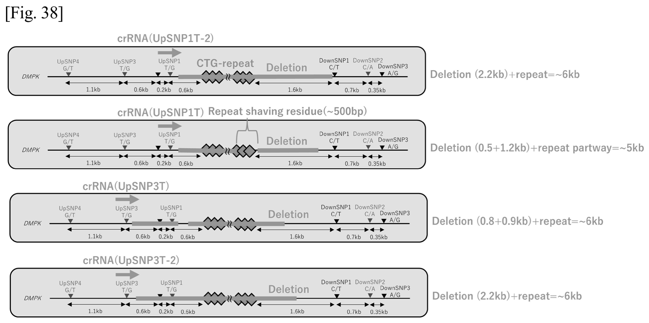

[FIG. 38] FIG. 38 shows the outline of the results of evaluation of the activity of the allele-specific crRNAs (DMPK CTG repeat upstream). iPS cells CiRA00213 derived from a DM1 patient with hetero SNPs of rs915915 (UpSNP3T/G) and rs635299 (UpSNP1T/G) were used to produce CiRA00213 iPS cells (Stable strain) in which a pPV-C1-crRNA-EF1a-BA vector for expression of a pre-crRNA targeting the DMPK CTG repeat shown in FIG. 37 and a pPV-TRE3G-Bi(263+751)-EF1a-Tet3G-iPA vector capable of inducing the expression of Cas3 and the Cascade protein group with DOX were integrated into the genome DNA, and genome-edited genome samples were analyzed thorough allele-specific nested PCR and Sanger sequencing.

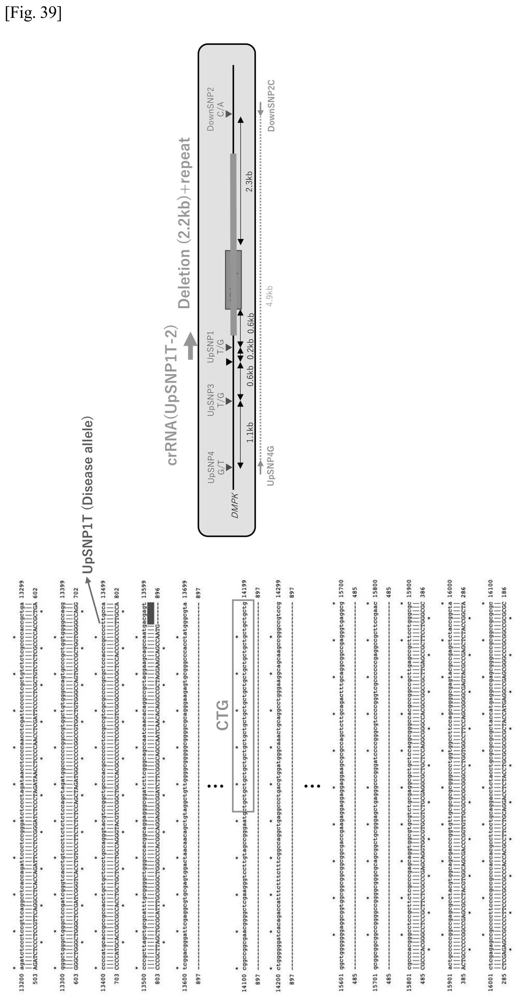

[FIG. 39] FIG. 39 shows details of the results obtained when the crRNA (UpSNP1T-2) was used (plasmid-based genome editing). Bands obtained through allele-specific nested PCR were analyzed through Sanger sequencing. As a result of alignment of the reference sequence (upper sequence) and the sequence of a genome-edited sample (lower sequence), it was found that extended CTG repeat and another sequence of about 2.2 kb were deleted. At this time, it was shown that the disease allele underwent genome editing because the sequence of UpSNP1 (rs635299) is "T".

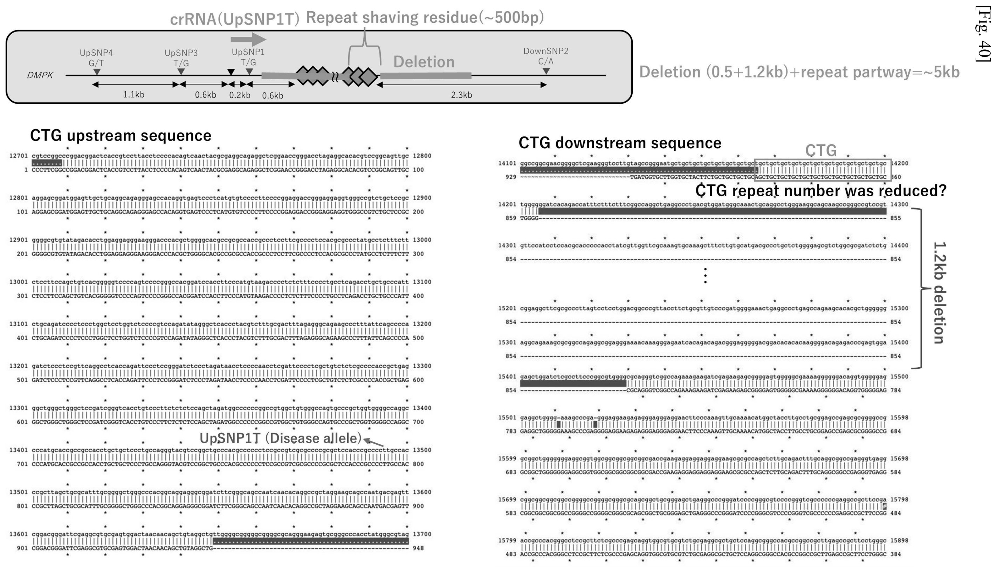

[FIG. 40] FIG. 40 shows details of the results obtained when the crRNA (UpSNP1T) was used. Plasmid-based genome editing was conducted, and bands obtained through allele-specific nested PCR were analyzed through Sanger sequencing.

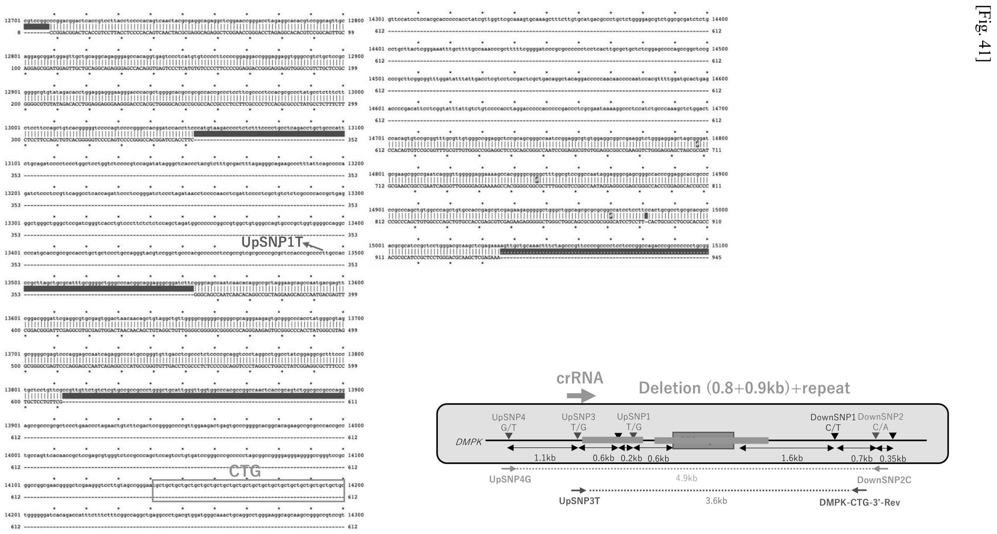

[FIG. 41] FIG. 41 shows details of the results obtained when the crRNA (UpSNP3T) was used. Plasmid-based genome editing was conducted, and bands obtained through allele-specific nested PCR were analyzed through Sanger sequencing.

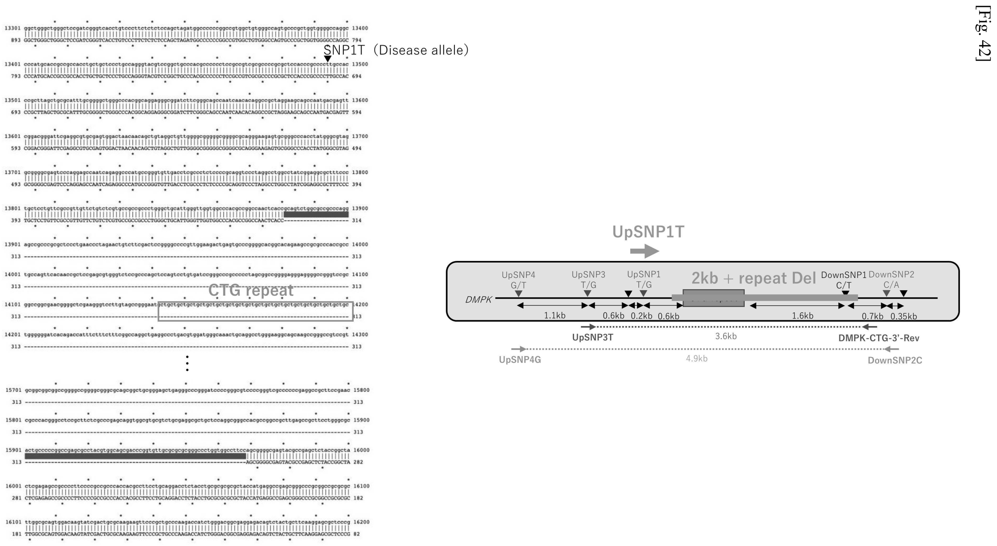

[FIG. 42] FIG. 42 shows details of the results obtained when the crRNA (UpSNP1T) was used. RNA-based Cas3 genome editing was conducted, and bands obtained through allele-specific nested PCR were analyzed through Sanger sequencing.

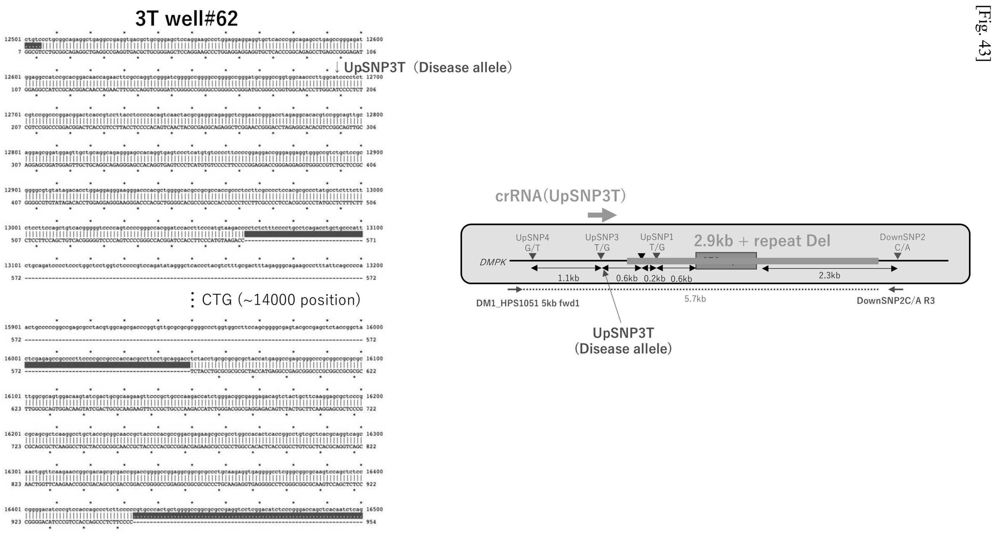

[FIG. 43] FIG. 43 shows details of the results obtained when the crRNA (UpSNP3T) was used. RNA-based Cas3 genome editing was conducted, the cells were isolated into about 10 cells/well, and bands obtained from these samples through allele-specific PCR were analyzed through Sanger sequencing (UpSNP3T well #62).

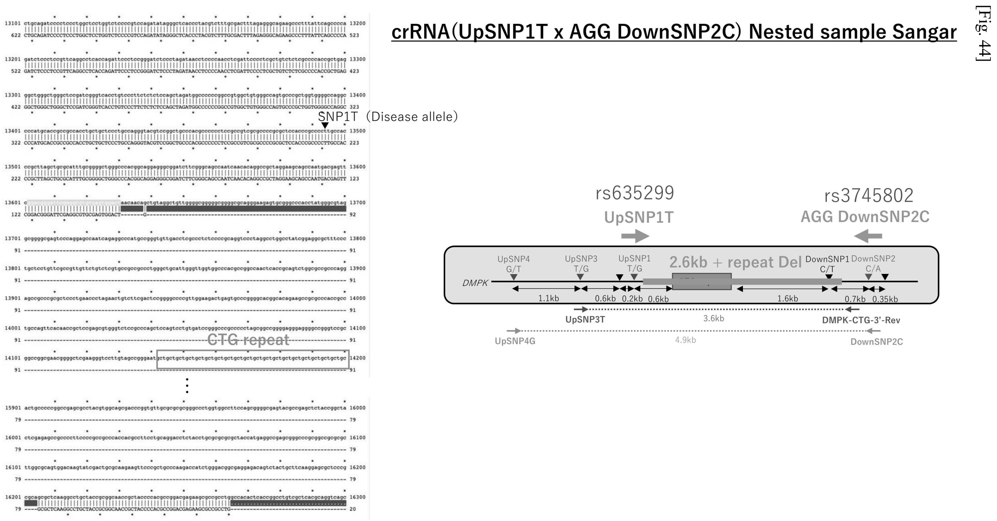

[FIG. 44] FIG. 44 shows the results of removal of the CTG repeat by allele-specific dual-Cas3 (example 1). RNA-based Cas3 genome editing was conducted, and bands obtained through allele-specific nested PCR were analyzed through Sanger sequencing.

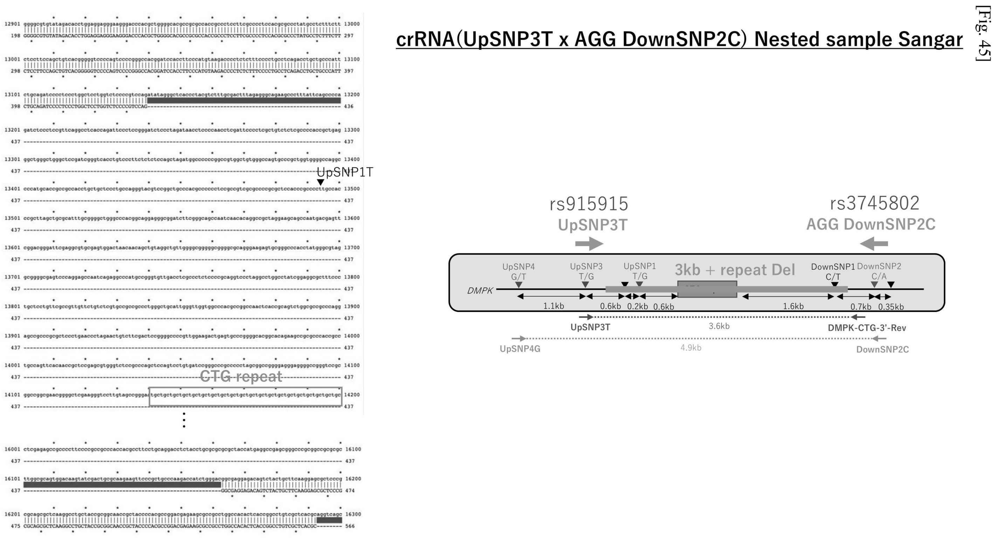

[FIG. 45] FIG. 45 shows the results of removal of the CTG repeat by allele-specific dual-Cas3 (example 2). RNA-based Cas3 genome editing was conducted, and bands obtained through allele-specific nested PCR were analyzed through Sanger sequencing.

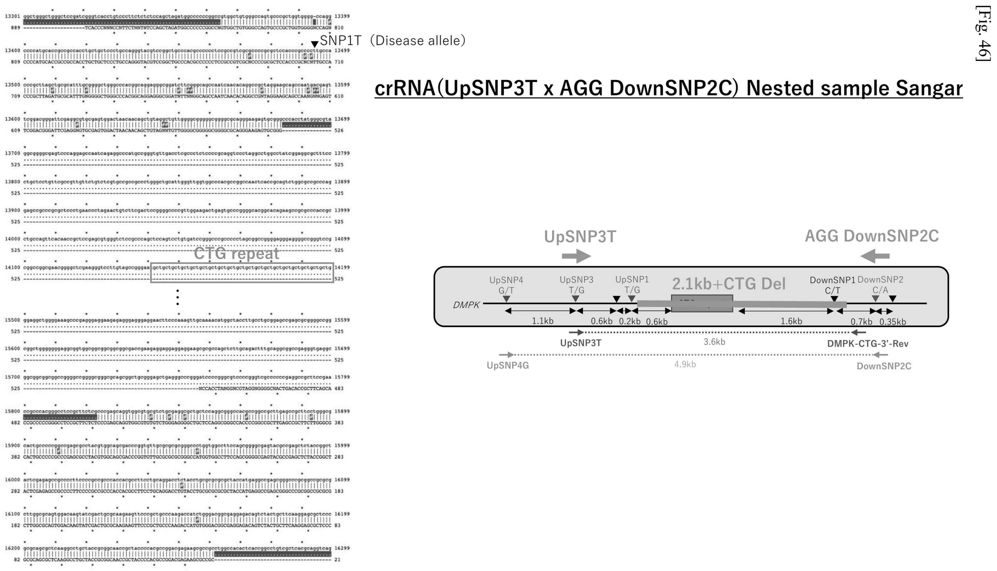

[FIG. 46] FIG. 46 shows the results of removal of the CTG repeat by allele-specific dual-Cas3 (example 2). Plasmid-based Cas3 genome editing was conducted, and bands obtained through allele-specific nested PCR were analyzed through Sanger sequencing.

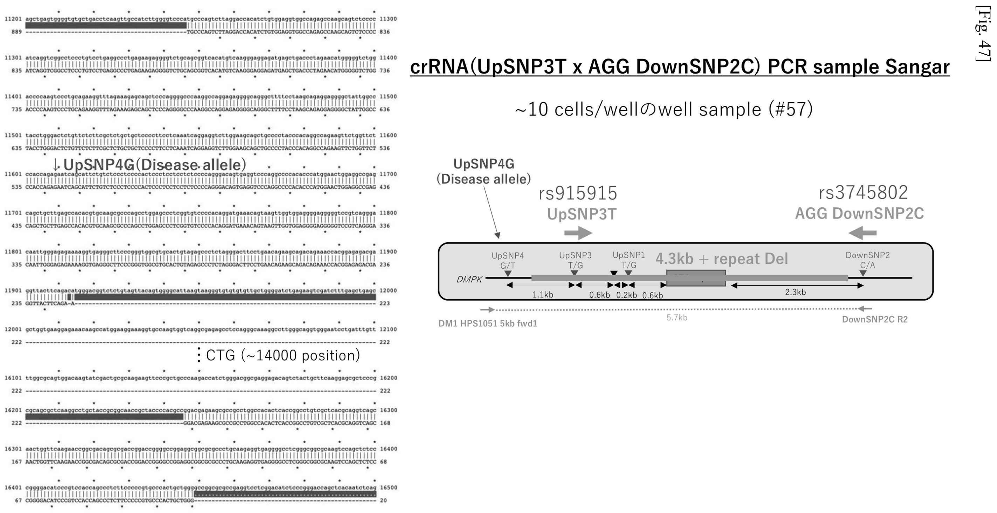

[FIG. 47] FIG. 47 shows the results of removal of the CTG repeat by allele-specific dual-Cas3 (example 3). RNA-based Cas3 genome editing was conducted, the cells were isolated into about 10 cells/well, and bands obtained from these samples through allele-specific PCR were analyzed through Sanger sequencing (UpSNP3T × AGG DownSNP2C well #57).

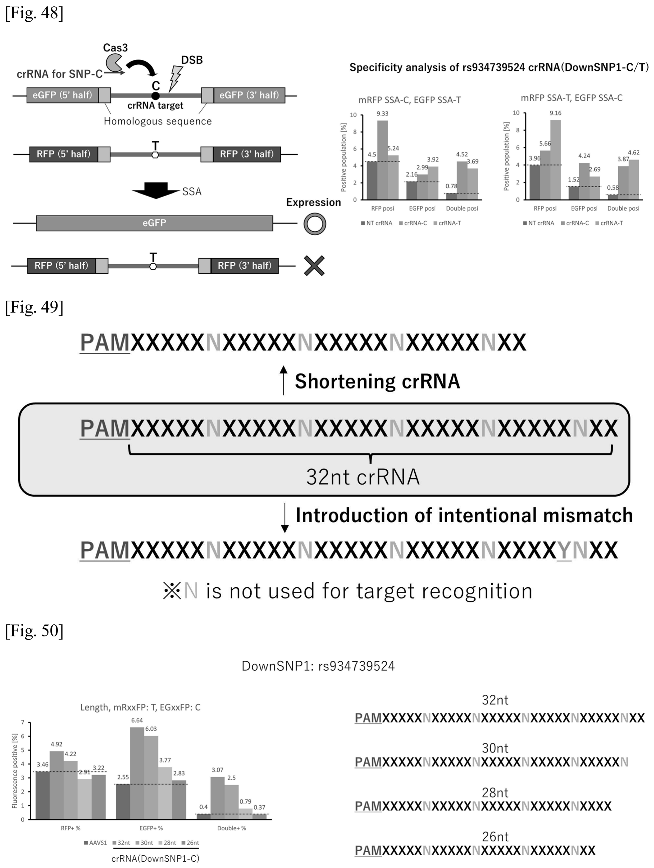

[FIG. 48] FIG. 48 shows a schematic diagram (left) illustrating a method for evaluating allele specificity using a bicolor SSA reporter vector, and the analysis results (right). 1 µM DOX was added to HEK293T Stable cells capable of expressing Cas3 and the Cascade protein group with DOX, a crRNA expression vector pPV-C1-crRNA-EF1a-BA (non-target: DMD#20, target: DownSNP1-C/T), an mRFP SSA vector (pPV-EF1a-mRxxFP-iPA), and an EGFP SSA vector (pPV-EF1a-EGxxFP-iPA) were simultaneously transfected to the cells using Lipofectamine 2000, and the ratio of cells positive for mRFP and EGFP was measured through flow cytometry after three days. A sequence that includes DownSNP1-T/C (rs934739524 hetero SNP) was used as the insertion sequence of the SSA vector.
[FIG. 49] FIG. 49 shows a schematic diagram illustrating a method for enhancing the specificity of an allele-specific crRNA.
[FIG. 50] FIG. 50 shows that the specificity could be adjusted by varying the length of the crRNA. 1 µM DOX was added to HEK293T Stable cells capable of expressing Cas3 and the Cascade protein group with DOX, a crRNA expression vector pPV-C1-crRNA-EF1a-BA (non-target: AAVS1#1, target: DownSNP1-C), an mRFP SSA vector (pPV-EF1a-mRxxFP-iPA), and an EGFP SSA vector (pPV-EF1a-EGxxFP-iPA) were simultaneously transfected to the cells using Lipofectamine 2000, and the ratio of cells positive for mRFP and EGFP was measured through flow cytometry after three days. An insertion sequence that includes DownSNP1-T (rs934739524 hetero SNP-T) was used for the mRFP SSA vector, and an insertion sequence that includes DownSNP1-C was used for the EGFP SSA vector. The crRNA (DownSNP1-C 32nt) has the same sequence as the conventional crRNA (DownSNP1-C).

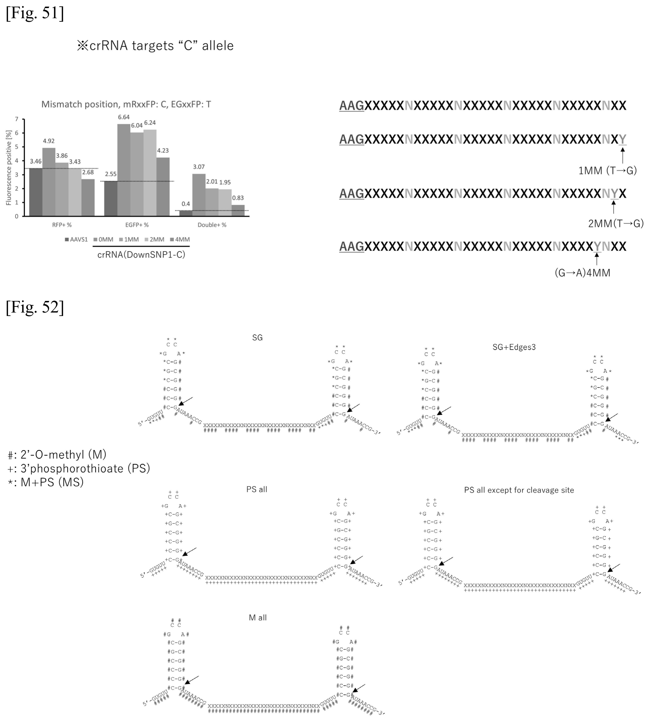

[FIG. 51] FIG. 51 shows that the specificity could be adjusted by introducing an intentional mismatch. The experimental conditions are the same as those shown in FIG. 50. The crRNA (DownSNP1-C 0MM) has the same sequence as the conventional crRNA (DownSNP1-C). Since the same batch as that in FIG. 50 was measured, FIG. 50 was taken as the data on the crRNA(AAVS1#1) and the crRNA (DownSNP1-C0MM).
[FIG. 52] FIG. 52 shows a schematic diagram illustrating the results of additional examination of RNA modification positions in the E. coli Cas3 pre-crRNA based on the protein structure. The nucleotides denoted by # have a 2'-O-methyl (M) modification, the nucleotides denoted by + have a 3'phosphorothioate (PS) modification, and the nucleotides denoted by * have a 2'-O-methyl 3'phosphorothioate (MS) modification. Arrows indicate the Cas6 cleavage sites.

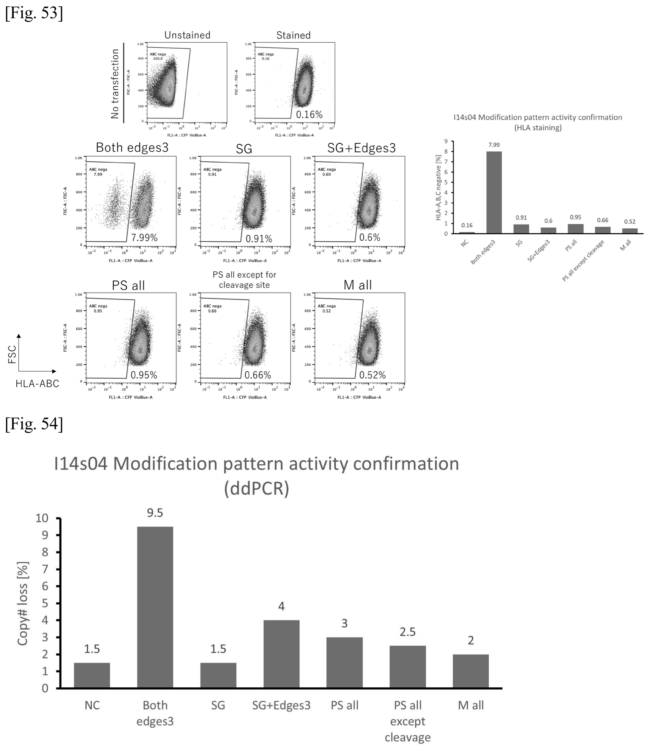

[FIG. 53] FIG. 53 shows changes in activity depending on the RNA modification positions shown in FIG. 52 in the E. coli Cas3 pre-crRNA. An mRNA for expression of Cas3 and the Cascade proteins and B2M#1 crRNAs (with the chemical modifications corresponding to FIG. 52) were transfected to I14s04 iPS cells, and the B2M gene KO efficiency was measured through HLA-A, B, C staining. "Both edges3" is a pre-crRNA with the same modifications as those of Pre-both3. The same applies hereinafter.
[FIG. 54] FIG. 54 shows changes in activity depending on the RNA modification positions shown in FIG. 52 in the E. coli Cas3 pre-crRNA. An mRNA for expression of Cas3 and the Cascade proteins and B2M#1 crRNAs (with the chemical modifications corresponding to FIG. 52) were transfected to I14s04 iPS cells, and the B2M gene KO efficiency was measured through ddPCR.

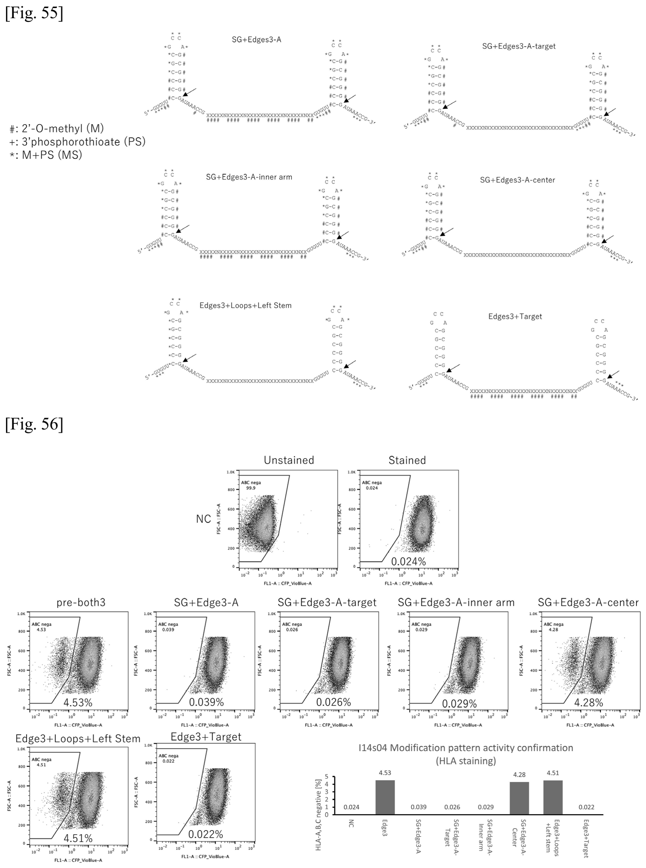

[FIG. 55] FIG. 55 shows a schematic diagram illustrating the results of additional examination of RNA modification positions in the E. coli Cas3 pre-crRNA based on the protein structure. The nucleotides denoted by # have a 2'-O-methyl (M) modification, the nucleotides denoted by + have a 3'phosphorothioate (PS) modification, and the nucleotides denoted by * have a 2'-O-methyl 3'phosphorothioate (MS) modification. Arrows indicate the Cas6 cleavage sites.
[FIG. 56] FIG. 56 shows changes in activity depending on the RNA modification positions shown in FIG. 52 in the E. coli Cas3 pre-crRNA. An mRNA for expression of Cas3 and the Cascade proteins and B2M#1 crRNAs (with the chemical modifications corresponding to FIG. 52) were transfected to I14s04 iPS cells, and the B2M gene KO efficiency was measured through HLA-A, B, C staining.

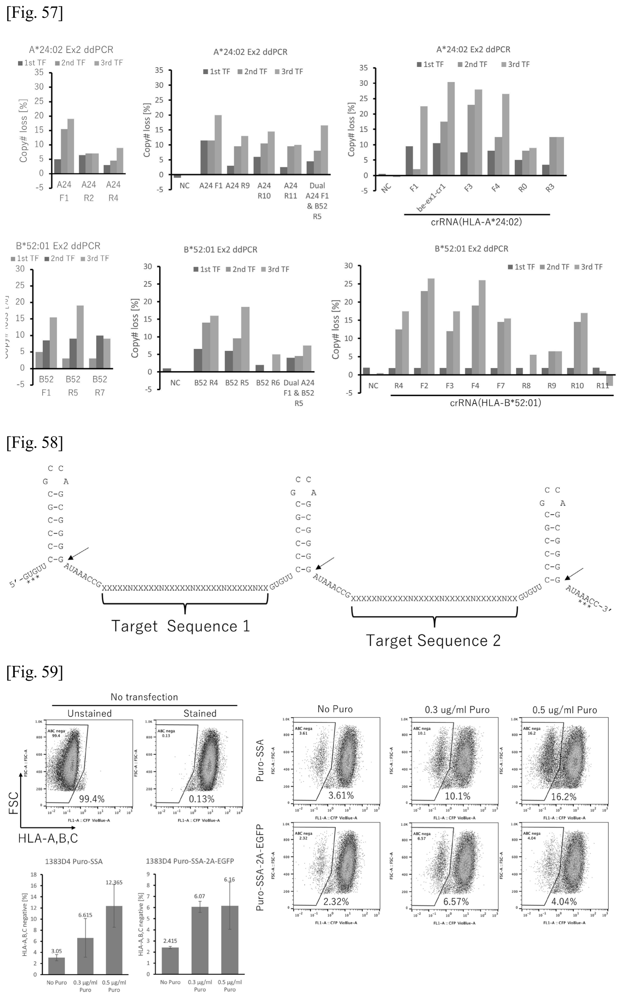

[FIG. 57] FIG. 57 shows that an mRNA for expression of Cas3 and the Cascade protein group and Pre-both3 pre-crRNA (FIG. 5-1) targeting HLA-A*24:02 and B*52:01 were transfected a plurality of times to I14s04 iPS cells using Lipofectamine Stem, and collected genome samples were analyzed through ddPCR. "Dual A24 F1 & B52 R5" indicates a tandem pre-crRNA in which three bases at both termini have the MS modification.
[FIG. 58] FIG. 58 shows a schematic diagram illustrating an example of a chemically modified tandem pre-crRNA. Since the Cas3 pre-crRNA undergoes Cas6 processing, two or more target recognition sequences can be linked in series as shown in the diagram. In this case as well, it is possible to modify three bases at both termini and the like.
[FIG. 59] FIG. 59 shows the results of evaluation of the effects of a Puro-SSA vector in 1383D4 iPS cells. 1.5×10⁵ cells were seeded on a 12-well plate, and an mRNA for expression of Cas3 and the Cascade proteins, the B2M#1 crRNA (with three bases at both termini having the MS modification), and pHL-EF1a-Puro-SSA(B2M#1)-A or pHL-EF1a-Puro-SSA(B2M#1)-2A-EGFP-A serving as the Puro-SSA reporter vector were transfected. Puro selection was conducted at a concentration of 0.3 or 0.5 µg/ml two to three days after the transfection, and the ratio of cells negative for HLA-A, B, C were measured through HLA-A, B, C staining after the recovery of the cells. Representative results of flow cytometry and quantitative graphs (n = 2) were shown. As a result, B2M knockout cells could be concentrated about 2- to 4-fold.

### DESCRIPTION OF EMBODIMENTS

### 1. Pre-crRNA with Modified Nucleotide

The present invention provides a pre-crRNA with a modified nucleotide for the type I CRISPR-Cas system. Specifically, the present invention provides a pre-crRNA (which may be referred to as the "modified crRNA of the present invention" hereinafter) in which at least one (e.g., two, three, four, five, six, seven, eight, nine, ten, or more) nucleotide is a modified nucleotide in a region consisting of the 5' arm region of the first repeat sequence, the 5'-side region (which may be referred to as the "5'-side stem-forming region" hereinafter) of the stem-forming region of the first repeat sequence, and the loop-forming region of the first repeat sequence.

As described in Examples, which will be described below, it was shown that the genome editing efficiency can be enhanced by adding a cap or cap analog to the 5' terminus of the pre-crRNA and the poly-A sequence to the 3' terminus of the pre-crRNA similarly to in-vivo modification manner of an mRNA. Accordingly, another aspect of the present invention provides a pre-crRNA (which may be referred to as the "5'-cap-type crRNA of the present invention" hereinafter) with a 5' cap and poly-A for the type I CRISPR-Cas system. In the description below, the "modified crRNA of the present invention" and the "5'-cap-type crRNA of the present invention" may be referred to collectively as the "crRNA of the present invention".

Six types of type I CRISPR-Cas systems, namely types A to F, are known, and any crRNAs for these types of systems can be used as the crRNA of the present invention as long as they have the arm region and the stem loop-forming region. In particular, a pre-crRNA for the type I-E (particularly the type I-E derived from E. coli) or the type I-D (particularly the type I-D derived from Microcystis aeruginosa) is preferable. The cas operon encoding Cas (CRISPR-associated) proteins such as Cas1, Cas2, and proteins included in the Cascade complex is present in proximity to CRISPR. Out of the cas operons of the types I-A to I-F, a protein group excluding Cas3 serving as a nuclease/helicase (however, Cas3 of the type I-D does not have such activity, and Cas10d functions as a nuclease/helicase) and Cas1, Cas2, and Cas4, which are involved in cleavage of an exogenous gene during immunity acquirement forms the Cascade complex. The Cascade complex and Cas3 (or a complex of Cas3 and Cas10d) added thereto exhibit the functions of recognizing and cleaving a DNA similar to those exhibited by Cas9 or Cas12a in the type II CRISPR-Cas system.

The types I-A, I-B, and I-D are distributed in relatively many archaeobacteria, and the types I-C, I-E, and I-F are distributed in relatively many eubacteria. The type I-A has been analyzed in S. solfataricus, T. tenax, and the like. The type I-B has been analyzed in Haloferax volcanii and the like. The type I-C has been analyzed in B. halodurans and the like. The type I-D has been analyzed in M. aeruginosa and the like. The type I-E has been analyzed in E. coli and the like. The type I-F has been analyzed in P. aeruginosa, E. coli, P. atospeticum, and the like. In the type I-E, Cse1 (also known as CasA and Cas8e), Cse2 (also known as CasB and Cas11), Cas7 (also known as CasC, and Cas4), Cas5 (also known as CasD), and Cas6 (also known as CasE) form the Cascade complex at a molecular ratio of 1:2:6:1:1 for one molecule of the crRNA. In the type I-D, Cas7, Cas5 (Csc1), and Cas6 form the Cascade complex at a molecular ratio of 6:1:1 for one molecule of the crRNA. As the protospacer adjacent motif (PAM) sequence, GTA, GTC, GTT, and the like can be used in the type I-D CRISPR-Cas system, and ATG, AAG, AGG, GAG, TAG, AAA, and the like can be used in the type I-E CRISPR-Cas system. Also, TCN (N is A, T, G, or C) and the like can be used in the type I-A CRISPR-Cas system, TTC, ACT, TAA, TAT, TAG, CAC, and the like can be used in the type I-B CRISPR-Cas system, NTTC (N is A, T, G, or C) can be used in the type I-C CRISPR-Cas system, and CC and the like can be used in the type I-F CRISPR-Cas system. In this specification, the direction of a base sequence is a 5'-to-3' direction unless otherwise stated.

The type I-A CRISPR-Cas system includes Cas8al, Csa5(Cas11), Cas5, Cas6, and Cas7 as the constituent elements of the Cascade complex, the type I-B CRISPR-Cas system includes Cas8b1, Cas5, Cas6, and Cas7 as the constituent elements of the Cascade complex, the type I-C CRISPR-Cas system includes Cas8c, Cas5, and Cas7 as the constituent elements of the Cascade complex, the type I-F CRISPR-Cas system includes Csy1 (Cas8f), Csy2 (Cas5), Cas6, and Csy3 (Cas7) as the constituent elements of the Cascade complex, and the type I-G CRISPR-Cas system includes Cst1 (Cas8al), Cas5, Cas6, and Cst2 (Cas7) as the constituent elements of the Cascade complex. In the description below, Cas3, Cas10d, and the Cascade protein group may be referred to collectively as the "Cas proteins".

In the type I CRISPR-Cas system, a pre-crRNA having a structure constituted by a repetition of a repeat sequence and a target recognition region (spacer region) as shown in FIG. 3-1 (when a plurality of spacer sequences are included, the configuration is as follows: repeat, spacer 1, repeat, spacer 2, repeat (further repeated)) is cleaved by Cas6 (in the types-I-A, B, D, and E) or Cas5 (in the type I-C) into a mature crRNA. In this specification, the term "pre-crRNA" means an RNA that has at least a repeat, a spacer 1, and a repeat and is cleaved by Cas6 or Cas5 in a cell into a functional crRNA. FIG. 3-1 shows an E. coli type I-E pre-crRNA constituted by a repeat, a spacer 1, and a repeat. FIG. 3-2 illustrates repeat structures of pre-crRNAs of other types (Reference: Zheng Y. et al., Front Bioeng Biotechnol. 2020 Mar 4; 8: 62). The sequence (which may be referred to as the "spacer sequence" hereinafter) of the target recognition region is originally a sequence derived from an exogenous DNA incorporated during the natural adaptation process, but can be designed based on the sequence of a target DNA. A pre-crRNA with one spacer region typically includes a 5' arm region of a first repeat sequence (corresponding to the region of the first to fifth nucleotides GUGUU in FIG. 3-1), a stem loop-forming region of the first repeat sequence (corresponding to the region of the sixth to twentyfirst nucleotides CCCCGCGCCAGCGGGG (SEQ ID NO: 1) in FIG. 3-1), a 3' arm region of the first repeat sequence (corresponding to the region of the twenty-second to twenty-ninth nucleotides AUAAACCG in FIG. 3-1), a spacer region (corresponding to the region of the thirtieth to sixty-first nucleotides XXXXXNXXXXXNXXXXXNXXXXXNXXXXXNXX in FIG. 3-1), a 5' arm region of a second repeat sequence (corresponding to the region of the sixtysecond to sixty-sixth nucleotides GUGUU in FIG. 3-1), a stem loop-forming region of the second repeat sequence (corresponding to the region of the sixty-seventh to eighty-second nucleotides CCCCGCGCCAGCGGGG in FIG. 3-1), and a 3' arm region of the second repeat sequence (corresponding to the region of the eighty-third to ninetieth nucleotides AUAAACCG in FIG. 3-1). FIG. 58 shows an example of a pre-crRNA with two spacer regions. FIG. 58 shows a pre-crRNA that includes a second spacer region (target sequence 2), a 5' arm region of a third repeat sequence, a stem loop-forming region of the third repeat sequence, and a 3' arm region of the third repeat sequence in addition to the 5' arm region of the first repeat sequence, the stem loop-forming region of the first repeat sequence, the 3' arm region of the first repeat sequence, the first spacer region (target sequence 1), the 5' arm region of the second repeat sequence, the stem loop-forming region of the second repeat sequence, and the 3' arm region of the second repeat sequence. When a pre-crRNA having a plurality of spacer regions in this manner is used, a plurality of regions can be altered by using one pre-crRNA with spacer regions having different sequences.

The type I Cascade complex forms a complex (which may be referred to as the "Cascade-crRNA complex" hereinafter) with a crRNA. Subsequently, the Cascade-crRNA complex partially unwinds a double-stranded DNA that includes a PAM sequence and a target sequence, forms a structure called the R loop, and binds thereto. At this time, the structure of the Cascade-crRNA complex itself changes, and the complex binds to the Cas3 protein (a complex of Cas3 and Cas10d in the case of the type I-D). The Cas3 protein (Cas10d in the case of the type I-D) has DNA nickase activity and DNA helicase activity, and introduces a nick to a non-target strand (a strand to which the target recognition region of the crRNA does not complementarily bind (hybridize)). In the type I CRISPR-Cas3 system, PAM is present on the non-target strand. Typically, a nick is introduced to a site on the upstream side (5' side) of the PAM sequence of the non-target strand, and then a deletion occurs on the upstream side of the PAM sequence due to winding reaction by a helicase. Accordingly, in this specification, the "target sequence" means a sequence that is targeted by a pre-crRNA, a crRNA, or a guide RNA, is also called a protospacer sequence, and is adjacent to the PAM on the 3'-side on the non-target strand in which the PAM is present unless otherwise stated. The target sequence is a sequence homologous with the sequence (which may be referred to as the "spacer sequence") of the target recognition region present in a crRNA (note that U in an RNA sequence is read as T in a DNA sequence).

In the type I CRISPR-Cas system, a base that is not recognized by a crRNA and does not contribute to sequence specificity is present every six bases in the target sequence. When a base of a site that does not contribute to the target sequence recognition of a crRNA is "X" and a base that contributes to the target sequence recognition is N (A, T, G, or C), an example of the target sequence can be 5'-NNNNNXNNNNNXNNNNNXNNNNNXNNNNNXNN-3'. Even when the spacer sequence of a crRNA and the target sequence do not have exactly the same sequence, these sequences may bind to each other, resulting in cleavage of the target DNA via Cas3 or Cas10d. Therefore, a crRNA having, as a spacer sequence, a sequence in the base sequence portion represented by N above with substitution, deletion, addition, and/or insertion of at least one (e.g., two, three, four, five, or more) base can also be used in the present invention. As described above, the "homologous sequence" encompasses not only exactly the same sequence as a sequence of interest (e.g., target sequence) but also a sequence with substitution, deletion, addition, and/or insertion of at least one (e.g., two, three, four, five, or more) base.

The target sequence can be selected as appropriate depending on the purpose. Specific examples of the target sequence include the sequence of the β2-microglobulin (B2M) gene and the regulatory region therefor (e.g., sequence represented by SEQ ID NO: 4 or 5), the sequence of the human leukocyte antigen (HLA) gene and the regulatory region therefor (e.g., sequence represented by any of SEQ ID NOS: 6 to 38), the sequence of the dystrophin (DMD) gene and the regulatory region therefor (e.g., sequence represented by any of SEQ ID NOS: 55 to 68), the sequence of the DMPK gene and the regulatory region therefor (e.g., sequence represented by any of SEQ ID NOS: 39 to 53), the sequence of the AAVS1 (adeno-associated virus integration site 1) region (e.g., sequence represented by SEQ ID NO: 54), repeat sequences and sequences in proximity thereof, and the like. Examples of the HLA gene include the HLA-A gene, the HLA-B gene, the HLA-C gene, the HLA-E gene, the HLA-F gene, the HLA-G gene, the HLA-DRA gene, the HLA-DRB gene, the HLA-DPA gene, the HLA-DPB gene, the HLA-DQA gene, the HLA-DQB gene, and the like. It is known that each HLA gene has a sequence variation. For example, the HLA-A gene includes a large number of HLA types such as HLA-A02 and HLA-A27 whose amino acid sequences or base sequences are different. Examples of the repeat sequence include a sequence in which three bases "CTG" are repeated, a sequence in which three bases "CGG" are repeated, a sequence in which three bases "CAG" are repeated, a sequence in which three bases "GAA" are repeated, a sequence in which four bases "CCTG" are repeated, a sequence in which five bases "TTTCA" are repeated, and the like.

Also, another aspect of the present invention provides a pre-crRNA targeting a sequence represented by any of SEQ ID NOS: 4 to 68. Out of these pre-crRNAs, a plurality of pre-crRNAs targeting the same gene or the same exon can also be used in combination. These pre-crRNAs may be wild-type pre-crRNAs or pre-crRNAs other than the wild-type pre-crRNAs. These pre-crRNAs may be provided in the form of a kit or an agent (e.g., a therapeutic agent and a reagent).

Before a modified nucleotide is introduced, the pre-crRNA may be a wild-type pre-crRNA, or at least one (e.g., two, three, four, five, or more) base may be substituted, deleted, added and/or inserted in at least one region of the 5' arm region of the first repeat sequence, the stem loop-forming region of the first repeat sequence, the 3' arm region (a region corresponding to the 5' handle of a mature crRNA) of the first repeat sequence, the spacer region, the 5' arm region of the second repeat sequence, the stem loop-forming region of the second repeat sequence, and the 3' arm region of the second repeat sequence (e.g., the 5' arm region of the first repeat sequence and/or the 3' arm region of the second repeat sequence) compared with the wild-type pre-crRNA as long as an ability to recognize the target sequence and an ability to recruit the Cascade are maintained. In this specification, in the latter case, the pre-crRNA may also be referred to as the "mutant pre-crRNA". Also, when two or more spacer regions are included, at least one (e.g., two, three, four, five, or more) base may be substituted, deleted, added and/or inserted in at least one of regions (e.g., the second spacer region (target sequence 2), the 5' arm region of the third repeat sequence, the stem loop-forming region of the third repeat sequence, and the 3' arm region of the third repeat sequence) other than the regions above compared with the wild-type pre-crRNA. The "mutant pre-crRNA" also encompasses such a pre-crRNA. When addition, deletion, or the like of a base is conducted on a stem-forming region of the stem loop-forming region, it is preferable to conduct the addition, deletion, or the like of a base (typically in terms of two bases that complementarily bind to each other) such that the stem structure is maintained.

The lengths of the spacer sequence and the target sequence in the type I CRISPR-Cas system are not particularly limited, but may be, for example, 30 to 45 nucleotides. 32 to 33 nucleotides are preferable in the types I-C, I-E, and I-F, and 34 to 44 nucleotides are preferable in the types of I-A, I-B, and I-D.

In this specification, the "modified nucleotide" means a nucleotide in which at least one of the constituent elements of a ribonucleotide is modified, other than ribonucleotides. A nucleotide includes a sugar moiety (e.g., ribose), a base, and a phosphate group as the constituent elements, and the modified nucleotide has a modification in at least one of the sugar moiety, the base, and the phosphate group (particularly the sugar moiety). Also, a nucleoside having a modification in at least one of the sugar moiety and the base is also referred to as the "modified nucleotide", and the "modified nucleotide" can also be read as the "modified nucleoside" as appropriate in this specification. Examples of the "modification" include substitution, addition, and/or deletion in the constituent elements and/or an internucleoside bond, and substitution, addition, and/or deletion of an atom and/or a functional group in the constituent elements and/or an internucleoside bond. In the present specification, the modified nucleotide also encompasses a deoxyribonucleotide, which is a natural nucleotide. The modified crRNA of the present invention may include only one type of modified nucleotide or a plurality of types of modified nucleotides.

Examples of the natural bases include adenine, cytosine, guanine, thymine, and uracil. Examples of a modified base obtained by modifying the base include a 5-methylcytosine, a 5-fluorocytosine, a 5-bromocytosine, a 5-iodocytosine, or an N4-methylcytosine; an N6-methyladenine or an 8-bromoadenine; and an N2-methylguanine or an 8-bromoguanine, but there is no limitation thereto.

Examples of a modification of the sugar moiety include a 2'-O-methoxyethyl modification of the sugar moiety, a 2'-O-methyl modification of the sugar moiety, a 2' fluoro modification of the sugar moiety, a crosslink between the 2' position and the 4' position of the sugar moiety (a nucleotide having this crosslink structure is BNA), and the like. In particular, the 2'-O-methyl modification of the sugar moiety is preferable. In this case, a portion (e.g., one, two, three, four, five, six, seven, eight, nine, ten, or more) of the nucleotides included in the modified crRNA of the present invention may be a nucleotide having a 2'-O-methyl modification (i.e., 2'-O-methylribonucleotide), or all the nucleotides may be a 2'-O-methylribonucleotide. Examples of the BNA include a locked nucleic acid (LNA), a 2'-O,4'-C-ethylenebridged nucleic acid (ENA), and the like. More specific examples of the BNA include those having a nucleoside structure below.

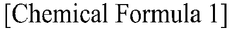

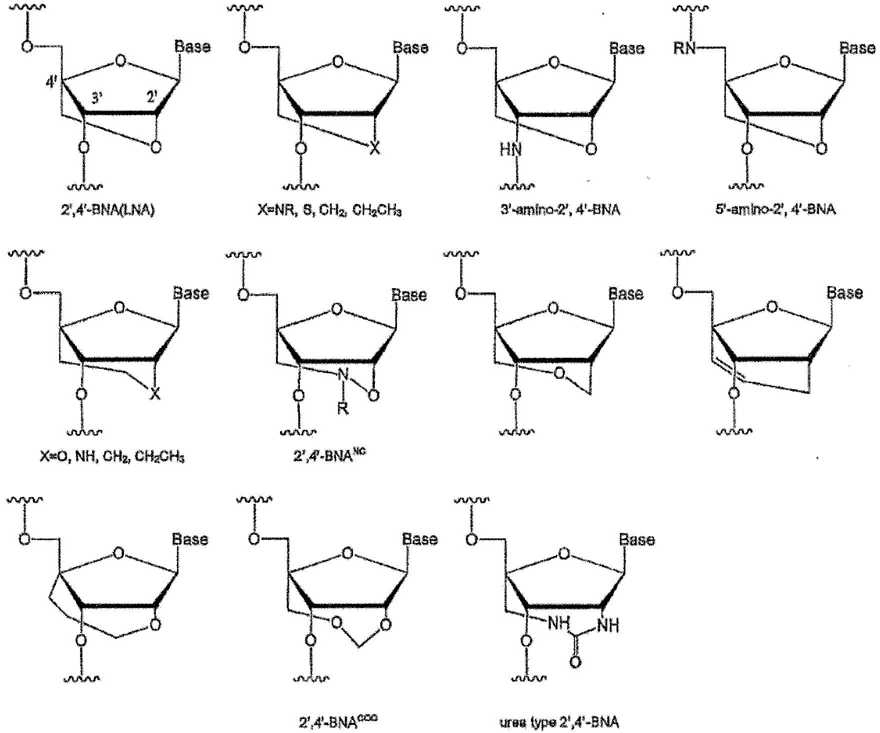

(In the formula, R represents a hydrogen atom, an alkyl group having 1 to 7 carbon atoms that may be branched or cyclic, an alkenyl group having 2 to 7 carbon atoms that may be branched or cyclic, an aryl group having 3 to 12 carbon atoms that may include a heteroatom, an aralkyl group with an aryl moiety having 3 to 12 carbon atoms that may include a heteroatom, or an amino group protecting group for nucleic acid synthesis. R is preferably a hydrogen atom, a methyl group, an ethyl group, an n-propyl group, an isopropyl group, a phenyl group, or a benzyl group, and more preferably a hydrogen atom or a methyl group. Base is a natural base or a modified base.)

A portion (e.g., one, two, three, four, five, six, seven, eight, nine, ten, or more) or all of the internucleoside bonds present in the modified crRNA of the present invention may be a bond other than the phosphodiester bond. Examples of the bond (which may be referred to as the "modified internucleoside bond" hereinafter) other than the phosphodiester bond include a phosphorothioate bond, a phosphorodithioate bond, a phosphotriester bond, a methylphosphonate bond, a methylthiophosphonate bond, a boranophosphate bond, a phosphoroamidate bond, and the like, but there is no limitation thereto. In one aspect, it is preferable that the internucleoside bonds on the 3'-side and/or the 5'-side (preferably internucleoside bonds on the 3'-side) of a portion (e.g., one, two, three, four, five, six, seven, eight, nine, ten, or more) or all of the nucleosides having a modification (e.g., 2'-O- methyl modification) in the sugar moiety are a modified internucleoside bond (preferably phsphorothioate bond). A favorable embodiment of the present invention provides a pre-crRNA in which the phosphodiester bonds on the 3'-side of all the modified nucleosides are a phosphorothioate bond. The modified crRNA of the present invention may include only one type of modified internucleoside bond or a plurality of types of modified internucleoside bonds.

In an aspect of the modified crRNA of the present invention, it is preferable that at least one (e.g., two, three, four, five, or more) modified nucleotide is present in the 5' arm region of the first repeat sequence, and all the nucleotides in the 5' arm region may be a modified nucleotide. In this case, at least one (e.g., two, three, four, five, six, seven, eight, or more) nucleotide in the 3' arm region of the second repeat sequence may be a modified nucleotide. In the modified crRNA of a preferable aspect of the present invention, the first nucleotide from the 5' terminus is a modified nucleotide, and the second nucleotide from the 3' terminus is also a modified nucleotide. In particular, a pre-crRNA is more preferable in which all of the first to third nucleotides (corresponding to GUG in FIG. 3-1) from the 5' terminus are a modified nucleotide, and all of the second to fourth nucleotides (corresponding to ACC in FIG. 3-1) from the 3' terminus are a modified nucleotide. Also, in the loop-forming regions of the first repeat sequence and the second repeat sequence, at least one (e.g., 2, 3, 4, or more) or all of the nucleotides may be a modified nucleotide. In this specification, the M^{th} (M is a positive integer) nucleotide from the 5' terminus means a M^{th} nucleotide in a direction toward the 3' terminus when the nucleotide located at the 5' terminus is taken as the first nucleotide in FIG. 3-1. A leader sequence may be added to the 5'-side of a pre-crRNA. In this case, the positions are defined based on the sequence excluding the leader sequence. Similarly, the M^{th} (M is a positive integer) nucleotide from the 3' terminus means a M^{th} nucleotide in a direction toward the 5' terminus when the nucleotide located at the 3' terminus is taken as the first nucleotide.

As shown in Examples, introduction of a modified nucleotide to the 3'-side region of the stem-forming portion (also referred to as the "3'-side stem-forming region) of the first repeat sequence and the stem-forming portion of the second repeat sequence did not exhibit a genome editing enhancement effect. Accordingly, these regions may include a modified nucleotide or no modified nucleotide, but it is preferable that no modified nucleotides are included (i.e., all the nucleotides are a ribonucleotide) from the viewpoint of cost. Surprisingly, it was shown that introduction of a modified nucleotide to the 3' arm region of the first repeat sequence, the 5' arm region of the second repeat sequence, and the 5'-side stem-forming region of the second repeat sequence reduced the genome editing efficiency. Accordingly, it is preferable that these regions include no modified nucleotides. Therefore, in an embodiment of the present invention, the modified crRNA of the present invention may have a configuration in which the 3' arm region of the first repeat sequence, the 5' arm region of the second repeat sequence, and the 5'-side stem-forming region of the second repeat sequence include only ribonucleotides, and the 3'-side stem-forming region of the first repeat sequence and the stem-forming region of the second repeat sequence also include only ribonucleotides. In the spacer sequence, at least one (e.g., two, three, four, five, six, seven, eight, or more) nucleotide may be a modified nucleotide, but all the nucleotides are a ribonucleotide in an aspect. In the modified crRNA of the present invention, the bond on the 3'-side of the ribonucleoside is typically a phosphodiester bond.

In the type I-E CRISPR-Cas system, cleaving a pre-crRNA into a mature crRNA in a cell may be important for the activation of genome editing. Accordingly, it is preferable that sites to be cleaved by Cas6 or Cas5 (specifically, a phosphodiester bond between the 3'-terminal nucleoside of the stem-forming region of the first repeat sequence and the 5'-terminal nucleoside of the 3' arm region of the first repeat sequence, and a phosphodiester bond between the 3'-terminal nucleoside of the stem-forming region of the second repeat sequence and the 5'-terminal nucleoside of the 3' arm region of the second repeat sequence) and/or nucleosides adjacent to these sites (particularly nucleosides on the 3'-side) are not modified.

In this specification, the 5' cap includes not only a 7-methylguanosine (m⁷G), which is found in a natural mRNA, but also an analog thereof (e.g., ARCA (anti-reverse cap analog) obtained through methylation of the OH group at position 3' of the sugar of m⁷G). The length of the poly-A added to the crRNA of the present invention is not particularly limited, but a poly-A consisting of 200 to 250 adenines is generally added in a mammal, a poly-A that includes 10 or more (e.g., 20, 30, 40, 50, or more) and 300 or less (e.g., 250, 240, 230, 220, 210, 200, or less) adenines may be typically added.

One or more (e.g., one, two, three, four, or more) functional molecules may be linked to the crRNA of the present invention. The functional molecule may be typically linked to the 5' terminus and/or the 3' terminus. The bond between the crRNA of the present invention and the functional molecule may be a direct bond or an indirect bond in which a separate substance is involved. However, it is preferable that the functional molecule is directly linked to the oligonucleotide via a covalent bond, an ionic bond, a hydrogen bond, or the like, and a covalent bond is more preferable from the viewpoint that a stabler bond can be obtained. The functional molecule may also be linked to the crRNA via a cleavable linking group. For example, the functional molecule may be linked via a disulfide bond. Only one type of functional molecule may be used, or a plurality of types of functional molecules may be used in combination.

The structure of the "functional molecule" is not particularly limited as long as the functional molecule imparts a desired function to the crRNA of the present invention. Examples of the desired function include a labeling function, a purification function, a delivery function, and the like. Examples of a molecule for imparting the labeling function include compounds such as fluorescent dyes (Cy3, Alexa, and the like), fluorescent proteins, and luciferase. Examples of a molecule for imparting the purification function include compounds such as biotin, avidin, a His-tag peptide, a GST-tag peptide, and a FLAG-tag peptide. Examples of a molecule for imparting the delivery function include an arginine-rich peptide P007 and a B peptide (HaiFang Yin et al., Human Molecular Genetics, Vol. 17(24), 3909-3918 (2008)), m3G-CAP (Pedro M. D. Moreno et al., Nucleic Acids Res., Vol. 37, 1925-1935 (2009)), a TAT peptide, N-acetylgalactosamine (GalNAc), lipids such as cholesterol and fatty acids (e.g., vitamin E (tocopherol and tocotrienol), vitamin A, and vitamin D), fat-soluble vitamins such as vitamin K (e.g., acyl carnitine), intermediate metabolites such as acyl-CoA, glycolipids, glycerides, and derivatives or analogs thereof.

The crRNA of the present inventio may be produced through chemical synthesis, and can also be produced using an in-vitro transcription reaction (IVT) method. Alternatively, a crRNA expressed in an organism (E. coli or a cultured mammalian cell) can be purified and used. Examples of the chemical synthesis method include a method in which a nucleoside phosphoramidite and a solid-phase support are used, and the like. Using a nucleoside phosphoramidite to which a chemical modification has been introduced (e.g., a phosphoramidite with a 2'-O-methylated sugar moiety, and a GalNAc phosphoramidite) makes it possible to introduce a modified nucleotide to a desired base site of the crRNA sequence.

With the IVT method, a single-stranded RNA can be synthesized, for example, using a T7 RNA polymerase from a DNA encoding a pre-crRNA linked under the control of a T7 promoter, and a pre-crRNA having a 5'-cap structure can be synthesized by adding a 5' cap in the reaction solution. Furthermore, a pre-crRNA that includes a modified nucleotide can also be synthesized by adding a modified nucleoside triphosphate to the reaction solution. Poly-A can be added by reacting a poly-A polymerase with the crRNA. Alternatively, a pre-crRNA to which poly-A is added can also be synthesized through IVT reaction by linking poly-T to the DNA encoding the pre-crRNA.

A 5' cap can be added to a pre-crRNA through chemical synthesis. For example, as described in Abe N. et al., ACS Chem Biol. 17(6): 1308-1314 (2022), a 5' cap can be added to a pre-crRNA by reacting an imidazole-activated m⁷G diphosphate with a pre-crRNA produced through chemical synthesis, in an organic solvent (e.g., dimethylsulfoxide). Accordingly, a pre-crRNA to which a 5' cap and poly-A are further added can also be favorably used as the modified crRNA of the present invention. Alternatively, a desired pre-crRNA can also be obtained by specifying a base sequence and a modification site or modification type and placing an order with a manufacturer. In the description below, a pre-crRNA other than a wild-type pre-crRNA that includes a mutant nucleotide, a 5'-cap structure, 3' poly-A, and the like similarly to the crRNA of the present invention is also referred to as the "artificial pre-crRNA". A region that is not found in the wild-type pre-crRNA may be added to the artificial pre-crRNA.

A DNA encoding the pre-crRNA can be chemically synthesized using a DNA/RNA synthesizer by, for example, designing a sequence encoding the pre-crRNA that includes a base sequence (i.e., spacer sequence) homologous with the target sequence.

### 2. Method for Designing Allele-Specific Pre-crRNA

As shown in FIG. 33, in the type I CRISPR-Cas system, a base (base represented by N in FIG. 33) that cannot be recognized by a crRNA is present every six bases in the target sequence, thus making it possible to design a pre-crRNA targeting a sequence in which a hetero SNP is present in the target sequence excluding those bases. In this case, the position of the hetero SNP is preferably close to the PAM sequence, and more preferably in the seed region or a vicinity thereof. The seed region is a region highly homologous (typically completely homologous) with the spacer sequence of the crRNA, and corresponds to a region between the first base and the eighth base from the downstream-(3'-)side terminal base of the PAM sequence in the E. coli CRISPR-Cas3 system (Non-Patent Literature 1). The vicinity of the seed region is typically a region between the ninth base and the eleventh base from the downstream-(3'-)side terminal base of the PAM sequence. A pre-crRNA targeting a sequence in which a hetero SNP is present in the PAM sequence may also be designed. In this case, designed is a crRNA targeting a sequence in which an effective PAM sequence (e.g., ATG in, for example, the type I-E CRISPR-Cas system) is present in an allele (target allele) in which introduction of deletion is desired, and the corresponding sequence in the other allele is an ineffective PAM sequence (e.g., CTG). The thus designed crRNA is also referred to as the "allele-specific crRNA", and using a CRISPR-Cas system that includes the allele-specific crRNA makes it possible to introduce a deletion site on the upstream (5'-side) of the PAM sequence in the target allele. The deletion site targeted by such a system is also referred to as the "deletion target site".

Therefore, another aspect of the present invention provides a method for designing an allele-specific pre-crRNA. Specifically, provided is a method for designing an allele-specific pre-crRNA for the type I CRISPR-Cas system (which may be referred to as the "designing method of the present invention" hereinafter) that includes a step of designing a pre-crRNA targeting a sequence in which a deletion target site is present on the upstream (5') side of the PAM sequence adjacent to the target sequence, and at least one base (e.g., one base, two bases, three bases, four bases, five bases, or more) that is different between the alleles at positions other than 6n^{th} (n is a positive integer) positions from the terminal base on the downstream (3') side of the PAM sequence or in the PAM sequence. The pre-crRNA designed using the designing method of the present invention is not particularly limited as long as it is a crRNA included in the type I CRISPR-Cas system, but the pre-crRNA is preferably a pre-crRNA for the type I-E (particularly the type I-E derived from E. coli) or the type I-D (particularly the type I-D derived from Microcystis aeruginosa) and more preferably a pre-crRNA for the type I-E.

When a plurality of types (generally two copies) of genes are present for one gene locus, each of them is called an allele (allelomorph). In this specification, the term "allele-specific" means targeting only one allele (generally a disease-causing allele). The wording "targeting only one allele" encompasses not only an aspect in which the other allele is not (or is predicted to be not) targeted at all but also an aspect in which the genome editing efficiency for the other allele is lower (or is predicted to be lower) than the genome editing efficiency for the target allelic gene.

The "single nucleotide polymorphism (SNP)" generally means variation from one base mutation that is often (e.g., at a frequency of 1% or more) found in the genome base sequences of a population of a certain biological species. However, in this specification, it means at least one base that is different between the alleles (and is also referred to as the "hetero SNP"). When two or more bases are a hetero SNP, all of these bases may be present at positions other than 6n^{th} (n is a positive integer) positions from the terminal base on the downstream side of the PAM sequence or in the PAM sequence, or some of these bases may be present at the above-described positions or in the PAM sequence. The hetero SNP can be identified by, for example, searching for a frequent SNP in a SNP database and confirming whether or not the hit SNP is also present in a cell derived from a disease patient. Examples of the SNP database include dbSNP, Kaviar, SNPedia, OMIM database, dbSAP, The Human Gene Mutation Database, The International HapMap Project, GWAS Central, and the like.

The allele targeted in the designing method of the present invention is not particularly limited, but examples thereof include alleles that cause repeat diseases caused by abnormal extension of a sequence (repeat sequence) in which a specific base sequence is repeated. It is considered that the abnormal repeat number causes the onset of a disease via a mechanism such as loss of functions of a gene product (a protein or an RNA), gain of functions, liquid-liquid phase separation disorder, or non-classical translation. Among the repeat diseases, a disease with abnormal extension of a three-base repeat sequence is known as a triplet disease. It is known that a triplet disease develops typically when a triplet repeat includes 20 or more repeats (typically 35 or more repeats). For shortening of such an abnormally extending repeat, it is difficult to delete the repeat sequence in an allele specific manner by use of a hetero SNP at a distant position using the CRISPR-Cas9 system with which the base recognition and the DNA cleavage occur at substantially the same position. However, the CRISPR-Cas3 system can be used to treat a triplet disease by inducing deletion to shorten the abnormally extending repeat. Accordingly, in an aspect of the present invention, the deletion target site is a region that includes a triplet repeat.

Examples of the repeat disease include fragile X syndrome (repeat: CGC, causative gene: FMR1), fragile XE syndrome (repeat: CCG, causative gene: AFF2), Friedreich ataxia (repeat: GAA, causative gene: FXN), Huntington's disease (repeat: CAG, causative gene: HTT), spinal and bulbar atrophy (repeat: CAG, causative gene: AR), spinocerebellar ataxia type 1 (repeat: CAG, causative gene: ATXN1), spinocerebellar ataxia type 2 (repeat: CAG, causative gene: ATXN2), spinocerebellar ataxia type 3 (repeat: CAG, causative gene: ATXN3), spinocerebellar ataxia type 6 (repeat: CAG, causative gene: CACNA1A), spinocerebellar ataxia type 7 (repeat: CAG, causative gene: ATXN7), spinocerebellar ataxia type 17 (repeat: CAG, causative gene: TBP), dentatorubral-pallidoluysian atrophy (repeat: CAG, causative gene: DRPLA), myotonic dystrophy type 1 (repeat: CTG, causative gene: DMPK), fragile X-associated tremor/ataxia syndrome (repeat: CGC, causative gene: FMR1), spinocerebellar ataxia type 8 (repeat: CTG, causative gene: ATXN8), spinocerebellar ataxia type 12 (repeat: CAG, causative gene: PPP2R2B), Huntington's disease-like syndrome type 2 (repeat: CTG, causative gene: JPH3), and the like.

In an embodiment, a repeat disease targeted in the designing method of the present invention is myotonic dystrophy type 1 (DM1), which is known as one of the triplet diseases. DM1 is a dominant genetic disease that develops due to a CTG repeat in the 3'-UTR region of the DMPK gene extending abnormally in one allele. Examples of the DM1 hetero SNP include SNP ID: rs16939 (distance from the repeat: 2.5 kb), SNP ID: rs915915 (distance from the repeat: 1.5 kb), SNP ID: rs558794490 (distance from the repeat: 0.8 kb), SNP ID: rs635299 (distance from the repeat: 0.6 kb), SNP ID: rs934739524 (distance from the repeat: 1.6 kb), SNP ID: rs3745802 (distance from the repeat: 2.3 kb), and the like in the dbSNP database.

Examples of a sequence (i.e., target sequence) targeted by the pre-crRNA produced using the designing method of the present invention include a sequence represented by any of SEQ ID NOS: 39, 40, and 42 to 47 that includes SNP ID: rs934739524 in the target sequence, a sequence represented by any of SEQ ID NOS: 48 to 50 that includes SNP ID: rs635299 in the target sequence, a sequence represented by SEQ ID NO: 51 or 52 that includes SNP ID: rs915915 in the target sequence, a sequence represented by SEQ ID NO: 53 that includes SNP ID: rs3745802 in the target sequence.

The crRNAs designed using the designing method of the present invention may vary in activity and specificity depending on the spacer sequence. Accordingly, the specificity for a target allele may be enhanced by adjusting the crRNA to reduce the activity for a non-target allele. Examples of such an adjustment method include a method in which the crRNA is shortened (e.g., a method in which one, two, three, four, five, six, seven, eight, nine, ten, or more nucleotides are deleted), a method in which a base mismatch is intentionally introduced to a position distant from the PAM, and the like. Actually, it was proved in Examples below that as a crRNA targeting "C" of the SNP of SNP ID No: rs934739524 was shortened, or the position of an intentional mismatch was brought closer to the PAM from a distant position, the activity for the target allele gradually decreased, but the specificity was enhanced when the activity for the non-target allele further decreased. Accordingly, the designing method of the present invention may include a step of adjusting the length of a pre-crRNA (the length of a spacer sequence in an aspect), and/or a step of introducing a mismatch to a pre-crRNA (a spacer sequence in an aspect). For example, one to several (e.g., two, three, four, five, or more) mismatches may be introduced. In the step of introducing a mismatch to a pre-crRNA, the allele specificity may be further adjusted by adjusting the mismatch introduction position.

In the designing method of the present invention, it is not necessary to actually synthesize a pre-crRNA, and it is sufficient that the pre-crRNA is imagined conceptually. However, such an image of the nucleic acid is typically embodied on paper or in a program (e.g., nucleic acid design software, a graphic design tool, office software, etc.) running on an electronic computer. A designed pre-crRNA may be actually synthesized, and the allele specificity and the genome editing activity of the synthesized pre-crRNA may be evaluated.

A crRNA designed using the designing method of the present invention may be a wild-type pre-crRNA or an artificial pre-crRNA including a mutant pre-crRNA. All the contents described in "1. Pre-crRNA with Modified Nucleotide" above are applied to the definitions and types of the mutant nucleotide, the 5'-cap structure, and the 3' poly-A, the definition of a pre-crRNA, the production method, and the like.

### 3. Type I CRISPR-Cas System and Applications Thereof

Yet another aspect of the present invention provides a type I CRISPR-Cas system that includes the crRNA of the present invention. Specifically, provided is a type I CRISPR-Cas system (which may be referred to as the "CRISPR-Cas system of the present invention" hereinafter) that includes (1) the crRNA of the present invention, and (2) the Cascade protein group (Cse1, Cse2, Cas7, Cas5, and Cas6 in the case of the type I-E) or a nucleic acid encoding the protein group. The CRISPR-Cas system of the present invention may include (3) the Cas3 protein or a nucleic acid encoding the protein. When the type I CRISPR-Cas system is the type I-D system, the CRISPR-Cas system of the present invention may include the Cas3 protein or a nucleic acid encoding the protein, and the Cas10d protein or a nucleic acid encoding the protein. The nucleic acid encoding the Cas protein may be an RNA or a DNA. The CRISPR-Cas system of the present invention is the type I CRISPR-Cas system, but is preferably the CRISPR-Cas system of the type I-E (particularly the type I-E derived from E. coli) or the type I-D (particularly the type I-D derived from Microcystis aeruginosa) and more preferably the type I-E CRISPR-Cas system. The type, the definition, and the like of the type I CRISPR-Cas system are as described in "1. Pre-crRNA with Modified Nucleotide".

The CRISPR-Cas system of the present invention can be used to alter a double-stranded DNA of a cell. Accordingly, an aspect provides a method (which may be referred to as the "method of the present invention" hereinafter) for altering a double-stranded DNA of a cell or producing a cell with an altered double-stranded DNA, the method including a step of introducing the CRISPR-Cas system of the present invention to the cell. Examples of the double-stranded DNA of a cell include a chromosomal DNA, a mitochondrial DNA, a chloroplast DNA (these are also referred to collectively as the "genome DNA" hereinafter), an exogenous DNA (e.g., a plasmid DNA and a viral DNA), and the like, but the genome DNA is preferable, and the chromosomal DNA is particularly preferable. In this specification, the term "alteration" means deletion of a certain nucleotide or nucleotide sequence in a DNA strand, substitution of another nucleotide and/or nucleotide sequence for the certain nucleotide or nucleotide sequence, and/or insertion of a nucleotide or nucleotide sequence into a certain region in a DNA strand. Alteration in a genome DNA may also be referred to as the "genome editing".

Cas3, Cas10d, and the Cascade proteins may be a fusion protein with a heteroprotein having desired enzyme activity. Examples of the enzyme activity of the heteroprotein include deaminase activity (e.g., cytidine deaminase activity and adenosine deaminase activity), methyl transferase activity, demethylase activity, histone acetyltransferase activity, histone deacetylase activity, activator activity, transcriptional repressor activity, DNA repair activity, DNA damage activity, dismutase activity, alkylation activity, depurination activity, oxidation activity, pyrimidine dimer formation activity, integrase activity, transposase activity, recombinase activity, polymerase activity, ligase activity, photolyase activity, and glycosylase activity, but there is no limitation thereto. In this case, the nuclease activity and the helicase activity of Cas3 and Cas10d are not necessarily needed, and therefore, Cas3 and Cas10d need not be used. Alternatively, mutants that partially or entirely lack the nuclease activity and the helicase activity (e.g., a D domain H74A mutant (dnCas3), a SF2 domain motif 1 K320N mutant (dhCas3), and a SF2 domain motif 3 S483A/T485A double mutant (dh2Cas3)) may also be utilized.

For example, using a fusion protein of a deaminase and a mutant of Cas3 that partially or entirely lacks the nuclease activity as a constitutional element of the CRISPR-Cas3 system of the present invention makes it possible to conduct precise genome editing through substitution of a base without causing large deletion in the target site. For example, as described in WO 2017/043573, fusing the Cascade protein (e.g., Cas6) and a deaminase (e.g., hAID or PmCDA1) makes it possible to conduct pinpoint base alteration without using Cas3. Using a protein having methyl transferase activity (e.g., a DNA methyl transferase and a histone methyl transferase), a protein having demethylase activity (e.g., a DNA demethylase and a histone demethylase), a histone acetyltransferase, or a histone deacetylase makes it possible to modify a DNA without a change in the base sequence (i.e., epigenome editing).

A DNA encoding the Cas protein is typically provided in the form of an expression vector that includes the DNA. Examples of the expression vector include viral vectors such as a retrovirus, a lentivirus, an adenovirus, an adeno associated virus, a herpes virus, and a Sendai virus, plasmid vectors, episomal vectors, artificial chromosome vectors, transposon vectors (piggyBac, piggyBat, and TolII), and the like.

Examples of a promoter used in the expression vector include an EF1α promoter, an ACTB promoter, a UbqC promoter, a PGK promoter, a CAG promoter, an SRα promoter, an SV40 promoter, an LTR promoter, a CMV (cytomegalovirus) promoter, an RSV (Rous sarcoma virus) promoter, an MoMuLV (Moloney murine leukemia virus) LTR, an HIV LTR, an HSV-TK (herpes simplex virus thymidine kinase) promoter, and the like. Out of these promoters, the EF1α promoter, the ACTB promoter, the UbqC promoter, the PGK promoter, the CAG promoter, the SRα promoter, and the like are preferable.

The expression vector may optionally include an enhancer, a terminator, an IRES, a 2A coding sequence enhancer, a poly-A addition signal, an SV40 replication origin, a selection marker gene, and the like in addition to the promoter. Examples of the selection marker gene include a drug resistance gene, a fluorescent protein gene, and the like.

The expression vector may be a combination of individual expression vectors capable of respectively expressing the constituent elements (e.g., in the case of the type I-E, the Cse1 protein, the Cse2 protein, the Cas7 protein, the Cas5 protein, the Cas6 protein, the Cas3 protein, and the crRNA) of the CRISPR-Cas system, or one expression vector may be prepared so as to be capable of expressing a plurality of elements out of these elements, or one expression vector may be prepared so as to be capable of expressing all of these elements.

When one expression vector is prepared so as to be capable of expressing a plurality of elements out of these elements, the constituent elements of the CRISPR-Cas system may be linked to one another via the 2A sequence, which induces self-cleavage, the IRES (internal ribosome entry site) sequence having a ribosome-binding site, or the like. Examples of the 2A sequence include the P2A sequence derived from Porcine teschovirus, the T2A sequence derived from Thosea asigne, the F2A sequence derived from the foot-and-mouth disease virus, the E2A sequence derived from the equine rhinitis A virus, and the like. The IRES sequence may be a sequence derived from a virus such as the encephalomyocarditis virus and the foot-and-mouth disease virus, or a sequence derived from an mRNA in a cell. This makes it possible to individually express two or more proteins from a single mRNA.

The inventors of the present invention previously revealed that when three types of expression vectors, namely (1) two types of expression vectors in which one promoter drives three genes out of the Cse1 gene, the Cse2 gene, the Cas7 gene, the Cas5 gene, the Cas6 gene, and the Cas3 gene and (2) a pre-crRNA expression vector, were introduced, it was possible to efficiently induce genome editing even in pluripotent stem cells. Accordingly, in an aspect in which the Cascade protein group is introduced to stem cells in the form of an expression vector, an aspect in which two to four genes of the proteins included in the Cascade complex are expressed using one promoter is preferable, and an aspect in which the genes of three constituent proteins are expressed using one promoter is more preferable.

The inventors previously revealed that when three types of RNAs, namely (1) two types of mRNAs that each express three constituent proteins out of the Cse1 protein, the Cse2 protein, the Cas7 protein, the Cas5 protein, the Cas6 protein, and the Cas3 protein and (2) a wild-type pre-crRNA, were introduced, it was possible to efficiently conduct genome editing even in pluripotent stem cells. Accordingly, in an aspect in which the Cascade protein group is introduced to stem cells in the form of an mRNA, for example, an mRNA encoding two to four of the proteins included in the Cascade in a polycistronic manner or an mRNA encoding three constituent proteins in a polycistronic manner may be used.

The inventors of the present invention previously further revealed that in an aspect in which genome editing is conducted by introducing two types of expression vectors, namely (1) a single expression vector that has two promoters and in which each of the promoters drives three genes out of the Cse1 gene, the Cse2 gene, the Cas7 gene, the Cas5 gene, the Cas6 gene, and the Cas3 gene and (2) a pre-crRNA expression vector, the genome editing efficiency in pluripotent stem cells was greatly different between when the transcriptional directions of the two promoters were the same (uni-directional promoter) and when the transcriptional directions were opposite (bi-directional promoter). Then, they revealed that the genome editing efficiency was the highest when the transcriptional directions of the two promoters were opposite. Accordingly, in an aspect in which the Cascade protein group is introduced to stem cells (particularly pluripotent stem cells) in the form of an expression vector, an expression cassette form in which three genes are expressed using one promoter (two promoters in total) is preferable, and the two expression cassettes may be present in the same expression vector or different expression vectors. The most preferable aspect is that in which two expression cassettes with the three genes are arranged in opposite directions in the same expression vector (bi-directional promoter).

The promoter for expression of the Cas protein may be an expression-inducing promoter. An example of the expression-inducing promoter is a promoter capable of inducing expression in response to addition of an expression control substance to a culture medium, removal of an expression control substance from a culture medium, photoirradiation, a change in temperature, or the like. The expression-inducing promoter may induce protein expression in response to addition of an expression control substance to a culture medium, or may induce protein expression in response to removal of an expression control substance from a culture medium. Specific examples of the expression-inducing promoter include a Tet-ON/Tet-OFF promoter (which induces the expression in response to addition or removal of tetracycline or a derivative thereof (e.g., doxycycline)), a metallothionein promoter (which induces the expression in response to the presence of a heavy metal), a heat-shock protein promoter (which induces the expression in response to heat shock), a steroid-responsive promoter (which induces the expression in response to the presence of a steroid hormone or a derivative thereof), and the like, but there is no limitation thereto.

A nucleic acid (e.g., the crRNA of the present invention), an expression vector that includes the nucleic acid, or a protein (e.g., Cas3, Cas10d, or proteins (also referred to as the "Cascade proteins" hereinafter) included in the Cascade complex) can be introduced to cells using various known methods. Examples of such methods include calcium phosphate-mediated transfection, electroporation, liposome transfection, lipofection, a gene gun, microinjection, a viral vector method, a virus-like particle method, an agrobacterium method, an agroinfiltration method, a PEG-calcium method, a sonoporation method, a lipid nanoparticle method, and the like.

The CRISPR-Cas system of the present invention may be introduced to cells a plurality of times. In a general genome editing experiment in which the CRISPR-Cas9 system is used, introduction is basically conducted once because of introduction of deletion or mutation to the target sequence and a high off-target risk. However, with the type I CRISPR-Cas system, a large deletion is introduced on the PAM sequence side of the target sequence, but a sequence (i.e., target sequence) targeted by a crRNA is conserved in many cases, thus making it possible to enhance the deletion efficiency and to extend (accumulate) the deletion site by introducing the same crRNA to the same cells a plurality of times. It is known that the off-target efficiency of the type I CRISPR-Cas system is lower than that of the type II CRISPR-Cas system, and therefore, the frequency of the introduction is not particularly limited either, but is typically two to ten times (e.g., two, three, or four times). Also, the introduction interval is not particularly limited either, but the CRISPR-Cas system introduction is introduced at intervals of 1 to 15 days, preferably every one day, two days, three days, four days, five days, six days, seven days, eight days, nine days, or ten days. Cells to which the CRISPR-Cas system has been introduced may be cryopreserved and then thawed after several days to several years, followed by another introduction of the CRISPR-Cas system to the cells.

Even when a CRISPR-Cas system in which a wild-type pre-crRNA is used instead of the crRNA of the present invention is used in the CRISPR-Cas system of the present invention, it is possible to enhance the deletion efficiency and extend (accumulate) the deletion site in the same manner. Accordingly, yet another aspect provides a method for extending a deletion site, the method including a step of introducing a type I CRISPR-Cas system to cells a plurality of times. Such a CRISPR-Cas system typically includes (I) a wild-type pre-crRNA, (II) the Cas3 protein or a nucleic acid encoding the protein, and (III) the Cascade protein group or a nucleic acid encoding the protein group. The CRISPR-Cas system is preferably the CRISPR-Cas system of the type I-E (particularly the type I-E derived from E. coli) or the type I-D (particularly the type I-D derived from Microcystis aeruginosa) and more preferably the type I-E CRISPR-Cas system. When the CRISPR-Cas system is the type I-D CRISPR-Cas system, the CRISPR-Cas system may further include the Cas10d protein or a nucleic acid encoding the protein. The type, the definition, and the like of the type I CRISPR-Cas system are as described in "1. Pre-crRNA with Modified Nucleotide".

A DNA encoding the Cas protein can be obtained by, for example, isolating a region that includes the ORF of the desired Cas protein from the cas operon through genome PCR using the genome DNA derived from the bacterial species above as a template. Also, a DNA encoding the Cas protein can be cloned by synthesizing an oligo DNA primer based on the information (e.g., information from the database such as NCBI GenBank) of the cDNA sequence or amino acid sequence of a protein for use, and conducting amplification through the RT-PCR method using, as a template, the total RNA or mRNA fraction prepared from cells producing the protein. Examples of the NCBI Accession No. of the Cse1 protein belonging to the type I-E derived from E. coli include NP_417240.1 and the like. Examples of the NCBI Accession No. of the Cse2 protein include NP_417239.1 and the like. Examples of the NCBI Accession No. of the Cas7 protein include NP_417238.1 and the like. Examples of the NCBI Accession No. of the Cas5 protein include NP_417237.2 and the like. Examples of the NCBI Accession No. of the Cas6 protein include NP_417236.1 and the like. Examples of the NCBI Accession No. of the Cas3 protein include NP_417241.1 and the like. Other Cas proteins are also available from NCBI GenBank and other databases. It is also possible to obtain a sequence from a novel microbial species by using the BLAST program for microbial genome data obtained through metagenomic analysis.

The Cas protein may have an amino acid sequence obtained through substitution, deletion, addition, and/or insertion of one or several (e.g., two, three, four, five, six, seven, eight, nine, ten, or more) amino acids in the amino acid sequence represented by Accession No. above as long as the functions of the protein are retained. Also, the Cas protein may have an amino acid sequence having 70% or more identity, preferably 80% or more identity, more preferably 90% or more identity, and even more preferably 95% or more (e.g., 96%, 97%, 98%, 99%, or more) identity to the amino acid sequence represented by Accession No. above. The identity between amino acid sequences can be calculated using the homology calculation algorithm NCBI BLAST (National Center for Biotechnology Information Basic Local Alignment Search Tool).

A DNA encoding a fusion protein can be prepared by ligating, to Cas3, Cas10d, or the Cascade proteins, the cloned DNA as it is or optionally after digestion with a restriction enzyme or addition of a sequence encoding an appropriate linker and/or a nuclear localization signal (or an organelle localization signal when the double-stranded DNA of interest is a mitochondria DNA or a chloroplast DNA). Only one organelle localization signal including the nuclear localization signal may be added, or a plurality of organelle localization signals may be added (for examples, to both the N terminus and the C terminus of the protein). A method for producing a DNA encoding a pre-crRNA is as described in "1. Pre-crRNA with Modified Nucleotide" above.

An RNA encoding Cas3, Cas10d, or the Cascade proteins can be synthesized through, for example, IVT reaction using a DNA encoding Cas3, Cas10d, or the Cascade proteins as a template.

Cas3, Cas10d, or the Cascade proteins can also be produced using the in-vitro translation system. Alternatively, Cas3, Cas10d, or the Cascade proteins can also be obtained by expressing the protein in a cell using an expression vector or the like and isolating or purifying the protein from the cell.

The cells used in the method of the present invention include, for example, bacteria of the genus Escherichia, bacteria of the genus Bacillus, yeast, insect cells, insects, animal cells, plant cells, and the like.

As the genus Escherichia, Escherichia coli K12·DH1 [Proc. Natl. Acad. Sci. USA, 60, 160 (1968)], Escherichia coli JM103 [Nucleic Acids Research, 9, 309 (1981)], Escherichia coli JA221 [Journal of Molecular Biology, 120, 517 (1978)], Escherichia coli HB101 [Journal of Molecular Biology, 41, 459 (1969)], Escherichia coli C600 [Genetics, 39, 440 (1954)] and the like are used.

As the genus Bacillus, Bacillus subtilis MI114 [Gene, 24, 255 (1983)], Bacillus subtilis 207-21 [Journal of Biochemistry, 95, 87 (1984)] and the like are used.

As the yeast, Saccharomyces cerevisiae AH22, AH22R⁻, NA87-11A, DKD-5D, 20B-12, Schizosaccharomyces pombe NCYC1913, NCYC2036, Pichia pastoris KM71 and the like are used.

As examples of insect cells, there are, for example, cells of a cabbage armyworm larvaderived established line (Spodoptera frugiperda cells; Sf cells), MG1 cells derived from the midintestine of Trichoplusia ni, High Five (trademark) cells derived from an egg of Trichoplusia ni, Mamestra brassicae-derived cells, Estigmena acrea-derived cells, and cells of a Bombyx moriderived established line (Bombyx mori N cells; BmN cells), and the like. As examples of Sf cells, Sf9 cells (ATCC CRL-1711), Sf21 cells [all above, In Vivo, 13, 213-217 (1977)], and the like are used.

As examples of plant cells, suspension-cultured cells, callus, protoplasts, leaf segments, root segments, and the like prepared from various plants (e.g., grains such as rice, wheat, corn, and the like; produce crops such as tomato, cucumber, eggplant, and the like; garden plants such as carnation, Eustoma russellianum, and the like; experimental plants such as tobacco, Arabidopsis thaliana, and the like; and the like) are used.

As examples of animal cells, cells isolated from a living organism, cells in a living organism, cell strains such as monkey COS-7 cells, monkey Vero cells, Chinese hamster ovarian (CHO) cells, mouse L cells, mouse AtT-20 cells, mouse myeloma cells, rat GH3 cells, HeLa cells, and human FL cells, pluripotent stem cells of human and other mammals (a mouse, a rat, a dog, a monkey, and the like), and primary cultured cells prepared from various tissues are used. Furthermore, zebrafish embryos, xenopus oocytes and the like can also be used. The animal cells used in the method of the present invention are preferably mammalian cells, and examples of the mammal include rodents such as a mouse, a rat, a hamster, and a guinea pig, primates such as a human, a rhesus monkey, a crab-eating macaque, a Japanese macaque, and a chimpanzee, a cow, a horse, a dog, a cat, and the like.

The method of the present invention is expected to exhibit a high genome editing effect on even pluripotent stem cells, for which the genome editing efficiency of a conventional method is low. Accordingly, it is preferable to use pluripotent stem cells in the method of the present invention.

As used herein, the term "pluripotent stem cells" refers to stem cells that can differentiate into tissue or cells having various different forms or functions in the body, and that can also differentiate into cells of any lineages of the three germ layers (endoderm, mesoderm and ectoderm). Examples of pluripotent stem cells to be used for the present invention include induced pluripotent stem cells (iPS cells), embryonic stem cells (ES cells), embryonic stem cells from cloned embryos obtained by nuclear transfer (nuclear transfer Embryonic stem cells; ntES cells), multipotent germline stem cells (mGS cells) and embryonic germ cells (EG cells), of which iPS cells (and especially human iPS cells) are preferred. When the pluripotent stem cells are ES cells or arbitrary cells derived from human embryos, the cells may be obtained by destruction of the embryo or they may be obtained without destruction of the embryo, but preferably they are cells obtained without destruction of the embryo.

ES cells are stem cells having pluripotency and auto-replicating proliferation potency, established from the inner cell mass of an early embryo (such as the blastocyst) of a mammal such as a human or mouse. ES cells were discovered in mice in 1981 (M.J. Evans and M.H. Kaufman (1981), Nature 292:154-156), and ES cell lines were later established in primates including humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cells can be established by extracting the inner cell mass from the blastocyst of a fertilized egg of a target animal and culturing the inner cell mass on a fibroblast feeder. Alternatively, ES cells can be established using a single embryo blastomere alone during the cleavage stage before the blastocyst stage (Chung Y. et al. (2008), Cell Stem Cell 2: 113-117), or they can be established using a developmentally arrested embryo (Zhang X. et al. (2006), Stem Cells 24: 2669-2676.).

The ES cell line used for the present invention may be mouse ES cells, and for example, the different mouse ES cell lines established by the inGenious Targeting Laboratory, Riken (Riken Research Institute), etc., may be used, or for human ES cell lines, the different human ES cell lines established by the University of Wisconsin, NIH, Riken, Kyoto University, the National Center for Child Health and Development, Cellartis, etc., for example, may be used. Specific examples of human ES cell lines include CHB-1 to CHB-12 lines, RUES1 line, RUES2 line and HUES1 to HUES28 lines, etc., distributed by ESI Bio Co., H1 and H9 lines, etc., distributed by WiCell Research, KhES-1 line, KhES-2 line, KhES-3 line, KhES-4 line, KhES-5 line, SSES1 line, SSES2 line and SSES3 line distributed by Riken, etc.

iPS cells are cells obtained by reprogramming of mammalian somatic cells or undifferentiated stem cells by transfer of specific factors (nuclear reprogramming factors). A large number of iPS cells currently exist, among which there may be used iPSCs established by transfer of the 4 factors Oct3/4·Sox2·Klf4·c-Myc into mouse fibroblasts by Yamanaka et al. (Takahashi K, Yamanaka S., Cell, (2006) 126: 663-676), iPSCs derived from human cells established by transfer of the same 4 factors into human fibroblasts (Takahashi K, Yamanaka S., et al. Cell, (2007) 131:861-872.), Nanog-iPSCs established by selecting of Nanog expression markers after transfer of the 4 factors (Okita, K., Ichisaka, T. and Yamanaka, S. (2007). Nature 448, 313-317.), iPSCs produced by methods without c-Myc (Nakagawa M, Yamanaka S., et al. Nature Biotechnology, (2008) 26, 101-106), and iPSCs established by transfer of 6 factors by a virus-free method (Okita K et al. Nat. Methods 2011 May; 8(5):409-12, Okita K et al. Stem Cells. 31(3):458-66.), and so on. The induced pluripotent stem cells established by transfer of the 4 factors OCT3/4·SOX2·NANOG·LIN28, created by Thomson et al. (Yu J., Thomson JA. et al., Science (2007) 318: 1917-1920), the induced pluripotent stem cells created by Daley et al. (Park IH, Daley GQ. et al., Nature (2007) 451:141-146) and the induced pluripotent stem cells created by Sakurata et al. (Japanese Unexamined Patent Publication No. 2008-307007), and so on, may also be used.

Induced pluripotent stem cell lines that are iPSC lines established by NIH, Riken, Kyoto University, etc., for example, may be used. Examples include human iPSC lines such as HiPS-RIKEN-1A line, HiPS-RIKEN-2A line, HiPS-RIKEN-12A line and Nips-B2 line, etc., by Riken, and 253G1 line, 253G4 line, 1201C1 line, 1205D1 line, 1210B2 line, 1383D2 line, 1383D4 line, 1383D6 line, 201B7 line, 409B2 line, 454E2 line, 606A1 line, 610B1 line, 648A1 line, 1231A3 line, Ff-I01s04 line and Ff-I14s04 line by Kyoto University, etc.

Cells to which the CRISPR-Cas system has been introduced can be cultured in accordance with a known method depending on the type of cell.

For example, when E. coli or a bacterium of the genus Bacillus is cultured, it is preferable to use a liquid culture medium as the culture medium for the culture. It is preferable that the culture medium contains a carbon source, a nitrogen source, inorganic substances, and the like that are necessary for growth of a transformant. Examples of the carbon source include glucose, dextrin, soluble starch, sucrose, and the like; examples of the nitrogen source include inorganic or organic substances such as ammonium salts, nitrates, corn steep liquor, peptone, casein, meat extract, soybean cake, potato extract, and the like, and examples of the inorganic substances include calcium chloride, sodium dihydrogenphosphate, magnesium chloride, and the like. Yeast extract, vitamins, a growth promoting factor, and the like may be added to the culture medium. The pH of the culture medium is preferably about 5 to about 8.

A preferable culture medium for culture of E. coli is, for example, the M9 culture medium containing glucose and casamino acid (Journal of Experiments in Molecular Genetics, 431-433, Cold Spring Harbor Laboratory, New York 1972) and the LB culture medium. For example, in order to efficiently operate the promoter, a drug such as 3β-indolylacrylic acid may be added to the culture medium as needed. E. coli is typically cultured at about 15°C to about 43°C. Aeration or stirring may be conducted as needed.

A bacterium of the genus Bacillus is typically cultured at about 30°C to about 40°C. Aeration or stirring may be conducted as needed.

Examples of a culture medium for culture of yeast include the Burkholder minimum culture medium (Proc. Natl. Acad. Sci. USA, 77, 4505 (1980)), the SD culture medium containing 0.5% casamino acid (Proc. Natl. Acad. Sci. USA, 81, 5330 (1984)), and the like. The pH of the culture medium is preferably about 5 to about 8. The culture is typically conducted at about 20°C to about 35°C. Aeration or stirring may be conducted as needed.

As examples of a culture medium for culture of insect cells or insects, the Grace's Insect Medium (Nature, 195, 788 (1962)) to which additives such as 10% inactivated bovine serum are added as appropriate is used. The pH of the culture medium is preferably about 6.2 to about 6.4. The culture is typically conducted at about 27°C. Aeration or stirring may be conducted as needed.

As examples of a culture medium for culture of animal cells, the minimum essential medium (MEM) containing about 5% to about 20% fetal bovine serum (Science, 122, 501 (1952)), the Dulbecco's modified Eagle medium (DMEM) (Virology, 8, 396 (1959)), the RPMI 1640 culture medium (The Journal of the American Medical Association, 199, 519 (1967)), the 199 culture medium (Proceeding of the Society for the Biological Medicine, 73, 1 (1950)), and the like are used. Examples of a culture medium for culture of pluripotent stem cells such as human iPS cells include the mTeSR culture medium, the Essential-8 culture medium, the StemFit AK03N culture medium, and the like. The pH of the culture medium is preferably about 6 to about 8. The culture is typically conducted at about 30°C to about 40°C. Aeration or stirring may be conducted as needed.

Examples of a culture medium for culture of plant cells include the MS culture medium, the LS culture medium, the B5 culture medium, and the like. The pH of the culture medium is preferably about 5 to about 8. The culture is typically conducted at about 20°C to about 30°C. Aeration or stirring may be conducted as needed.

Also, yet another aspect provides an agent for altering a target sequence of a double-stranded DNA and an agent for regulating a target gene expression (which are also referred to as the "agents of the present invention" hereinafter) that include the CRISPR-Cas system of the present invention. These agents can also be used to treat or prevent a disease (e.g., the repeat disease above) of a mammal. Examples of the mammal include rodents such as a mouse, a rat, a hamster, and a guinea pig, primates such as a human, a rhesus monkey, a crab-eating macaque, a Japanese macaque, and a chimpanzee, a cow, a horse, a dog, a cat, and the like.

The agents of the present invention can be administered to a mammal orally or parenterally (e.g., through subcutaneous injection, intramuscular injection, intravenous infusion, topical infusion (topical external use, topical application), intraventricular administration, intraspinal administration, and intraperitoneal administration). The dosage form for oral administration is a solid or liquid, and specific examples include a tablet (including a sugar-coated tablet and a film-coated tablet), a pill, a granule, a powder, a capsule (including a soft capsule), a syrup, an emulsion, a suspension, and the like. Meanwhile, examples of the dosage form for parenteral administration include an agent for external use (e.g., an ointment, a cream, and a liquid agent for external use), an injection, a suppository, and the like, and the injection may encompass the dosage forms such as an intravenous injection, a subcutaneous injection, an intracutaneous injection, an intramuscular injection, and a drip infusion.

The CRISPR-Cas system of the present invention may be formulated with any carrier (e.g., a pharmacologically acceptable carrier). Examples of the pharmacologically acceptable carrier include vehicles such as sucrose and starch, binding agents such as cellulose and methyl cellulose, disintegrating agents such as starch and carboxymethyl cellulose, lubricants such as magnesium stearate and Aerosil, aromatic agents such as citric acid and menthol, preservatives such as sodium benzoate and sodium hydrogen sulfite, stabilizing agents such as citric acid and sodium citrate, suspending agents such as methyl cellulose and polyvinyl pyrrolidone, dispersing agents such as a surfactant, diluting agents such as water and a physiological saline solution, base wax, and the like, but there is no limitation thereto.

When the active ingredient of the agent of the present invention is in the form of a nucleic acid, the agent of the present invention may further contain a reagent for introduction of a nucleic acid for the purpose of promoting the introduction of the nucleic acid to target cells. Examples of the reagent for introduction of a nucleic acid include calcium chloride, a calcium enrichment reagent, atelocollagen, a liposome, a nanoparticle, a lipofectin, a lipofectamine, DOGS (Transfectam), DOPE, DOTAP, DDAB, DHDEAB, HDEAB, polybrene, and a cationic lipid such as poly(ethylenimine) (PEI).

### 4. Single-Stranded Annealing (SSA) Reporter Nucleic Acid and Applications Thereof

Another aspect of the present invention provides an SSA reporter nucleic acid for the type I CRISPR-Cas system. Specifically, provided is a double-stranded nucleic acid (which may be referred to as the "long-chain insertion-type nucleic acid of the present invention" hereinafter) for evaluating activity of a type I CRISPR-Cas system, wherein one nucleic acid strand includes:
(1) a sequence encoding a portion of a reporter protein (i.e., sequence of a portion of a reporter gene (also referred to as a "selection marker gene");
(2) an insertion sequence that includes a sequence targeted by a crRNA or a sequence complementary to the sequence targeted by the crRNA; and
(3) a sequence that encodes a portion of the reporter protein and is different from the sequence of (1), in this order,

the sequences of (1) and (3) have an overlapping sequence, the sequence of (1) excluding the overlapping sequence, the overlapping sequence, and the sequence of (3) excluding the overlapping sequence constituting a sequence encoding the entire reporter protein, and
the insertion sequence of (2) has a length of 100 to 3000 bases.

Alternatively, provided is a double-stranded nucleic acid (which may be referred to as the "double nicking-type nucleic acid of the present invention" hereinafter) for evaluating activity of a type I CRISPR-Cas system, wherein one nucleic acid strand includes:
(I) a sequence encoding a portion of a reporter protein;
(II) an insertion sequence that includes a sequence targeted by a crRNA and a sequence complementary to the sequence targeted by the crRNA; and
(III) a sequence that encodes a portion of the reporter protein and is different from the sequence of (I), in this order, and
the sequences of (I) and (III) have an overlapping sequence, the sequence of (I) excluding the overlapping sequence, the overlapping sequence, and the sequence of (III) excluding the overlapping sequence constituting a sequence encoding the entire reporter protein. In the description below, the "long-chain insertion-type nucleic acid of the present invention" and the "double nicking-type nucleic acid of the present invention" may be referred to collectively as the "SSA reporter nucleic acid of the present invention".

In the SSA reporter nucleic acid of the present invention, the DNA is cleaved in the vicinity of the target sequence by the type I CRISPR-Cas system, and then a single-stranded DNA region is formed. The sequences of (1) and (3) or (I) and (III) have an overlapping sequence, and annealing reaction occurs between the overlapping sequences, so that the DNA damage in the SSA reporter nucleic acid is repaired. As a result, a sequence encoding the entire reporter protein occurs in the SSA reporter nucleic acid, and the protein is expressed. That is to say, using, as an index, whether or not the reporter protein is expressed, or the expression level of the reporter protein makes it possible to evaluate the presence or absence of the activity of the type I CRISPR-Cas system in cells, or the degree of the activity. The SSA reporter nucleic acid of the present invention can be applied to the type I CRISPR-Cas system, but the type I CRISPR-Cas system is preferably the CRISPR-Cas system of the type I-E (particularly the type I-E derived from E. coli) or the type I-D (particularly the type I-D derived from Microcystis aeruginosa) and more preferably the type I-E CRISPR-Cas system. The type, the definition, and the like of the type I CRISPR-Cas system are as described in "1. Pre-crRNA with Modified Nucleotide".

Examples of the reporter protein for the SSA reporter nucleic acid of the present invention include a fluorescent protein, a luminescent enzyme, a chromogenic enzyme, a drug-resistant protein, and the like, but there is no limitation thereto. Examples of the fluorescent protein include blue fluorescent proteins such as Sirius, TagBFP, and EBFP, cyan fluorescent proteins such as mTurquoise, TagCFP, AmCyan, mTFP1, MidoriishiCyan, and CFP, green fluorescent proteins such as TurboGFP, AcGFP, TagGFP, Azami-Green (e.g., hmAG1), GFP, ZsGreen, EmGFP, EGFP, GFP2, and HyPer, yellow fluorescent proteins such as TagYFP, EYFP, Venus, YFP, PhiYFP, PhiYFP-m, TurboYFP, ZsYellow, and mBanana, orange fluorescent proteins such as KusabiraOrange (e.g., hmKO2), and mOrange, red fluorescent proteins such as TurboRFP, DsRed-Express, DsRed2, TagRFP, DsRed-Monomer, AsRed2, and mStrawberry, and near-infrared fluorescent proteins such as TurboFP602, mRFP, JRed, KillerRed, mCherry, HcRed, KeimaRed (e.g., hdKeimaRed), mRasberry, and mPlum, but there is no limitation thereto. Examples of the luminescent enzyme include firefly luciferase, bacterial luciferase, synthetic Renilla luciferase, Oplophorus gracilirostris luciferase (e.g., NanoLuc (registered trademark)), secretory luciferase, and the like. Examples of the chromogenic enzyme include β-galactosidase, β-glucuronidase, alkaline phosphatase, and the like. Examples of the drug-resistant protein include a kanamycin-resistant protein, an ampicillin-resistant protein, a neomycin-resistant protein, a puromycin-resistant protein, a blastcidin-resistant protein, a Zeocin-resistant protein, and the like.

In the long-chain insertion-type nucleic acid of the present invention, the length of the insertion sequence is typically 100 bases or more (e.g., 200 bases, 300 bases, 400 bases, 500 bases, or more) and 3000 bases or less (e.g., 2500 bases, 2000 bases, 1900 bases, 1800 bases, 1700 bases, or less), preferably 500 bases to 2000 bases, and more preferably 1000 bases to 1700 bases. In an aspect, the length of the insertion sequence is 1000 bases. The left diagram in FIG. 27 shows a schematic diagram of an example of the long-chain insertion-type nucleic acid.

In the double nicking-type nucleic acid of the present invention, the insertion sequence includes a sequence (i.e., target sequence) targeted by the crRNA and a sequence complementary to the target sequence. This allows both strands of the double-stranded nucleic acid to include the target sequence, and it is thus considered that the two target sequences are arranged in the form of a palindromic sequence. The CRISPR-Cas system acts on both the strands to introduce DNA cleavage (DNA nick or the like), thus causing DNA repair reaction via SSA. A sequence serving as a spacer (which may be referred to as the "SSA spacer sequence" hereinafter) may or may not be present between the 3'-terminal base of the sequence targeted by the crRNA and the 5'-terminal base of the sequence complementary to the target sequence, but it is preferable that such a sequence is not present. When the SSA spacer sequence is present, the length thereof is not particularly limited, but is typically 1 base to 100 bases, preferably 1 base to 50 bases, and more preferably 15 bases to 35 bases. Accordingly, in the double nicking-type nucleic acid of an embodiment of the present invention, the SSA spacer sequence (the length is typically 1 base to 100 bases) is present between the 3'-terminal base of the sequence targeted by the crRNA of (II) and the 3'-terminal base of the sequence complementary to the target sequence. The right diagram in FIG. 27 shows a schematic diagram of an example of the double nicking-type nucleic acid. In the double nicking-type nucleic acid, the length of the insertion sequence is not particularly limited, but is typically 64 bases or more (e.g., 100 bases, 150 bases, 200 bases, or more) and 1000 bases or less (e.g., 800 bases, 600 bases, 400 bases, or less).

In this specification, the term "complementary" means a relationship between nucleic acid bases that can form the so-called Watson-Crick base pair (natural base pairing) or a non-Watson-Crick base pair (e.g., a Hoogsteen base pair, a wobble base pair, or the like) via a hydrogen bond. Therefore, the wording "complementary sequence" implicates not only a sequence that is completely complementary to (i.e., capable of hybridizing, without mismatches, with) a sequence of interest (e.g., the sequence targeted by the crRNA), but also sequences with one to several (e.g., two, three, four, five, or more) mismatches as long as they can hybridize with a target sequence under stringent conditions or under physiological conditions for mammalian cells. That is to say, it is allowable that the complementary strand includes mismatches. Examples of such sequences include those having 80% or more (e.g., 85% or more, 90% or more, 95% or more, 96% or more, 97% or more, 98% or more, or 99% or more) identity to a sequence that is completely complementary to a sequence of interest, and a sequence having 100% identity thereto is most preferable. The identity between base sequences in this specification can be calculated using the homology calculation algorithm NCBI BLAST.

As described above, the SSA reporter nucleic acid of the present invention can be used to evaluate the degree of the activity of the type I CRISPR-Cas system. Accordingly, another aspect provides a method (which may be referred to as the "evaluation method of the present invention" hereinafter) for evaluating the degree of the activity of the type I CRISPR-Cas system, the method including (A) a step of introducing the SSA reporter nucleic acid of the present invention to cells to be evaluated for the activity of the type I CRISPR-Cas system, and (B) a step of evaluating the degree of the activity of the type I CRISPR-Cas system through a step of detecting or measuring the expression of a reporter protein in the cells to which the SSA reporter nucleic acid has been introduced in the step (A).

The cells to which the type I CRISPR-Cas system has been introduced in the step (A) may be cells to which the type I CRISPR-Cas system (i.e., a pre-crRNA or a nucleic acid encoding the RNA, and the Cas proteins or a nucleic acid encoding the proteins) has been introduced in advance, or cells to which the type I CRISPR-Cas system was introduced simultaneously with the step (A), or cells to which the type I CRISPR-Cas system was introduced after the step (A). The nucleic acid encoding the proteins may be an RNA or a DNA. Alternatively, the cells to which the type I CRISPR-Cas system has been introduced in the step (A) may be cells having a nucleic acid in which the Cas proteins are linked under the control of the expression-inducing promoter. In this case, the addition of an expression-inducing factor corresponding to the promoter to the culture medium or the removal thereof from the culture medium makes it possible to express the type I CRISPR-Cas system at desired timing.

When the reporter protein is a fluorescent protein, a luminescent enzyme, or a chromogenic enzyme, the step (B) can be conducted by, for example, detecting or measuring the fluorescence intensity, the luminescent intensity, or the chromogenic intensity of the cells using a known method. When the reporter protein is a drug-resistant protein, the step (B) can be conducted by, for example, confirming whether or not the cells are viable in the culture medium to which a corresponding drug has been added, or adjusting the concentration of the drug in the culture medium. Alternatively, the detection or measurement can also be conducted through Western blotting, immunostaining, enzyme immunoassay (e.g., EIA or ELISA), or the like using an antibody that specifically recognizes the protein.

It is assumed that the genome editing efficiency enhances in proportion to the activity of the type I CRISPR-Cas system, and therefore, it is also possible to concentrate genome-edited cells using the SSA reporter nucleic acid of the present invention. Accordingly, yet another aspect provides a method (which may be referred to as the "cell sorting method of the present invention" hereinafter) for sorting cells, the method including (a) a step of introducing the SSA reporter nucleic acid of the present invention to cells of interest, and (b) a step of sorting cells expressing a reporter protein from the cells to which the SSA reporter nucleic acid has been introduced in the step (a).

The step (b) is not particularly limited as long as the cells expressing the reporter protein can be sorted, but when the reporter protein is a fluorescent protein, the cells can be sorted using a cell sorter such as FACS (fluorescence-activated cell sorter) using the fluorescent as an index. Alternatively, when the reporter protein is present on the cell surface, magnetic beads or agarose beads to which a substance (e.g., antibody) capable of binding to the reporter protein has been linked can be used to sort the cell population expressing the reporter protein. When the reporter protein is a drug-resistant protein, the step (b) can be conducted by adding a drug to the culture medium and sorting viable cells.

The SSA reporter nucleic acid of the present invention is typically provided in the form of an expression vector that includes the nucleic acid. All the contents described in "1. Pre-crRNA with Modified Nucleotide" above and "3. Type I CRISPR-Cas System and Applications Thereof" above are applied to the type of expression vector, the type and description of the promoter (including the expression-inducing promoter) and other constituent elements used in the vector, the SSA reporter nucleic acid of the present invention, the method for introducing an expression vector that includes the nucleic acid or the Cas proteins to cells, the type of cells used in the present invention, the method for culturing the cells, and the like.

In the evaluation method of the present invention and the sorting method of the present invention, only one type of the SSA reporter nucleic acid of the present invention may be used, or two or more types of the SSA reporter nucleic acids may be used. When two or more types of the SSA reporter nucleic acids are used, using, for example, SSA reporter nucleic acids that differ in the sequence targeted by the crRNA and in the sequence encoding the reporter protein makes it possible to evaluate a plurality of crRNAs for the target recognition ability through a single operation and to sort only cells in which a plurality of target sites simultaneously undergo genome editing. In these cases, it is preferable to use, as the reporter protein, a combination of proteins different in the fluorescent color (e.g., a combination of a green fluorescent protein such as EGFP and a red fluorescent protein such as mRFP), or a combination of reporter proteins of different types (e.g., a combination of a fluorescent protein and a drug-resistant protein).

Alternatively, using two or more types of the SSA reporter nucleic acids of the present invention to evaluate a crRNA designed using the designing method of the present invention also makes it possible to evaluate the specificity of the crRNA. For example, insertion sequences different in one base in the sequence targeted by the crRNA are used in two types of SSA vectors for expression of EGFP or mRFP as the reporter protein, thus making it possible to analyze which of EGFP and mRFP preferentially sends out a signal. As shown in Examples below, when a SNP sequence "C" of rs934739524 was inserted into the SSA reporter nucleic acid with EGFP, a SNP sequence "T" was inserted into the SSA reporter nucleic acid with mRFP, and genome editing was conducted using crRNAs targeting these SNP sequences, it was shown that the EGFP signal was preferentially observed in the crRNA targeting "C" and the mRFP signal was preferebtially observed in the crRNA targeting "T".

The SSA reporter nucleic acid of the present invention may also be provided in the form of a kit. The kit may contain only one type of the SSA reporter nucleic acid of the present invention, or a plurality of types of the SSA reporter nucleic acids. The kit may also contain a drug for cell sorting, a culture medium for cell culture, cells, a reagent for introducing a nucleic acid or protein, an operation manual describing the procedure of the evaluation method or cell sorting method of the present invention, and the like.

### 5. Method for Analyzing Pattern of Editing by CRISPR-Cas System

As shown in Examples below, a plurality of droplet digital PCR (ddPCR) probes were designed at various positions around the target sequence, and the DNA copy number at each position was directly measured in the genome DNA of the genome-edited cell, thus making it possible to capture a situation where the genome editing efficiency changed. Accordingly, another aspect of the present invention provides a method (which may be referred to as the "analysis method of the present invention" hereinafter) for analyzing an editing pattern of a double-stranded DNA edited by the type I CRISPR-Cas system, the method including a step of conducting digital PCR using a plurality of PCR probes for a deletion target site. Using the analysis method of the present invention makes it possible to easily analyze the editing pattern of the double-stranded DNA (e.g., the length and the ratio of a deletion mutation).

As described above, it is known that the type I-E CRISPR-Cas system typically introduces a long-chain deletion on the 5'-side of the target sequence, and the type I-D CRISPR-Cas system typically introduces an indel (insertion/deletion) to the target site or a vicinity thereof and a long-chain deletion on the 5'-side and 3'-side of the target sequence. The class-1 CRISPR-Cas system is preferably the CRISPR-Cas system of the type I-E (particularly the type I-E derived from E. coli) or the type I-D (particularly the type I-D derived from Microcystis aeruginosa) and more preferably the type I-E CRISPR-Cas system.

In this specification, the term "deletion target site" means a site that is expected to be deleted by the introduced CRISPR-Cas system. The type I CRISPR-Cas system can delete a region of 100 or more (e.g., 1000, 10000, or more) nucleotides on the upstream side and/or the downstream side of the PAM sequence, thus making it possible to design a pair of a probe and a primer for the region. When a plurality of pre-crRNAs or guide RNAs are used, the region surrounded by the regions targeted by these RNAs is also the deletion target site.

Arranging probes on sequences targeted by the probes at narrow intervals enables detailed analysis, whereas the number of probes designed increases. The interval between the probes can be adjusted as appropriate depending on the purpose of the analysis, but the probes are typically set at an interval of 100 bases to 2000000 bases (e.g., an interval of 1000 bases to 100000 bases, or an interval of 3000 bases to 70000 bases). The length of each probe is typically 5 bases to 40 bases, preferably 10 bases to 35 bases, and more preferably 18 bases to 30 bases.

A fluorescent dye can be linked to each probe. Various fluorescent dyes are commercially available, and examples thereof include 6-FAM (fluorescein), HEX, TE, Quasar 670, Quasar 570, Quasar 705, Pulsar 650, TET, HEX, VIC, JOE, CAL Fluor Orenge, CAL Fluor Gold, CAL Fluor Red, Texas Red, Cy, Cy5, and the like. It is preferable that a quencher capable of quenching the fluorescence from the fluorescent dye is further linked to each probe. The quencher is not particularly limited as long as it can quench the fluorescence from the fluorescent dye, and it may be a fluorescent dye or a non-fluorescent dye, but a non-fluorescent dye is preferable from the viewpoint of detection accuracy. Specific examples of the quencher include Black Hole Quencher 1 (BHQ1), 6-carboxytetramethylrhodamine (TAMRA), 6-carboxy-X-rhodamine (ROX), Eclipse Dark Quencher, Iowa black FQ (IBFQ), minor groove binders (MGBs), non-fluorescent quenchers (NFQs), and the like. Examples of a combination of the fluorescent dye and the quencher include a combination of 6-FAM and BHQ1, a combination of HEX and BHQ1, and the like, but there is no limitation thereto. In a preferable aspect, a fluorescent dye is linked to the 5' or 3' terminus of each probe, and a quencher is linked to the terminus on the opposite side.

Examples of the digital PCR method used in the present invention include a droplet digital PCR (ddPCR) method, a chip-based digital PCR (cdPCR) method, and the like. The digital PCR is conducted in accordance with, for example, the following procedure. A reaction solution containing a probe set, a DNA sample, a PCR primer set, and DNA polymerase is set in a digital PCR apparatus. The mixing ratio of each component of the reaction solution can be selected as appropriate from a known range and optimized, and can be changed as appropriate depending on the primer set, the probe set, or the like used.

In the case of ddPCR, minute droplets (nanoliter-sized, for example) of the reaction solution are formed using a water-oil emulsion. In the case of cdPCR, the reaction solution is distributed, using a digital PCR apparatus, to a large number of (several thousands to tens of thousands) minute reaction wells (with an opening of several ten µm) provided in a chip. The amount of the DNA sample in the thus obtained minute fractions of the reaction mix is adjusted so as to create a condition where the region to be measured in the DNA is contained (preferably about 1 copy) and a condition where such a region is not contained, and then PCR is conducted. At this time, PCR amplification reaction occurs in the reaction droplet or well in which the DNA region to be measured is present, and the probe bound to the target site is cleaved due to the nuclease activity of the DNA polymerase. Thus, the fluorescent dye separates from the quencher and emits fluorescence. Measuring the number of droplets or wells in which fluorescence is detected using the reader of the digital PCR apparatus makes it possible to quantify the DNA copy numbers from the total number of the droplets or wells through statistical calculation. Also, using two or more types of probe fluorescent dyes (e.g., 6-FAM and HEX) simultaneously makes it possible to compare the copy number at two or more DNA regions (e.g., compare a region that is expected to undergo DNA deletion and a region that is expected not to undergo DNA deletion) using the same sample.

In this way, it is possible to calculate the DNA deletion rate depending on the distance from the crRNA or guide RNA. Specifically, for example, when a Cas3 deletion induction pattern in a gene X is analyzed, digital PCR probes and primers are designed at positions about 1 kb, 10 kb, and 20 kb away from the crRNA target sequence in the forward direction and positions 1 kb (-1 kb), and 3.5 kb (-3.5 kb) away therefrom in the direction opposite to the forward direction, and digital PCR probes and primers are designed in a region (e.g., different gene Y) considered not to be in a range of genome editing by Cas3 as a comparison target. When the gene X is a gene on an autosome, a cell retains 2 copies. Therefore, unless genome editing is not conducted, the measurement result is approximately 2 copies at any positions. However, when Cas3 induces a deletion mutation, the copy number of the comparison target (gene Y) is still approximately 2 copies, but the copy number of the gene X decreases depending on the position (e.g., when the copy number at a position 1 kb away from the crRNA is 1.5 copies, this shows that the copy number decreases by 25%, that is, deletion over a length of 1 kb or more occurs at a rate of 25%). A position in the gene X at which the copy number decreases, and an extent to which the copy number decreases depend on the cleavage pattern of the crRNA target sequence by Cas3, and therefore, a deletion mutation induction pattern by Cas3 can be measured.

Specific examples of the primers that can be used for digital PCR include primers that include a sequence represented by any of SEQ ID NOS: 103, 104, 106, 107, 109, 110, 112, 113, 115, 116, 118, 119, 121, 122, 124, and 125 for the human B2M gene, primers that include a sequence represented by any of SEQ ID NOS: 127, 128, 130, and 131 for the human HLA gene, primers that include a sequence represented by any of SEQ ID NOS: 132, 133, 135, 136, 138, 139, 141, 142, 144, 145, 147, 148, 150, 151, 153, 154, 156, 157, 159, 160, 162, 163, 165, and 166 for the human DMD gene, and the like. These primers can be used as a combination of a forward primer and a reverse primer as appropriate.

Specific examples of the probes that can be used for digital PCR include probes that include a sequence represented by any of SEQ ID NOS: 102, 105, 108, 111, 114, 117, 120, and 123 for the human B2M gene, probes that include a sequence represented by any of SEQ ID NOS: 126 and 129 for the human HLA gene, probes that include a sequence represented by any of SEQ ID NOS: 134, 137, 140, 143, 146, 149, 152, 155, 158, 161, 164, and 167 for the human DMD gene, and the like. Two or more types of these probes may be used in combination.

Also, another aspect of the present invention provides primers that include a sequence represented by any of SEQ ID NOS: 103, 104, 106, 107, 109, 110, 112, 113, 115, 116, 118, 119, 121, 122, 124, 125, 127, 128, 130, 131, 132, 133, 135, 136, 138, 139, 141, 142, 144, 145, 147, 148, 150, 151, 153, 154, 156, 157, 159, 160, 162, 163, 165, and 166, and/or probes that include a sequence represented by any of SEQ ID NOS: 102, 105, 108, 111, 114, 117, 120, 123, 126, 129, 134, 137, 140, 143, 146, 149, 152, 155, 158, 161, 164, and 167. These primers and probes may be provided in the form of a kit or an agent (e.g., a therapeutic agent and a reagent).

The present invention will be described more specifically with reference to the following examples. It is to be noted, however, that the present invention is not limited to these examples by any means.

### Examples

### <Materials and Methods>

### Ethical approval

The establishment and use of patient-derived iPSCs were approved by the Ethics Committee of the Graduate School of Medicine, Kyoto University, and Kyoto University Hospital (approval numbers R0091 and G259). All patient information was kept anonymous, and written informed consent was obtained.

### Production of Chemically Modified Pre-crRNA

Modified nucleic acids (RNAs) were produced by Integrated DNA Technologies Inc. (IDT) to which synthesis thereof was requested.

### Production of Pre-crRNA to Which 5' Cap and poly-A are added

A pre-crRNA sequence that includes a 5'-Cap analog (ARCA) and a poly-A tale was synthesized through in vitro transcription (IVT) using Invitrogen mMESSAGE mMACHINE T7 Ultra Transcription Kit. The IVT template was prepared through PCR using a plasmid (pPV-C1-crRNA-EF1a-G2ABA) that includes a pre-crRNA sequence (B2M#1 or B2M#4) as a template with a PCR forward primer
(GAAATTAATACGACTCACTATAGGATGTGTTGTTTGTGTGATACTA; SEQ ID NO: 2) that includes the T7 promoter sequence and a reverse primer (CCCGGGCtgcaggaattc; SEQ ID NO: 3). The 5'-Cap analog was incorporated during transcription by T7 polymerase due to the Cap analog being mixed with GTP at a ratio of 4:1, and the poly-A tale was added through reaction by E. coli Poly(A) Polymerase (E-PAP) after the transcription. The final pre-crRNA sequence included a leader sequence at 5'.

Sequences (target sequences) targeted by the pre-crRNAs used in this example are as follows.

**[Table 1-1]**

| Name | Sequence (5'→3') | SEQ ID NO. |
|---|---|---|
| B2M#1 | GCCACGGAGCGAGACATCTCGGCCCGAATGCT | 4 |
| B2M#4 | AGAGAGTAGCGCGAGCACAGCTAAGGCCACGG | 5 |
| A24-be-ex1-cr1 | GCTGGGACTCCCCAATCCATACACCGCCTTCG | 6 |
| A24-ex1-cr1 | CCAATCAGTGTCGTCGCGGTCGCTGTTCTAAA | 7 |
| A24-ex2-cr1 | TGAAGGCCCACTCACAGACTGACCGAGAGAAC | 8 |
| A24-ex2-cr2 | GCCCACTCACAGACTGACCGAGAGAACCTGCG | 9 |
| A24-ex3-cr1 | GATTACATCGCCCTGAAAGAGGACCTGCGCTC | 10 |
| A24-ex3-cr2 | TGGGAGGCGGCCCATGTGGCGGAGCAGCAGAG | 11 |
| A24-ex3-cr3 | GAGACGCTGCAGCGCACGGGTACCAGGGGCCA | 12 |
| A24-ex3-cr4 | CGCCCGTCCGACCCCACGTCGCAGCCAAACAT | 13 |
| A24-ex3-cr5 | AGGACCTGCGCTCTTGGACCGCGGCGGACATG | 14 |
| HLA-A24:02 F1 | AAACTGCGGAGTTGGGGAATCCCCAAGGCTGG | 15 |
| HLA-A24:02 R2 | ATGGCCACATGCATGCTGGTGGAGTGTCCCAT | 16 |
| HLA-A24:02 R3 | ATTATCCCAGGTGCCTGTGTCCAGGCTGGTGT | 17 |
| HLA-A24:02 R4 | TCGCTGTTCCCTCCTCAGGGAATAGAAGATTA | 18 |
| HLA-B52:01 F1 | GCTCTCGGAGCCTGAGACCCTGAGAGCCCCGC | 19 |
| HLA-B52:01 R5 | AGGAGGAAAATGGGATCAGCGCTAGAATGTCG | 20 |
| HLA-B52:01 R7 | AGCAGAGATACACATGCCATGTACAGCATGAG | 21 |
| HLA-A24:02 F3 | GACCCTACATAGGTTGGGAGAGGGAGAAAAGA | 22 |
| HLA-A24:02 F4 | AAGGACCCTACATAGGTTGGGAGAGGGAGAAA | 23 |
| HLA-A24:02 R0 | GGGTGAAGGGTGGGGTCTGAGATTTCTTGTCT | 24 |
| HLA-A24:02 R3 | ATTATCCCAGGTGCCTGTGTCCAGGCTGGTGT | 25 |
| HLA-A24:02 R9 | ACGATCCCTCGAATACTGATGACTGGTTCCCT | 26 |

**[Table 1-2]**

| Name | Sequence (5'→3') | SEQ ID NO. |
|---|---|---|
| HLA-A24:02 R10 | GGATAAAATCTCTGACGGAATGACGGAAAGAC | 27 |
| HLA-A24:02 R11 | GAGGGGAGACAATTGGGACCAACACTAGAATA | 28 |
| HLA-B52:01 F2 | TGAAACTCGTGGGAGTGGGGAATCCCCAACGC | 29 |
| HLA-B52:01 F3 | AAGTGAAACTCGTGGGAGTGGGGAATCCCCAA | 30 |
| HLA-B52:01 F4 | GACCCGACATAGGTTGGGAGAAGAAGTGAAAC | 31 |
| HLA-B52:01 F7 | AAGGACCCGACATAGGTTGGGAGAAGAAGTGA | 32 |
| HLA-B52:01 R4 | ATTACATCGCCCTGAACGAGGACCTGAGCTCC | 33 |
| HLA-B52:01 R6 | CGCCTGAATTTTCTGACTCTTCCCATCAGACC | 34 |
| HLA-B52:01 R8 | GAGGGGGATGAGGGGTCATATCTCTTCTCAGG | 35 |
| HLA-B52:01 R9 | GGATGACGTCTCTGAGGAAATGGAGGGGAAGA | 36 |
| HLA-B52:01 R10 | TCCCTGTTCCCCGCTCAGAGACTCGAACTTTC | 37 |
| HLA-B52:01 R11 | AGCTCAGGTAGGGAAGGGGTGAGGGGTGGGGT | 38 |
| DMPK DownSNP1C (FIG. 34 Top) (FIG. 50: 32nt) | gccgcttccgaaCcgcccacgggcctccgctt | 39 |
| DMPK DownSNP1T | gccgcttccgaaTcgcccacgggcctccgctt | 40 |
| DMPK DownSNP1 12nt (FIG. 34 Bottom) | ccgcttccgaaccgcccacgggcctccgcttc | 41 |
| DMPK DownSNP1C 30nt (FIG. 50: 30nt) | gccgcttccgaaCcgcccacgggcctccgc | 42 |

**[Table 1-3]**

| Name | Sequence (5'→3') | SEQ ID NO. |
|---|---|---|
| DMPK DownSNP1C 28nt (FIG. 50: 28nt) | gccgcttccgaaCcgcccacgggcctcc | 43 |
| DMPK DownSNP1C 26nt (FIG. 50: 26nt) | gccgcttccgaaCcgcccacgggcct | 44 |
| DMPK DownSNP1C 1MM (FIG. 51: 1MM) | gccgcttccgaaCcgcccacgggcctccgctG | 45 |
| DMPK DownSNP1C 2MM (FIG. 51: 2MM) | gccgcttccgaaCcgcccacgggcctccgcGt | 46 |
| DMPK DownSNP1C 4MM (FIG. 51: 4MM) | gccgcttccgaaCcgcccacgggcctccActt | 47 |
| DMPK UpSNP1T | cgggtggcaaggggcgggtggagcgcggggcg | 48 |
| DMPK UpSNP1T-2 | cgcagctaagcgggtggcaaggggcgggtgga | 49 |
| DMPK UpSNP1T-3 | tgcgcagctaagcgggtggcaaggggcgggtg | 50 |
| DMPK UpSNP3T | ggatgccaagggttgccaccggcccgcatccc | 51 |
| DMPK UpSNP3T-2 | gggatgccaagggttgccaccggcccgcatcc | 52 |
| DMPK AGG DownSNP2C | tcctcggacatctcccgggaccagctcacaat | 53 |
| AAVS1#1 | gtggtcccagctcggggacacaggatccctgg | 54 |

**[Table 1-4]**

| Name | Sequence (5'→3') | SEQ ID NO. |
|---|---|---|
| DMD#20 (Described in filed specification) | ctgacagtagaccccagtacatgcttcctaaa | 55 |
| DMD#23 (Described in filed specification) | tcttggtgagaatcatattctgtagtacaagg | 56 |
| DMDexon46_1 | actccatctcaaatacatacatacatacgtac | 57 |
| DMDexon47 1 | gaaactgagacaaaggggttaaataccagctc | 58 |
| DMDexon48_1 | attgtaaaaggaagtcccttcatgtacaagga | 59 |
| DMDexon49_1 | cttattatttattaatgagtcaaacaggcctt | 60 |
| DMDexon50_1 | ttcatgcctttccccaggcagccctcattcag | 61 |
| DMD#25 (Targeting Ex51) | ttggcatttatgcaatgccatgttcaaatgaa | 62 |
| DMDexon52_1 | acccatctgactagacgctgtgcatattcttt | 63 |
| DMDexon53 1 | atcacttcaaaattggtatacgtatttatgta | 64 |
| DMDexon54 1 | ccttgtatccttattatacctctcttgatctc | 65 |
| DMDex45_1kb (FIG. 16) | cctgaatctgcggtggcaggaggtctgcaaac | 66 |
| DMDex45_40kb (FIG. 16) | ctcaagcagacaaatctccagtggataaaggt | 67 |
| DMDex45_95kb (FIG. 16) | acaaaatattacttgttaaagtgtggtaagga | 68 |

### Transduction of CRISPR-Cas3 and CRISPR-Cas9

For transduction to iPSC, 1.5 to 3×10⁵ cells were seeded on wells of a 12-well plate coated with iMatrix-511 or iMatrix-511 silk. On the following day, 1000 to 2000 ng of a plasmid DNA or RNA in total was transfected to the cells using 2 to 4 µL of Lipofectamine Stem (Thermo Fisher Scientific Inc.). For transduction to HEK293T cells, 2×10⁵ cells were seeded on wells of a 12-well plate or 1×10⁶ cells were seeded on wells of a 6-well plate. On the following day, 1000 to 2000 ng of a plasmid DNA or RNA in total was transfected using 4 to 8 µL of Lipofectamine 2000 (Thermo Fisher Scientific Inc.). As the plasmid for expression of CRISPR-Cas3 and the Cascade protein group, pPV-Dual_promoter-EF1α-2xNLS-Cascade+Cas3(RD)-iPA (expressing the puromycin-resistant gene), pPV-Dual_promoter-EF1α-2xNLS-Cascade+Cas3(RD)-iZA (expressing the zeocin-resistant gene), pPV-Dual_promoter-EF1α-2xNLS-Cascade+Cas3(RD)-iCA (expressing the mCherry), and pPV-TRE3G-Bi(263+751)-EF1a-Tet3G-iPA (DOX-inducing type, expressing the puromycin-resistant gene) were used. As the crRNA expression plasmid, pBSIIKS-U6-crRNA, which expresses a pre-crRNA using the U6 promoter, or the pPV-C1-crRNA vector (including those simultaneously expressing EGFP and a drug-resistant gene using EF1α) was used. When SSA reporter sorting was applied, pPVEF1a-EGxxFP-iPA, pPV-EF1a-EGxxFP-iBA, pPV-EF1a-mRxxFP-iPA, pHL-EF1a-EGxxFP-iPA, pHL-EF1a-mRxxFP-iPA, pHL-EF1a-Puro-SSA-A, or pHL-EF1a-EGFP-IRES-Puro-SSA-A with an appropriate target sequence was used. When drug selection (puromycin, blasticidin, zeocin) was conducted, culture was conducted in a culture medium containing the drug at a desired concentration one to three days after the transfection. The cells were collected for extraction of the genome DNA or proteins or FACS (fluorescence-activated cell sorting) analysis. When an RNA was introduced through electroporation, the electroporation was conducted on 3×10⁵ cells using P4 Primary Cell 4D-Nucleofector X Kit S of 4D-Nucleofector (LONZA) and electroporation program CA-189.

When the transfection was conducted a plurality of times, the transfection was conducted in the way described above, the cells were cultured for several days in depending on the cytotoxicity at that time, and the operations from the cell seeding to the transfection were repeated. The transfection interval was adjusted to approximately three days to fifteen days in consideration of the cell growth rate, the transfection conditions, and the susceptibility to transfection. In some cases, the cells were cryopreserved once in the middle of a plurality of the transfection processes, and the transfection was resumed after thawing.

### Determination of Presence or Absence of Genome Editing through PCR, and Measurement of Editing Efficiency through HLA Staining

It was determined through PCR whether or not Cas3 induced a deletion mutation. Norma PCR was conducted for the HLA-A*24:02 gene (FIG. 19), and allele-specific PCR was conducted for the DMPK gene using an allele-specific primer designed at a hetero SNP (FIGS. 35 and 36). Nested PCR was also used to enhance the detection sensitivity (FIGS. 38 to 47). La Taq HS (TaKaRA) and KOD One (TOYOBO) were used as PCR enzymes. PCR primers used are listed in Table 2 below.

When the human B2M gene was used as the genome editing target, the gene knockout efficiency was calculated through cell-surface HLA-A/B/C staining. After the genome editing, the cells were cultured for a while (preferably for three to four days or more until the HLA molecules disappeared from the cell surface), and then the cells were isolated and collected using trypsin (including a trypsin alternative such as TrypLE Select). The collected cells were washed with PBS and stained with anti-HLA-A/B/C antibody (iPS cells: BioLegend Pacific Blue anti-human HLA-A,B,C Antibody, HEK293T cells: BD BV421 Mouse anti-human HLA-A2) diluted 50 to 100 times in 2% FBS/PBS (on ice for 30 minutes). The HLA-stained cells were analyzed through flow cytometry to calculate HLA-staining-negative cells %.

**[Table 2]**

| Name | Sequence | SEQ ID NO. |
|---|---|---|
| HLA-A24_4kb_F5 (For HLA-A24 gene) | GAGTGTGGCTTGGACTTAATGAGG | 69 |
| HLA-A24 Rev2 | AGCTCAGTGCACCATGAAGTTG | 70 |
| DM1_HPS1051_5kb_fwd1 (For HPS1051 cells) | ccctggctcacacgtgtactgacac | 71 |
| HPS1051_SNP_C_rev_1mm | aagcggaggcccgtggAcgG | 72 |
| HPS1051_SNP_T_rev_1mm | aagcggaggcccgtggAcgA | 73 |
| UpSNP4G 1mm F (Nested 1^{st} PCR for CiRA00213 iPS cells) | ggccagaagttctggttctccaccCgag | 74 |
| DownSNP2C_R | cgggcgectgagattgGgag | 75 |
| UpSNP3_T_1mm_dir2 | cgggccggtggcaacccttgacaT | 76 |
| DMPK-CTG-3'-Rev | ACCAGTTGCTGACCTGCGTG | 77 |
| DownSNP2C R2 | cgcgggcgcctgagattgGgag | 78 |
| DownSNP2C R3 | cgcgggcgcctgagattgtAag | 79 |

### TapeStation Analysis

The DNA samples were analyzed using High Sensitivity D5000 ScreenTape/reagent kit in Agilent 2200 TapeStation or Agilent 4200 TapeStation. The ratio of DNAs with a deletion mutation was calculated as the ratio of DNAs having a size of 400 to 3700 bp using the function of TapeStation software.

### Method for Constructing SSA Reporter Vector

The reporter genes EGFP, mRFP, and Puro^{R} were divided into two fractions, a sequence was added such that the genes had a homologous sequence of about 150 to 350 bp, and two restriction enzyme sites (AfeI or Esp3I site) for insertion of a target sequence were further added. These divided reporter genes were inserted into a vector with pHL-EF1a or pPV-EF1a as a backbone. A crRNA target sequence with a desired length was inserted between the fractions of the divided reporter gene through cleavage by a restriction enzyme and the In-Fusion reaction.

### Cell Sorting via SSA Reporter Vector

When cell sorting was conducted using an SSA reporter vector, the SSA reporter vector was transfected to the cells in addition to the vector for expression of Cas3 and the Cascade protein group and the vector for expression of the crRNA. When a Puro-SSA vector was used as the reporter gene for the SSA vector, cell sorting was conducted by culturing the cells in a puromycin-containing culture medium. When fluorescent proteins, EGFP and mRFP, were used as the reporter genes, the cells were collected using trypsin (or a trypsin alternative) after the transfection, and cells positive for EGFP and mRFP were separated using a BD FACSAria II (BD) cell sorter.

### Single Cell Cloning

The limiting dilution method or the colony picking method was used to separate subclones of genome-edited iPS cells (FIG. 36). When the SSA vector with fluorescent proteins, EGFP and mRFP, were used, single cell sorting in wells of a 96-well plate pre-coated with iMatrix-511 silk was conducted during cell sorting of cells positive for both EGFP and mRFP. Expansion culture of the isolated cells was conducted, and the cells were collected for cryopreservation and genome DNA extraction. For genotyping, the target regions were amplified through PCR using primers in Table 2 or Table 3 below. Sanger sequencing was conducted to analyze deletion patterns.

**[Table 3]**

| Name | Sequence (5'→3') | SEQ ID NO. |
|---|---|---|
| crRNA_DMDexon45check_rev | acatctgatgtgtgcccatgc | 80 |
| crRNA_DMDexon55check_dir | cgcaaatgttgaggttttcagaggc | 81 |
| crRNA_DMDexon55check_rev | ggatttggctgaaaatcacattgtctcg | 82 |
| crRNA_DMDexon51check_dir | gaggctataaaagccaagaactgacaatg | 83 |
| crRNA_DMDexon51check_rev | catttgccagtcagcctggtgttctgc | 84 |
| crRNA_DMDexon45check_rev2 | ctatggtatacatctgatgtgtgcccatgc | 85 |
| crRNA_DMDexon45check_rev3 | cttctggcctaccatttgttgatcacc | 86 |
| DMDexon45-55check_dir1 | cctaccatagctgatgggtaaaatgtaaac | 87 |
| DMDexon45-55check_dir2 | gaagcttgcagtttcttgagattagaaatagag | 88 |
| DMDexon45-55check_rev1 | gactggtgtctctattactaagcaatgactgte | 89 |
| DMDexon45-55check_rev2 | gcaaatgcttactgaaaccttccatgcatc | 90 |
| DMDexon45(45-55)check_rev3 | gactccatggtgatgatgagcc | 91 |
| DMDexon45(45-55)check_rev4 | ctcctccgctcatccttttcactg | 92 |
| DMDexon45(45-55)check_rev5 | caccacaggctttaacttctgccg | 93 |
| DMDexon55(45-55)check_dir3 | ggttaagccactggacaaaaacg | 94 |
| DMDexon55(45-55)check_dir4 | gagtagcctgatcgacactggac | 95 |
| DMDexon55(45-55)check_dir6 | CCTGAAGTACAAGGACGACGG | 96 |
| DMDexon55(45-55)check_dir7 | CTTCAGAATTAATCCGTGCGCC | 97 |
| DMDexon55(45-55)check_dir8 | cacacacaAACCACCGaaccaaag | 98 |
| DMDexon45(45-55)check_rev6 | gtagacttagaatggaattctgggc | 99 |
| DMDexon45(45-55)check_rev7 | gaatgggtcctggtgctgtgcttagc | 100 |
| DMDexon45(45-55)check_rev8 | ccgcggtatctgttgttcgc | 101 |

### Quantification of Deletion in Genome through ddPCR Assay

In order to measure the copy number of the genome DNA, ddPCR probes and primer pairs were designed using Primer3Plus (https://www.bioinformatics.nl/cgi-bin/primer3plus/primer3plus.cgi) software. In order to detect target genome regions (e.g., the B2M gene and the gene locus of the dystrophin exon 44-55), DNA probes labeled with 5'-6-FAM fluorophore and 3'-BHQ1 were used. Primers labeled with 5'-HEX fluorophore and 3'-BHQ1 were used as reference probes. All primer pairs and probes are listed in Table 4 below.

**[Table 4-1]**

| Name | Sequence (5'→3') | SEQ ID NO. |
|---|---|---|
| ddPCR_B2M_behind_3.5kb_probe | CCCAACCCCAGGCAGGGCAGC | 102 |
| ddPCR_B2M_behind_3.5kb_fwd | AGCTGAGCTGGTATCCAATG | 103 |
| ddPCR_B2M_behind_3.5kb_rev | ATTCCTGGGTAAGTCCTGGT | 104 |
| ddPCR_B2M_behind_1kb_probe | CCACCACCACGAAATGGCGGCACC | 105 |
| ddPCR_B2M_behind_1kb_fwd | GACTTCCAAGATCTCTGCCC | 106 |
| ddPCR_B2M_behind_1kb_rev | TAAACATGACAGGCAGACCC | 107 |
| ddPCR_B2M_1kb_probe | CCACAGGGCCCATGCCGCCC | 108 |
| ddPCR_B2M_1kb_fwd1 | ACACAGGGGATAAATGGCAG | 109 |
| ddPCR_B2M_1kb_rev1 | GAAACAACCAGGCAAAGAGC | 110 |
| ddPCR_B2M_10kb_probe | GGCGTGAGCCACCGTGCCTGC | 111 |
| ddPCR_B2M_10kb_fwd1 | TTAACTCTTCACCTGGCTGC | 112 |
| ddPCR_B2M_10kb_rev1 | CTGCTCACCTTGACTTTCCA | 113 |
| ddPCR_B2M_10kb_probe2 | TGTGCCCTGAGTGCTTCTCCCTGGC | 114 |
| ddPCR_B2M_10kb_fwd2 | AGCAGGTCATTTCAGTAGCC | 115 |
| ddPCR_B2M_10kb_rev2 | ACTAAGGCATGCTCCAAAGG | 116 |
| ddPCR_B2M_35kb_probe | ACCCCATCCTGTTGGCCACAACCCA | 117 |
| ddPCR_B2M_35kb_fwd | TCTATGAGGAAGGACTGCCA | 118 |
| ddPCR_B2M_35kb_rev | TTGTTCTGTACCAGGGCTTG | 119 |
| ddPCR_B2M_50kb_probe | GTGAGACTCTGTCCCACCCTCCCACCA | 120 |
| ddPCR_B2M_50kb_fwd2 | TGGGGTAGTCCATGGTAGTT | 121 |
| ddPCR_B2M_50kb_rev2 | GAGTTGAGATGCACCACTGT | 122 |

**[Table 4-2]**

| Name | Sequence (5'→3') | SEQ ID NO. |
|---|---|---|
| ddPCR B2M 71kb probe | TCCCCAGGGAAGCCTTCTCATCCAGCA | 123 |
| ddPCR B2M 71kb fwd | TGCTCCACCTGCTACTAAGA | 124 |
| ddPCR B2M 71kb rev | GAGGACAGGTGTGATTACGG | 125 |
| A24_ddPCR_probe | CCTCTGGCTCGCGGCGTCGC | 126 |
| A24 ddPCR F | CTCCCACTCCATGAGGTATT | 127 |
| A24 ddPCR R3 | CCTTCACTTTCCCTGTCTCC | 128 |
| B52 ddPCR probe | CCTCGGACTCGCGGCGTCGC | 129 |
| B52 ddPCR F | ATTTCTACACCGCCATGTCC | 130 |
| B52 ddPCR R2 | GCAGGTTCTCTCGGTAAGTC | 131 |
| DMDexon7 ddPCR F | tgtgtttTAGGCCAGACC | 132 |
| DMDexon7_ddPCR_R | AGCAGTGGTAGTCCAGAA | 133 |
| DMDexon7_probe | AGTCGTTGTGTGGCTGACTGCTGGCA | 134 |
| DMDex45 ddPCR F | ggaactccaggatggcattgg | 135 |
| DMDex45 ddPCR R | gacagctgtttgcagacct | 136 |
| DMDexon45_probe | tgggaagcctgaatctgcggtggca | 137 |
| DMDin50 ddPCR F | ccccaggaagggcattaag | 138 |
| DMDin50_ddPCR_R | agttcagatgttgtcccc | 139 |
| DMDin50 probe | cccccatgaccccacacttcccgt | 140 |
| DMDin47 ddPCR F | ctgacttccctcttggtgac | 141 |
| DMDin47 ddPCR R | caggaatgagcatgtgacct | 142 |
| DMDintron47_probe | tggcctgaagccgtttcagaacccaca | 143 |
| DMDin48 ddPCR F | tgcagctttactccacac | 144 |
| DMDin48 ddPCR R | ggcagttcaagatttgctgg | 145 |

**[Table 4-3]**

| Name | Sequence (5'→3') | SEQ ID NO. |
|---|---|---|
| DMDintron48_probe | cccaggaccctagcggaaagagcagc | 146 |
| DMDin51_ddPCR_F | ctgttgctggatactttcgc | 147 |
| DMDin51_ddPCR_R | cgactcactctcctctcat | 148 |
| DMDintron51_probe | cccaggaccctagcggaaagagcagc | 149 |
| DMDex53_ddPCR_F | caatggctggaagctaagga | 150 |
| DMDex53_ddPCR_R | tgtatagggaccctccttcc | 151 |
| DMDexon53_probe | agcaggtcttaggacaggccagagcca | 152 |
| DMDex55_ddPCR_F | ggctgctttggaagaaactc | 153 |
| DMDex55_ddPCR_R | cagctcttttactcccttgg | 154 |
| DMDexon55_probe | actgcaacagttccccctggacctgga | 155 |
| ddPCR_ex45_16kb_fwd | cccagcttggtcaagcataa | 156 |
| ddPCR_ex45_16kb_rev | agactgcaactttaggccag | 157 |
| ddPCR_ex45_16kb_probe | tcagtaacggtggagacctaggcggca | 158 |
| ddPCR_ex45_38kb_fwd | tcaccacttcagcctctact | 159 |
| ddPCR_ex45_38kb_rev | aggacggtatgaaaagccac | 160 |
| ddPCR_ex45_38kb_probe | accaacaccacgtcccacaccccc | 161 |
| ddPCR_ex45_90kb_fwd | tggtaggaagatgagctggt | 162 |
| ddPCR_ex45_90kb_rev | agtatgggggatcctcttcc | 163 |
| ddPCR_ex45_90kb_probe | ccccacatgtcaagggtggagccagg | 164 |
| ddPCR_ex45_Neg1kb_fwd | acgttgcctcaagttctctg | 165 |
| ddPCR_ex45_Neg1kb_rev | agtgaaaaggatgagcggag | 166 |
| ddPCR_ex45_Neg1kb_probe | cccaggccccaatatacaccgagggc | 167 |

Isolated genomic DNA was first digested by the EcoRI restriction enzyme (TaKaRa) at 37°C overnight. Next, ddPCR reaction mixes were prepared by mixing 11 µL of ddPCR Supermix for Probes (no dUTP) (Bio-Rad Laboratories), 1 µL of 20 µM target forward and reverse primer pair each, and 0.6 µL of 10 µM target probe, 1 µL of 20 µM reference forward and reverse primer pair each and 0.6 µL of 10 µM reference probe, EcoRI-digested genomic DNA and the total volume was adjusted to 22 µL by ultrapure water. Droplets were generated using QX200 Droplet Generator or QX200 Automated Droplet Generator (Bio-Rad Laboratories), and PCR reactions were performed in a C1000 Thermal Cycler (Bio-Rad laboratories). ddPCR reactions were run under the following 3-step thermal conditions: step 1, 95°C for 10 min, step 2, 94°C for 30 sec, step 3, 55 to 60°C for 1 to 2 min, repeat steps 2 and 3 for 40 times, step 4, 98°C for 10 min, step 5, store at 4°C. After the PCR reaction, the fluorescence of the droplets was measured using a QX200 Droplet reader (Bio-Rad Laboratories) and analyzed by the Bio-Rad QuantaSoft Software.

### Statistical Analysis

To compare between two groups, two-tailed, unpaired Student's t-tests were performed using Prism 8 software.

### Example 1: Chemical Modification of crRNA in Cas3 Genome Editing Using RNA

Experimental cancer cell strains that are often used in the fields of biology and medicine include many strains having high gene transfer efficiency, such as HEK293T cells, and such strains are often used as a model strain for genome editing. However, iPS cells, primary cultured cell strains, and the like intended to be practically applied to regenerative medicine and the like do not necessarily have high gene transfer efficiency. Furthermore, only a small number of cell strains selected for clinical use have been established. Therefore, the most suitable strain for gene transfer cannot always be selected. For this reason, it is important to develop a method capable of achieving stable and efficient genome editing even in a cell strain having low gene transfer efficiency. Therefore, the inventors of the present invention applied a chemical modification to a crRNA and verified the genome editing efficiency depending on the modification. First, mRNAs for expression of six types of proteins in total (Cas3, Cse1, Cse2, Cas5, Cas6, Cas7) were gathered into two mRNAs, Cse2-P2A-Cas6-T2A-Cas3 and Cas7-P2A-Cas5-T2A-Cse1, using a 2A peptide capable of allowing one mRNA to express a plurality of proteins. In addition to the 5' cap and the poly-A tale, base modifications, 5-methyl-cytosine and pseudo-uracil (also used in Examples in Patent Literature 3: TriLink), were applied to these mRNAs in order to suppress cytotoxicity caused by a natural immune reaction and enhance the translation efficiency. Next, in order to analyze the protein expression level and the protein expression time in iPS cells, gene transfer of these mRNAs to the I01s04 iPS cell strain and the I14s14 iPS cell strain was conducted using Lipofectamine Stem Reagent. For comparison, gene transfer of a pPV-Dual_Promoter-EF1a-2xNLS-Cascade+Cas3(RD)-iPA plasmid vector (also used in the previous patent) capable of expressing all of the six types of proteins was conducted using Lipofectamine Stem Reagent in the same manner. The proteins were analyzed through Western blotting using an anti-2A peptide antibody (Cse2, Cas5, Cas6 and Cas7 are detected). As a result, it was found that the expression levels were higher compared with the case where the plasmid vector was used (FIG. 2). However, it was found that regarding the expression time, the expression peaked 8 hr (hours) to 24 hr after the gene transfer, and converged after 48 hr. It was revealed from these results that using the mRNAs in iPS cells makes it possible to enhance the expression of Cas3 and the Cascade protein group although for a short period of time.

Next, regarding the crRNA, an unmodified crRNA that had been previously synthesized through in-vitro transcription reaction was introduced to cells, and the activity was evaluated (Patent Literature 3). However, despite the high expression of Cas3 and the Cascade protein group caused by the mRNAs, the genome editing efficiency was at a level of a small percent, which suggests that the introduced crRNA did not function well. Therefore, the inventors thought that degradation of the RNA by RNase can be suppressed by applying a chemical modification to a specific region of the pre-crRNA, and the genome editing efficiency can be consequently enhanced. However, modifying an RNA may change the physical properties, the secondary structure, and the interaction with a protein of the RNA and thus lead to loss of original functions, and the fact is that even specialists cannot predict which type of modification should be selected and where the modification should be introduced. Accordingly, the inventors of the present invention introduced a 2'-O-methyl 3'phosphorothioate modification (MS modification) to the pre-crRNA for the E. coli CRISPR-Cas3 system to search for a modification position for enhancing the activity. As a result, it was found that introducing the MS modification to three bases at both termini of the pre-crRNA significantly enhanced the genome editing efficiency without loss of the functions (FIGS. 3-1 to 4-2). It was confirmed that this was independent of the types of iPS cell strains (I01s04 and I14s04) and gene transfer methods (lipofection (Lipofectamine Stem Reagent) and electroporation (4D-Nucleofector)). Surprisingly, when (2) and (3), which are the termini of a mature-crRNA produced after Cas6 cleaves the pre-crRNA, were modified, it was observed that the activity was lower than or equal to that of the unmodified crRNA (FIGS. 4-1 and 4-2). Therefore, not all the positions are suitable for the modification, and it can be said that modification positions for enhancing the activity are limited.

Next, changes in activity depending on the modification positions were examined in more detail. Pre-crRNAs with modifications at the positions shown in FIG. 5-1 to 5-4 were prepared and introduced to cells through lipofection in the same manner as described above. As a result, introducing the modification to regions at both termini (Both Edges) and the loop portions of both the stem loop-forming regions enhanced the genome editing efficiency, whereas introducing the modification to the central portion (i.e., the 3'-side stem-forming region (Left Right stem) of the first repeat sequence, the 3' arm region (Left Right arm) of the first repeat sequence, the 5' arm region (Right left arm) of the second repeat sequence, and the 5'-side stem-forming region (Right left stem)of the second repeat sequence) tended to reduce the genome editing efficiency or cause substantially no changes in the genome editing efficiency (FIG. 6-1). It was observed that introducing the modification to the Cas6 cleavage site significantly reduced the genome editing efficiency. Surprisingly, although the pre-crRNA has a nearly symmetric structure, introducing the modification to the 5'-side portion (specifically, the 5'-terminal region (Pre-5'), the 5' arm region (Left left arm) of the first repeat sequence, the 5'-side stem-forming region (Left left stem) of the first repeat sequence, and the loop-forming region (Left loop) of the first repeat sequence) and the 3' arm region (Right right arm) of the second repeat sequence tended to enhance the genome editing efficiency, whereas introducing the modification to the 3'-side portion (specifically, the 3'-terminal region (Pre-3') and the 3'-side stem-forming region (Right right stem) of the second repeat sequence) tended to have substantially no effect on the genome editing efficiency. That is to say, it was suggested that left-right asymmetry is observed in the pre-crRNA modification effects. When Cas6 cleaves the pre-crRNA, the 3' arm region of the first repeat of the pre-crRNA is made into the 5' terminus of the cleaved (mature) crRNA, but it was not observed that introducing the modification to the arm region enhanced the genome editing efficiency. That is to say, a difference in the influence of the introduction of the modification between the pre-crRNA and the mature crRNA was also observed.

The effects of the modification of the pre-crRNA were verified in another type of iPS cells (1383D2). The genome editing efficiency was evaluated through ddPCR using a pre-crRNA targeting B2M (FIG. 6-2) or DMD EX45 (FIG. 6-3). These results indicated that even when the type of iPS cells or a genome editing target gene was changed, the effects of the pre-crRNA modification pattern were reproduced. Also, it was shown that introducing the modification to a nucleotide in the spacer region reduced the genome editing efficiency ("Target" in FIG. 6-3).

Next, it was examined whether the activity of the pre-crRNA could be enhanced by a method other than introduction of a chemical modification to the RNA base and the backbone. Therefore, an attempt was made to add the 5'-cap analog (ARCA) and the poly-A tale to a pre-crRNA similarly to an mRNA (FIGS. 7 and 8). It was found from the results from HEK293T cells (FIG. 9) and I14s04 iPS cells (FIG. 10) that adding the 5'-Cap and the poly-A tale also makes it possible to enhance the activity of the pre-crRNA. Furthermore, in the case of a cell strain that is highly susceptible to a gene transfer operation, desired genome editing efficiency cannot be often obtained even when the genome editing is conducted only once. In this case, repeatedly conducting gene transfer also made it possible to accumulate the effects of the genome editing (FIG. 11). In a general genome editing experiment in which the CRISPR-Cas9 system is used, introduction is basically conducted once, which introduces a deletion or mutation to the target sequence. However, with the CRISPR-Cas3 system, a large deletion is introduced on the PAM sequence side of the target sequence, but the sequence targeted by the crRNA is conserved in many cases. Therefore, it is assumed that introducing the same crRNA to the same cell a plurality of times made it possible to enhance the cleavage efficiency.

Example 2: Analysis of Deletion Mutation Pattern through ddPCR

In order to make the genome editing a practical technique, it is necessary to precisely analyze a type of DNA mutation that can be induced using the technique, and the efficiency of inducing mutation (cleavage). In particular, it is known that, unlike Cas9, which induces minute cleavage of a DNA double strand, Cas3 induces various long-chain deletions different in length, but a method capable of easily analyzing the length of deletion mutation and the ratio thereof has not been developed yet. Also, the inventors of the present invention made an attempt to develop a technique for measuring the length distribution and the ratios of deletion mutations caused by Cas3 by applying the droplet digital PCR (ddPCR) technology. First, the inventors came up with a method in which a plurality of ddPCR probes are designed at various positions around a target sequence and the copy number at each position is directly measured in the genome DNA of a genome-edited cell (FIG. 12). This method enables analysis of the length and the ratio of a mutation introduced to the target genome sequence by the used crRNA, and is very useful for the design of a crRNA, examination of activity enhancement, and the like. Actually, genome editing of the B2M gene (using B2M#1 as the crRNA) was conducted using HEK293T cells in which the expression of Cas3 and the Cascade protein group can be controlled using doxycycline (DOX). As a result, it was possible to capture a situation where the editing efficiency changed depending on the DOX concentration and the duration of action (FIG. 13). This analysis revealed that a deletion mutation was partially introduced in a direction opposite to the forward direction of Cas3 (on the PAM side of the crRNA) at a certain rate. Furthermore, a similar experiment was also conducted using CiRA00213 iPS cells, and the deletion pattern was successfully analyzed (FIG. 14). Interestingly, when genome editing was conducted using the CRISPR-Cas3 system in the form of an RNA (upper diagram in FIG. 15), it was observed that the ratio of a long-chain deletion of 10 kb or more was lower compared with the case where Cas3 and the Cascade protein group were expressed through DOX induction for a plurality of days (lower diagram in FIG. 15). It is assumed that as shown in FIG. 2, when the protein is expressed using an mRNA, the expression time is short, and therefore, a difference from other methods capable of maintaining a long expression time was observed.

A pattern of genome editing by Cas3 is complicated, and it is thus difficult to expect what deletion pattern is supposed to occur when a plurality of crRNAs are used. With this measurement technique in which ddPCR is used, it was possible to analyze not only genome editing in which one crRNA is used, but also what deletion mutation pattern was shown when a plurality of crRNAs were used. For example, when two crRNAs were located on the dystrophin gene so as to face each other, it was also possible to analyze the degree of a decrease in the copy number at the intermediate position therebetween (FIG. 16). In more complicated cases, it was also possible to measure changes in the copy numbers at 7 positions when four to eleven crRNAs were located (FIG. 17). The technique is also useful for evaluation of the activity when a new crRNA is designed, and evaluation of the degree of a deletion of a desired region of a target gene. When HLA-A(A*24:02) and B(B*52:01) are knocked out in I14s04 iPS cells, it is ideal that the functionally important exons 1 to 3 (particularly the exon 2) are deleted (FIG. 18). To achieve this, various crRNAs can be designed. However, in the analysis after the genome editing, with conventional PCR for the target region, the presence or absence of the activity can be confirmed, but the activity level cannot be determined in many cases. In particular, when the designed crRNAs vary in the positions and the directions, the deletion mutations induced by Cas3 also vary in the positions and the length, the ladder band pattern indicating deletions changes after PCR, and thus the quantitativeness is lost (FIG. 19: Lipofectamine Stem was used, Cas3 and the Cascade protein group were expressed using an mRNA, and the crRNA was expressed using a plasmid). The inventors of the present invention designed ddPCR probes/primers on the exons 2 of the HLA-A gene and HLA-B gene, and analyzed the activity level by measuring the exon 2 deletion efficiencies of some crRNAs. An mRNA of Cas3 and the Cascade protein group and a pre-crRNA (Pre-both3 in FIG. 5-1) in which three bases at both termini have the MS modification were transfected to I14s04 iPS cells three times using Lipofectamine Stem. Then, first, it was confirmed that the genome editing by the control crRNA (B2M#1), which has already been shown to have the activity, was successful (FIG. 20, left). Next, the activity of three Pre-both3 pre-crRNAs (A24 F1, R2, R4, and B52 F1, R5, R7) for each of HLA-A and B was measured through ddPCR. It was revealed from the results that although the activity was observed in all the crRNAs, the crRNAs differed in the activity level (FIG. 20, right). This difference in the activity cannot be determined using conventional PCR (FIG. 19). Furthermore, in order to simultaneously knock out the HLA-A gene and the HLA-B gene, genome editing was conducted using simultaneously two crRNAs, one targeting HLA-A24 and the other targeting B52. In this case as well, it was possible to measure the exon 2 deletion efficiency for HLA-A and B (FIG. 21).

As described above, this measurement technique is useful for various evaluations such as analysis of a deletion pattern, evaluation of the activity of a designed crRNA, and measurement of the ratio of induction of a mutation in a direction opposite to the direction of interest, due to the positions of ddPCR probes being changed depending on the purpose.

### Example 3: Concentration of Genome-Edited Cell Population Using SSA Reporter Vector for CRISPR-Cas3 System

The type I CRISPR-Cas3 system has many requisite elements (seven elements in total, namely six proteins and an crRNA), and therefore, the genome editing does not occur if only one of the elements is expressed insufficiently, which is a problem from the viewpoint of achieving efficient genome editing when the type I CRISPR-Cas3 system is used as a genome editing tool. Therefore, the inventors of the present invention developed a technique for specifically concentrating a cell population in which Cas3 has been activated as a result of sufficient expression of these requisite elements. Regarding Cas9, it is reported that genome-edited cells are concentrated by using a reporter vector utilizing a single strand annealing (SSA) DNA repair mechanism and sorting cells in which Cas9 is activated. However, it is necessary to design a reporter vector suitable for Cas3 that causes long-chain DNA cleavage unlike Cas9 that simply cleaves a DNA double-strand.

Therefore, the inventors of the present invention designed an SSA reporter vector based on a fluorescent protein (e.g., EGFP or mRFP) and a drug-resistant gene (e.g., Puro^{R}) (FIG. 22), and searched for an insertion sequence suitable for the CRISPR-Cas3 system. First, Cas3 causes a long-chain deletion in one direction, and thus it was thought that when a very short sequence such as only a crRNA recognition sequence is inserted, Cas3 may introduce a deletion to the reporter gene as well. Therefore, the inventors evaluated the activity of an SSA vector for the CRISPR-Cas3 system in which a relatively long (0.5 kb, 1 kb, or 2 kb) insertion sequence had been incorporated. At this time, mRFP was used as the reporter gene. A HEK293T stable expression strain in which expression of Cas3 and the Cascade protein group can be induced using DOX was used, and a crRNA expression vector (non-target: AAVS1#1, target: DownSNP1T) and an mRFP SSA vector (having DownSNP1T as the target insertion sequence, the insertion sequence having a length of 0.5 to 2 kb: pPV-EF1a-mRxxFP-iPA) were transfected using Lipofectamine 2000. After three days, the ratio of mRFP-positive cells was analyzed through flow cytometry. As a result, when the target crRNA was used, it was possible to obtain high reporter signal, compared with the case of using the non-target crRNA (FIG. 23). The background signal tended to decrease as the insertion sequence was longer, but when the 2-kb insertion sequence was used, a decrease in the on-target signal was also observed simultaneously. As is clear from the value of the S/N ratio, it can be said that in this experiment, an insertion sequence of about 1 kb is suitable for Cas3.

Next, it was examined if this SSA vector developed for the CRISPR-Cas3 system could be used to concentrate actually genome-edited cells. An SSA vector (pHL-EF1a-EGFP-IRES-Puro-SSA(B2M 1kb)-A vector) having Puro^{R} as the reporter was used to design cells such that the cells have resistance against puromycin when Cas3 is activated. Genome editing targeting the B2M gene in HEK293T cells was conducted (a pPV-Dual_Promoter-EF1a-2xNLS-Cascade+Cas3(RD)-iCA vector and a pPV-C1-crRNA(B2M#4)-EF1a-BA vector were transfected). At this time, the Puro-SSA vector was simultaneously introduced, and puromycin selecton (2 µg/ml, for three to five days after the transfection) was conducted after the transfection. The genome editing efficiency for the samples was measured through HLA staining. As a result, the significantly high (about 6 times) genome editing efficiency was observed compared with the case where the puromycin selection was not conducted (FIG. 24). It was proven that the SSA vector for the CRISPR-Cas3 system could be used to concentrate genome-edited cells. Next, the specificity of the Puro-SSA vector was verified. A vector for expression of Cas3 and the Cascade protein group (the same as described above), a crRNA expression vector (non-target: AAVS1#1, target: B2M#1; pPV-C1-crRNA-EF1a-BA vector), and a Puro-SSA vector having the B2M#1 target sequence (pHL-EF1a-Puro-SSA(B2M 1kb)-A) were introduced to HEK293T cells using Lipofectamine 2000, and two to four days after the introduction, the cells were treated with 2 µg/ml puromycin for two days. The number of cells was counted six days after the introduction of the vector. As a result, it could be confirmed that Puro-resistant cells significantly increased among the cells to which the crRNA (B2M#1) had been introduced, and thus the specificity of the Puro-SSA vector to the crRNA was confirmed (FIG. 25). Also, the B2M knockout efficiency for the samples was measured. As a result, the genome-edited cells could be concentrated about 10-fold through Puro selection (FIG. 26).

It was possible to use combination of a plurality of different reporter genes (e.g., EGFP and mRFP) in this SSA vector, and it was also possible to apply the SSA vector to genome editing in which a plurality of crRNAs are used (FIG. 27). For example, in the case of the induction of multi-exon skipping for introducing a deletion in the exons 45 to 55 (about 340 kb) of the human dystrophin (DMD) gene as a model of treatment of Duchenne muscular dystrophy, even when two crRNAs are designed so as to face each other (crRNAs targeting Ex45 and Ex55), the deletion efficiency expected from the copy number at substantially an intermediate position is low due to the broad deletion range (FIG. 29: SSA unsorted). However, it was also possible to concentrate cells with a deletion of about 340 kb by simultaneously using EGFP-mRFP-based SSA vectors with these two target sequences and sorting a cell population positive for both EGFP and mRFP. First, in the experiment in which the length of the target insertion sequence was changed in HEK293T cells, it was confirmed that similarly to the experiment in FIG. 23, the specific signal and the non-specific signal decreased as the insertion sequence was longer (FIG. 28, lanes 1 to 6 on the left side). From here onward, an SSA vector having an insertion sequence of about 1 kb that does not reduce the specific signal and reduces the non-specific signal as much as possible is used. Next, cells positive for both EGFP and mRFP were actually sorted using a bicolor SSA vector having an insertion sequence of 1 kb, and the deletion efficiency was measured based on the copy number at substantially an intermediate position in about 340 kb through ddPCR. As a result, it was confirmed that compared with the case where the sorting was not conducted, the copy number decreased at a ratio as high as about 6% to about 40% in HEK293T, and also decreased at a ratio as high as about 0% to about 30% in F12020 iPS cells (FIG. 29, lanes 1 to 3 on the left side). When Cas9, which can induce only minute cleavage of a double strand, was used for the comparison, the cell concentration efficiency was poor (FIG. 29, lanes 6 to 9 on the left side), and it is understood that this SSA vector having a long insertion sequence is suitable for Cas3, which can induce a long deletion. Furthermore, the inventors succeeded in concentrating genome-edited cells from various iPS cell strains (FF12020, CiRA00458, CiRA00646) as well as obtaining a plurality of subclones with a deletion mutation of about 340 kb, by using this bicolor SSA vector system (FIG. 30). It is known that with this multi exon skipping method for introducing a deletion in the exon 45-55 region, it is possible to recover the reading frame of the dystrophin protein, and this method is also useful as a method for treating Duchenne muscular dystrophy.

Finally, the S/N ratio was compared with an SSA vector having a conventional short insertion sequence. When a crRNA (DMD20) and a crRNA (DMD23) were used simultaneously on the assumption that about 340 kb of the DMD gene is deleted by Cas3 in HEK293T cells, and the length of the insertion sequence of the SSA vector was increased to 0 kb (i.e., only the target sequence), 0.5 kb, 1 kb, and 1.7 kb, it was observed that the non-specific signal significantly decreased when the longer insertion sequence was used (FIG. 31). Meanwhile, it was observed that the on-target signal also decreased as the insertion sequence was longer. It was found from these results that the S/N ratio was maximum when the length of the insertion sequence was between about 1 and 1.7 kb.

Also, in addition to utilization of this long insertion sequence, the inventors noted that Cas3 is an enzyme nickase, which is a single-stranded DNA cleavage enzyme, and thus came up with an idea that a mechanism to induce cleavage of a double strand through DNA double nicking can also be applied to this SSA vector (FIG.27, right). In this SSA vector, two crRNA recognition sequences were arranged in the form of a palindromic sequence, and a spacer sequence of 0 to 35 bp was inserted between the two recognition sequences in consideration of steric hindrance in Cas3-Cascade binding. In the same manner as described above, when an experiment was conducted using two crRNAs targeting DMD Ex45 and Ex55 (B2M#1 as the non-target control), and a bicolor double-nick SSA vector (EGFP: Ex45, mRFP: Ex55) having the target sequence, higher on-target signals, fluorescence of EGFP and mRFP, were observed compared with the case where a non-target crRNA was used (FIG. 28, lanes 7 to 10). Out of these vectors, the double-nick SSA vector with no spacer was used to sort cells positive for both EGFP and mRFP, and a decrease in the copy number was measured through ddPCR in the same manner. As a result, it was found that the ratio of the decrease in the copy number increased to about 5% to about 40% compared with unsorted samples (FIG. 29, lanes 4 to 6 on the left side).

It was revealed from the results above that using this SSA vector (a long insertion sequence and an insertion sequence for double nicking) makes it possible to monitor or concentrate cells in which Cas3/crRNA is active.

### Example 4: Allele-Specific Genome Editing Technique Using CRISPR-Cas3 System

A gene on an autosome is generally composed of 2 copies that become paired, but an allele-specific genome editing technique targeting only one allelic gene is also important for treatment of a genetic disease. For example, myotonic dystrophy I (DM1) is a genetic disease caused by a CTG repeat in the 3'-UTR region of the DMPK gene abnormally extending in only one allele, and when DM1 is treated using genome editing, it is desirable that only the repeat sequence of this extended allele (disease allele) can be edited without affecting an allele (normal allele) having no problem. Therefore, the inventors of the present invention noted a hetero single nucleotide polymorphism (SNP) and developed a technique for a CRISPR-Cas3 system capable of deleting a CTG repeat that has extended in only the disease allele. First, the inventors searched for SNPs with high allele frequency on the SNP database, and simultaneously confirmed whether these SNPs were actually present in iPS cells derived from a plurality of DM1 patient (FIG. 32).

Next, crRNAs that can be designed on these SNPs were narrowed down. Such crRNAs are as follows: AAG, ATG, TAG, AGG, GAG, AAC, AAT, AAA, GTG, TTG, or GGG is used as the protospacer adjacent motif (PAM) sequence, the hetero SNP sequence is present in the crRNA recognizable sequence (position other than 6n^{th} (n is a positive integer) bases from the 3'-terminal base of the PAM sequence), and their direction is a direction in which Cas3 advances toward the CTG repeat (FIG. 33). As an actual example of the thus designed crRNAs, a crRNA for the rs934739524 SNP (C: disease allele, T: normal allele) on the downstream side of the CTG repeat found in the HPS1051 iPS cell strain that is a cell strain derived from a DM1 patient was designed (FIG. 34). A crRNA in which a hetero SNP is present at the twelfth base was used as a control crRNA that does not (or is less likely to) distinguish the alleles. As a result of conducting genome editing on the iPS cells HPS1051 strain derived from a DM1 patient using this crRNA (stable expression strain expressing Cas3, the Cascade protein group, and the crRNA expression vector), a deletion mutation was successfully caused preferentially in the target allele (FIG. 35). When this cell population was subjected to cell subcloning in order to confirm whether the target allele actually underwent genome editing, many strains in which the CTG repeat was deleted in only the disease allele could be actually obtained (FIG. 36: clone #9, 19, 20, 33, 84). As yet another example, it was possible to design crRNAs for SNPs, rs635299 and rs915915, on the upstream side of the CTG repeat found in the iPS cells CiRA00213 cell strain derived from a DM1 patient (FIG. 37), and it was confirmed from the results of a Sanger sequencing analysis of PCR products obtained by allele-specific PCR or Nested PCR that it was possible to delete or shorten the CTG repeat in both genome editing in which the RNAs were used (mRNA and Pre-both3 pre-crRNA) and genome editing in which the plasmid was used (plasmid-based genome editing: FIGS. 38 to 41, RNA-based genome editing: FIGS. 42 and 43). It was also possible to conduct this allele-specific genome editing by utilizing two hetero SNPs and using a pair of crRNAs facing each other with the target sequence being therebetween (allele-specific dual-Cas3), and it was also possible to conduct genome editing on the CiRA00213 strain by, for example, using a crRNA designed for rs635299 on the upstream side of the CTG repeat and a crRNA designed for rs3745802 on the downstream side (RNA-based editing: FIG. 44) and using a crRNA for rs915915 on the upstream side of the repeat and a crRNA for rs3745802 on the downstream side (RNA-based editing: FIGS. 45 and 47, plasmid-based editing: FIG. 46).

As described above, an allele-specific crRNA can be designed by arranging a hetero SNP in a crRNA recognition sequence site, but its activity and specificity can be confirmed using an SSA reporter vector (FIG. 48, left). For example, using insertion sequences different in one base in the sequence targeted by the crRNA are used in EGFP-mRFP bicolor SSA vectors makes it possible to analyze which of EGFP and mRFP preferentially sends out a signal. A crRNA targeting "C" of rs934739524 (crRNA-C), a crRNA targeting "T" (crRNA-T), and a non-targeting crRNA (NT crRNA) were used, sequences that include a SNP sequence of "C" or "T" were inserted into an EGFP SSA vector and a vector, and fluorescence was measured. As a result, fluorescence from the SSA vector targeted by the crRNA was preferentially observed (FIG. 48, right). It is expected that using these SSA vectors in combination with cell sorting makes it possible to concentrate cells having genome editing activity.

It is expected that designed allele-specific crRNAs vary in activity and specificity depending on their sequences, but the activity and specificity can be adjusted to some extent. Examples of the adjustment method include a method of shortening the crRNA, and a method of intentionally introducing a mismatch at a position distant from the PAM (FIG. 49). For example, in an experiment in which EGFP SSA targets "C" and mRFP SSA targets "T" for a crRNA targeting "C" of the SNP of rs934739524, the activity gradually decreased as the crRNA was shortened (FIG. 50) or the position of an intentional mismatch was brought closer to the PAM from a distant position (FIG. 51). However, it was simultaneously observed that non-specific activity (mRFP signal) also decreased. When this crRNA had a 30nt crRNA recognition sequence or had a mismatch at a position of the second base from 3' of the crRNA recognition sequence, the non-specific activity was successfully reduced while the activity against the target was retained. As described above, it can be said that adjustment of the length of a crRNA recognition sequence and introduction of an intentional mismatch are effective in adjusting the activity and specificity of an allele-specific crRNA.

### Example 5: Chemical Modification of crRNA Based on Protein Structure

Based on the crystal structure of E. coli Cascade/crRNA (Zhao et al. Nature, vol. 515, 147-150 (2014), Jackson et al. Science, 345(6203), 1473-1479 (2014)), potential 2'-O-Me (M) or phosphorothioate (PS) modification sites were modified as shown in FIG. 52 (Structure-guided: SG). It is known that three bases at both termini of a pre-crRNA can be modified without problems, and therefore, these bases were also modified in addition to SG (SG+Edges3). In addition, a crRNA in which all bases have the PS modification or the M modification (PS all, M all) and a crRNA in which at least a Cas6 processing site does not have the PS modification (PS except for cleavage site) were also prepared.

The genome editing activity was measured in I14s04 iPS cells using the pre-crRNAs above. 1.5×10⁵ cells were seeded on a 12-well plate, an mRNA for expression of Cas3 and the Cascade proteins and B2M#1 crRNAs (with the chemical modifications corresponding to FIG. 52) were transfected to the cells, and the B2M gene KO efficiency was measured through HLA-A, B, C staining (FIG. 53) or ddPCR (FIG. 54). B2M_1kb/PATL2 probes were used in ddPCR. As a result, it is found that it is difficult to predict the positions of a pre-crRNA to which a chemical modification can be introduced even if the site thereof capable of interacting with the Cascade complex is determined based on the crystal structure.

A further modification pattern was examined in the SG+Edges3 crRNA whose activity was not observed in FIGS. 53 and 54 with a focus on which modification site has an adverse effect on the activity. A pre-crRNA obtained by removing a modification in proximity of the Cas6 processing site (SG+Edges3-A), a pre-crRNA obtained by further removing the target sequence recognition site in SG+Edges3-A (SG+Edges3-A-Target), a pre-crRNA obtained by removing a modification of the inner arm portion in the SG+Edges3-A (SG+Edges3-A-Inner arm), a pre-crRNA obtained by removing a modification of the inner arm portion and the target sequence recognition site (SG+Edges3-A-Center), a pre-crRNA obtained by simultaneously modifying the three bases at both termini (the activity is enhanced by modifying these bases), both loops, and the left stem sequence of the left repeat (Edge3+Loops+Left Stem), and a pre-crRNA obtained by modifying the target sequence recognition site in addition to the three bases at both termini (Edge3+Target) were produced. These were transfected to I14s04 iPS cells in the same manner, and the B2M gene KO efficiency was measured through HLA-A, B, C staining. As a result, it was shown that modifying the inner arm portion and the target sequence recognition site had an adverse effect on the activity.

### Example 6: Measurement of Exon 2 Deletion Efficiency of HLA-A*24:02 and B*52:01 through ddPCR

An mRNA for expression of Cas3 and the Cascade protein group and Pre-both3 pre-crRNA (FIG. 5-1) targeting HLA-A*24:02 and B*52:01 were transfected a plurality of times to I14s04 iPS cells using Lipofectamine Stem, and collected genome samples were analyzed through ddPCR. "Dual A24 F1 & B52 R5" indicates a tandem pre-crRNA in which three bases at both termini have the MS modification. The information on crRNA target sequences used in this example is shown in Table 5.

**[Table 5]**

| Name | Sequence (5'→3') | SEQ ID NO. |
|---|---|---|
| A*24:02 F3 | GACCCTACATAGGTTGGGAGAGGGAGAAAAGA | 22 |
| A*24:02 F4 | AAGGACCCTACATAGGTTGGGAGAGGGAGAAA | 23 |
| A*24:02 R0 | GGGTGAAGGGTGGGGTCTGAGATTTCTTGTCT | 24 |
| A*24:02 R9 | ACGATCCCTCGAATACTGATGACTGGTTCCCT | 26 |
| A*24:02 R10 | GGATAAAATCTCTGACGGAATGACGGAAAGAC | 27 |
| A*24:02 R11 | GAGGGGAGACAATTGGGACCAACACTAGAATA | 28 |
| B*52:01 F2 | TGAAACTCGTGGGAGTGGGGAATCCCCAACGC | 29 |
| B*52:01 F3 | AAGTGAAACTCGTGGGAGTGGGGAATCCCCAA | 30 |
| B*52:01 F4 | GACCCGACATAGGTTGGGAGAAGAAGTGAAAC | 31 |
| B*52:01 F7 | AAGGACCCGACATAGGTTGGGAGAAGAAGTGA | 32 |
| B*52:01 R6 | CGCCTGAATTTTCTGACTCTTCCCATCAGACC | 34 |
| B*52:01 R8 | GAGGGGGATGAGGGGTCATATCTCTTCTCAGG | 35 |
| B*52:01 R9 | GGATGACGTCTCTGAGGAAATGGAGGGGAAGA | 36 |
| B*52:01 R10 | TCCCTGTTCCCCGCTCAGAGACTCGAACTTTC | 37 |
| B*52:01 R11 | AGCTCAGGTAGGGAAGGGGTGAGGGGTGGGGT | 38 |

### Example 7: Evaluation of Effects of Puro-SSA vector in 1383D4 iPS cells

1.5×10⁵ cells were seeded on a 12-well plate, and an mRNA for expression of Cas3 and the Cascade proteins, the B2M#1 crRNA (with three bases at both termini having the MS modification), and pHL-EF1a-Puro-SSA(B2M#1)-A or pHL-EF1a-Puro-SSA(B2M#1)-2A-EGFP-A serving as the Puro-SSA reporter vector were transfected. Puro selection was conducted at a concentration of 0.3 or 0.5 µg/ml two to three days after the transfection, and the ratio of cells negative for HLA-A, B, C were measured through HLA-A, B, C staining after the recovery of the cells. FIG. 59 shows representative results of flow cytometry and quantitative graphs (n = 2). As a result, B2M knockout cells could be concentrated about 2- to 4-fold.

### Industrial Applicability

With the present invention, it is possible to improve the efficiency of genome editing by the type I CRISPR-Cas system and to analyze a pattern of the genome editing by the system. It is also possible to provide a tool capable of concentrating genome-edited cells and to specifically conduct genome editing on one allele. Accordingly, the present invention is particularly useful in genome editing of pluripotent stem cells, which have been difficult to achieve using conventional method, and the like.

The present application claims priority based on Japanese Patent Application No. 2023-050681 (filed in Japan on March 27, 2023), the disclosures of which are incorporated herein in their entirety by reference.

## Claims

1. A pre-crRNA for a type I CRISPR-Cas system, comprising a modified nucleotide,
wherein at least one nucleotide is a modified nucleotide in a region consisting of a 5' arm region of a first repeat sequence, a 5'-side stem-forming region of the first repeat sequence, and a loop-forming region of the first repeat sequence.

2. The pre-crRNA according to claim 1, wherein at least one nucleotide in a 3' arm region of a second repeat sequence is a modified nucleotide.

3. The pre-crRNA according to claim 1 or 2, wherein first to third nucleotides from a 5' terminus and second to fourth nucleotides from a 3' terminus are modified nucleotides.

4. The pre-crRNA according to any one of claims 1 to 3, wherein all nucleotides in the loop-forming region of the first repeat sequence and/or a loop-forming region of a second repeat sequence are modified nucleotides.

5. The pre-crRNA according to any one of claims 1 to 4, wherein first to third nucleotides from a 5' terminus and second to fourth nucleotides from a 3' terminus are modified nucleotides, all nucleotides included in a 3' arm region of the first repeat sequence, a 5' arm region of a second repeat sequence, and a spacer region are ribonucleotides, and a site to be cleaved by Cas6 or Cas5 is not modified.

6. The method according to any one of claims 1 to 5, comprising two or more spacer regions.

7. The pre-crRNA according to any one of claims 1 to 6, wherein the type I CRISPR-Cas system is a type I-E CRISPR-Cas system.

8. A pre-crRNA for a type I CRISPR-Cas system, comprising a 5' cap and poly-A.

9. A method for designing an allele-specific pre-crRNA for a type I CRISPR-Cas system, comprising a step of designing a pre-crRNA targeting a sequence in which
a deletion target site is present on an upstream side of a PAM sequence adjacent to a sequence targeted by the pre-crRNA, and
at least one base that is different between alleles at positions other than 6n^{th} (n is a positive integer) positions from a terminal base on a downstream side of the PAM sequence or in the PAM sequence.

10. The method according to claim 9, comprising a step of adjusting a length of the pre-crRNA, and/or a step of introducing a mismatch to the pre-crRNA.

11. The method according to claim 9 or 10, wherein the deletion target site comprises a repeat.

12. A type I CRISPR-Cas system comprising (1) or (2) below:
(1) the pre-crRNA according to any one of claims 1 to 8, or a pre-crRNA designed using the method according to any one of claims 9 to 11, and
(2) a protein group included in Cascade, or a nucleic acid encoding the protein group.

13. A double-stranded nucleic acid for evaluating activity of a type I CRISPR-Cas system, wherein one nucleic acid strand comprises:
(1) a sequence encoding a portion of a reporter protein;
(2) an insertion sequence that includes a sequence targeted by a crRNA or a sequence complementary to the sequence targeted by the crRNA; and
(3) a sequence that encodes a portion of the reporter protein and is different from the sequence of (1), in this order,
the sequences of (1) and (3) have an overlapping sequence, the sequence of (1) excluding the overlapping sequence, the overlapping sequence, and the sequence of (3) excluding the overlapping sequence constituting a sequence encoding the entire reporter protein, and
the insertion sequence of (2) has a length of 100 to 3000 bases.

14. A double-stranded nucleic acid for evaluating activity of a type I CRISPR-Cas system, wherein one nucleic acid strand comprises:
(1) a sequence encoding a portion of a reporter protein;
(2) an insertion sequence that includes a sequence targeted by a crRNA and a sequence complementary to the sequence targeted by the crRNA; and
(3) a sequence that encodes a portion of the reporter protein and is different from the sequence of (1), in this order, and
the sequences of (1) and (3) have an overlapping sequence, the sequence of (1) excluding the overlapping sequence, the overlapping sequence, and the sequence of (3) excluding the overlapping sequence constituting a sequence encoding the entire reporter protein.

15. A method for producing a cell with an altered double-stranded DNA, comprising a step of introducing the CRISPR-Cas system according to claim 12 to a cell.

16. The method according to claim 15, wherein the step of introducing the CRISPR-Cas system to a cell is conducted a plurality of times.

17. The method according to claim 15 or 16, comprising a step of introducing the double-stranded nucleic acid according to claim 13 or 14 to a cell.

18. The method according to claim 17, wherein two or more types of the double-stranded nucleic acids are introduced.

19. The method according to claim 17 or 18, comprising a step of sorting a cell expressing a reporter protein.

20. The method according to any one of claims 15 to 19, wherein the cell is a pluripotent stem cell.

21. A method for analyzing a double-stranded DNA editing pattern by a type I CRISPR-Cas system, comprising a step of conducting digital PCR using a plurality of PCR probes for a deletion target site.
